# FASCICULE DE BREVET EUROPEEN

(11) **EP 2 499 250 B1**
(45) Date de publication et mention de la délivrance du brevet: **06.04.2016**
(21) Numéro de dépôt: 10784587.7
(22) Date de dépôt: 09.11.2010
(51) Int. Cl.: C12N 15/82

(54) **INDUCTION DE L'APOMIXIE CHEZ LES PLANTES CULTIVEES A REPRODUCTION SEXUEE ET UTILISATION POUR LA PRODUCTION DE PLANTES TOTALEMENT OU PARTIELLEMENT APOMICTIQUES**
INDUZIERUNG VON APOMIXIS BEI ZUCHTPFLANZEN MIT GESCHLECHTLICHER FORTPFLANZUNG UND VERWENDUNG ZUR HERSTELLUNG VON VOLLSTÄNDIG ODER TEILWEISE APOMIKTISCHEN PFLANZEN
INDUCTION OF APOMIXIS IN SEXUALLY REPRODUCING CULTIVATED PLANTS AND USE FOR PRODUCING TOTALLY OR PARTIALLY APOMICTIC PLANTS

(30) Priorité: 09.11.2009 FR 0905375
(43) Date de publication de la demande: 19.09.2012
(73) Titulaire: Institut de Recherche pour le Développement ( IRD), 13572 Marseille Cédex 02 (FR)
(72) Inventeur: GRIMANELLI, Daniel, F-34820 Assas (FR); LEBLANC, Ollivier, F-34270 Saint Croix de Quintillargues (FR); GARCIA AGUILAR, Marcelina, F-34090 Montpellier (FR)
(74) Mandataire: Cabinet Armengaud Aîné
(86) Numéro de dépôt international: PCT/IB2010/055084
(87) Numéro de publication internationale: WO 2011/055352

(56) Documents cités:
- FR-A1- 2 759 708
- OZIAS-AKINS PEGGY ET AL: "Mendelian genetics of apomixis in plants", 2007, ANNUAL REVIEW OF GENETICS, VOL. 41, PAGE(S) 509-537, XP002583194, ISSN: 0066-4197 le document en entier
- TAKASHI OKADA ET AL: "An Hieracium mutant, loss of apomeiosis 1 (loa1) is defective in the initiation of apomixis", SEXUAL PLANT REPRODUCTION, SPRINGER, BERLIN, DE LNKD- DOI:10.1007/S00497-007-0057-5, vol. 20, no. 4, 8 novembre 2007 (2007-11-08), pages 199-211, XP019564547, ISSN: 1432-2145
- CATANACH ANDREW S ET AL: "Deletion mapping of genetic regions associated with apomixis in Hieracium", décembre 2006 (2006-12), PROCEEDINGS OF THE NATIONAL ACADEMY OF SCIENCES OF THE UNITED STATES OF AMERICA, VOL. 103, NR. 49, PAGE(S) 18650-18655, XP002583195, ISSN: 0027-8424 le document en entier
- BICKNELL R A ET AL: "Monogenic inheritance of apomixis in two Hieracium species with distinct developmental mechanisms", HEREDITY, vol. 84, no. 2, février 2000 (2000-02), pages 228-237, XP002583196, ISSN: 0018-067X
- NATALIA V LASPINA ET AL: "Gene expression analysis at the onset of aposporous apomixis in Paspalum notatum", PLANT MOLECULAR BIOLOGY, KLUWER ACADEMIC PUBLISHERS, DORDRECHT, NL, vol. 67, no. 6, 15 mai 2008 (2008-05-15), pages 615-628, XP019613462, ISSN: 1573-5028
- LEBLANC OLIVIER ET AL: "Seed development and inheritance studies in apomictic maize-Tripsacum hybrids reveal barriers for the transfer of apomixis into sexual crops.", 2009, THE INTERNATIONAL JOURNAL OF DEVELOPMENTAL BIOLOGY 2009 LNKD- PUBMED:19247928, VOL. 53, NR. 4, PAGE(S) 585 - 596, XP002583197, ISSN: 1696-3547 le document en entier

## Description

Induction de l'apomixie chez les plantes cultivées à reproduction sexuée et utilisation pour la production de plantes totalement ou partiellement apomictiques.

L'invention a pour objet des moyens pour réguler le développement reproducteur chez les plantes cultivées. Plus particulièrement, l'invention a pour objet le développement de plantes se reproduisant totalement ou partiellement par apomixie gamétophytique, c'est-à-dire de manière asexuée par l'intermédiaire de graines.

L'apomixie gamétophytique est une forme de reproduction asexuée par graine. Elle existe chez de nombreuses angiospermes, et près de 400 espèces apomictiques ont été recensées. On ne trouve cependant pas de plantes apomictiques chez les principales céréales cultivées (maïs, blé, ou riz), mais uniquement chez des plantes sauvages, quelque espèces fourragères cultivées, et certaines espèces fruitières. L'apomixie est un mécanisme contrôlé génétiquement. Les plantes apomictiques développent les gamètes femelles sans méiose préalable. Les gamètes ainsi formés contiennent un génome identique à celui des tissus somatiques dont ils sont dérivés. Le développement de l'embryon à partir de ces gamètes se fait sans fécondation par un gamète mâle, c'est à dire par parthénogenèse. Le génome de l'embryon ainsi formé est donc strictement identique à celui de sa plante mère, sans contribution paternelle. L'apomixie est donc un mode de clonage par graine qui assure la perpétuation à l'identique des génotypes au travers des générations.

L'utilisation de l'apomixie de manière contrôlée chez les espèces cultivées offre de nombreuses applications potentielles. Ces applications concernent la propagation de génotypes instables, le contrôle des contaminations polliniques, les méthodes d'amélioration des plantes, et les méthodes de production commerciale de graines.

Aucune des ces applications n'est envisageable chez les principales espèces cultivées telles le blé, maïs, riz, et autres sur la base des technologies actuelles. On ne connaît en effet pas de formes apomictiques chez ces différentes espèces, et on ne connaît aucun système génétique qui permette d'induire de l'apomixie chez des plantes sexuées.

De nombreux laboratoires se sont attachés, au cours des années passées, à développer des plantes apomictiques, soit en tentant de transférer les déterminants de l'apomixie depuis des plantes sauvages vers des plantes cultivées, soit en induisant par mutagenèse des phénotypes apomictiques chez des plantes sexuées. Aucune de ces deux approches n'a produit de génotype apomictique chez une espèce où ce mode de reproduction n'existait pas antérieurement.

Des résultats récents, publiés récemment dans la revue Nature (Ravi, M., Marimuthu, M.P., and Siddiqi, I. (2008). Gamete formation without meiosis in Arabidopsis. Nature 451: 1121-1124.), montrent chez Arabidopsis que l'inactivation d'un gène impliqué dans la méiose, appelé DYAD, dont la fonction est de réguler la cohésion des chromatides durant la méiose, permet de produire environ 0,1% de gamètes qui échappent à la méiose (Ravi et al., Nature 2008 -451 :1121-1124). Le reste des gamètes, et donc 99,9% des descendances, sont stériles. Il est même probable que la fréquence de gamètes non-méiotiques ne soit pas significativement différente chez ces plantes mutantes de celle chez des plantes sexuées, et que ces gamètes n'apparaissent en fait à l'observation que parce que la mutation tue par ailleurs tous les gamètes normalement issus de sexualité. Un autre travail récent (d'Erfurth, I., Jolivet, S., Froger, N., Catrice, O., Novatchkova, M., and Mercier, R. (2009). Turning meiosis into mitosis. PLoS Biol. 7: e1000124.), beaucoup plus prometteur, montre qu'il est possible, chez Arabidopsis, de changer la division méiotique en division mitotique par l'inactivation simultanée de trois gènes impliqués dans la méiose (*osd1*/*Atspo11-1*/*Atrec8*)*.* Le triple mutant produit des gamètes diploïdes fonctionnels. Ces gamètes sont cependant fécondés, les descendances ne sont donc pas apomictiques, et on ne sait pas si ce résultat est transposable chez d'autres espèces qu'Arabidopsis.

Les travaux des inventeurs dans ce domaine ont montré qu'il est possible d'induire un phénotype tout ou partiellement apomictique chez le maïs en manipulant l'expression de plusieurs gènes qui sont collectivement impliqués dans la régulation de l'expression des gènes dans les organes reproducteurs femelles (les ovules) de maïs. Les graines produites échappent à la réduction méiotique et sont fertiles. Ces résultats s'appliquent avantageusement aux autres plantes cultivées telles que le riz ou le blé.

L'invention vise donc l'utilisation de séquences de nucléotides spécifiques dont la manipulation permet le développement de plantes se reproduisant totalement ou partiellement par apomixie gamétophytique.

Elle a également pour but de fournir une méthode de production de plantes apomictiques.

Selon encore un autre aspect, l'invention vise à utiliser de manière contrôlée l'apomixie chez des espèces cultivées à reproduction sexuée pour développer de nombreuses applications comme il sera exposé ci-après.

L'invention vise ainsi, pour la production de plantes partiellement ou totalement apomictiques, l'utilisation d'un gène codant pour une protéine à motif DNA Méthyl Transférase. Il s'agit plus spécialement d'un gène de la famille des DNA Méthyl Transférases codant pour une protéine de séquence SEQ ID N°1 ou SEQ ID N°5.

Selon un mode de réalisation, l'invention vise l'utilisation du gène DMT 103 de la famille des DNA Méthyl Transférases répondant à la séquence SEQ ID N°2 ou du transcrit d'un tel gène répondant à la séquence SEQ ID N°3, ou de l'ORF de séquence SEQ ID N°4.

Dans un autre mode de réalisation, l'invention vise l'utilisation du gène DMT102 de la famille des ADN Methyl Transférases, répondant à la séquence SEQ ID N°6, ou du transcrit d'un tel gène répondant à la séquence SEQ ID N°7, ou de l'ORF de séquence SEQ ID N°8.

Ces gènes sont exprimés de manière spécifique dans les ovules où se déterminent les cellules reproductives.

L'inactivation de ces gènes par mutagenèse et donc des transcrits et des protéines chez des plantes sexuées conduit à la formation de gamètes non-réduits et de gamétophytes multiples dans l'ovule, caractéristiques du développement apomictique.

L'invention vise également une méthode pour induire, dans les espèces cultivées telles que le maïs, riz ou blé, un phénotype tout ou partiellement apomictique, caractérisée en ce qu'elle comprend l'inactivation ciblée, par un élément transposable, par exemple de type Mutator, d'un gène, d'un transcrit de gène ou de son ORF, tels que définis ci-dessus, et l'identification du locus muté.

L'utilisation de l'apomixie de manière contrôlée chez les espèces cultivées offre de nombreuses applications potentielles. Elles concernent la propagation de génotypes instables, le contrôle des contaminations polliniques, les méthodes d'amélioration des plantes, et les méthodes de production commerciale de graines.

La première application concerne la propagation clonale, par graine, de génotypes génétiquement instables. C'est le cas en particulier de toutes les plantes hybrides; ces plantes hybrides produisent, par brassage génétique au cours de la méiose et de la fécondation des descendances qui sont différentes entre elles, et différentes de leur plante mère. C'est encore le cas des espèces cultivées présentant des niveaux de ploïdie instables en méiose, comme les formes triploïdes.

Chez la plupart des espèces cultivées, il est nécessaire, pour maintenir un haut niveau de pureté génétique, de contrôler rigoureusement la pollinisation, pour éviter une contamination par du pollen issu de champs voisins, plus ou moins éloignés, le pollen pouvant se déplacer sur des distances assez variables, fonctions de l'espèce, des conditions climatiques, ou des vecteurs de dissémination comme les insectes. Dans le cas de plantes apomictiques, cependant, le génome issu des gamètes mâles ne participe pas à la génération suivante. L'utilisation de plantes apomictiques permettrait donc de s'affranchir des risques de contamination. L'apomixie constitue donc une méthode totalement unique de contrôle de pureté génétique. C'est aussi potentiellement une méthode efficace pour éviter les flux indésirables de transgènes dans le cas de culture d'organismes génétiquement modifiés.

L'apomixie offre aussi de nouvelles perspectives en amélioration des plantes. Elle permettrait en effet d'utiliser comme variété nouvelle tout génotype sélectionné comme intéressant, dès lors qu'il s'agit d'un critère génétiquement déterminé, quelle qu'en soit la structure génétique, puisque celui-ci, dès lors qu'il est apomictique, devient génétiquement stable. On peut donc envisager de développer des variétés directement à partir de formes hybrides, éventuellement interspécifiques, en s'affranchissant des étapes de stabilisation actuellement nécessaires, comme les étapes d'autofécondation successives, ou la production d'haploïdes doublés. Cette méthode permet donc un gain de temps considérable, mais ouvre aussi certainement la porte à l'introduction de matériels génétiques totalement nouveaux dans les programmes de sélection, et en particulier de matériels génétiques qui, chez des plantes sexuées, induisent une forte stérilité. C'est le cas par exemple de la plupart des croisements interspécifiques.

Une application très importante concerne la production de graines hybrides. Telle qu'elle se pratique aujourd'hui, celle-ci implique l'hybridation contrôlée à large échelle d'écotypes parentaux génétiquement stables. Il s'agit généralement de lignées homozygotes, obtenues par différentes méthodes (production d'haploïdes doublés, autofécondations...). L'un des deux parents est utilisé comme mâle, l'autre comme femelle. Seules les femelles produisent les graines commerciales. Le rendement des parcelles de production de semences est généralement faible comparativement aux hybrides, pour trois raisons: (1) les lignées mâles sont nécessaires mais utilisent une part importante de l'espace sans produire de graines; (2) les lignées parentales ont généralement un rendement très inférieur aux hybrides du fait de la dépression de consanguinité ; (3) le contrôle des pollinisations implique la castration physique ou génétique des lignées utilisées comme femelle, un processus qui entraîne une perte de rendement importante. Dans le cas de plantes apomictiques, cependant, on pourrait envisager de produire les graines directement à partir d'hybrides, donc avec des rendements très supérieurs, en utilisant 100% de la surface disponible, sans nécessité de contrôler la pollinisation, et sans étape de castration. L'intérêt d'utiliser l'apomixie pour la production de graine est très significatif chez les espèces comme le maïs, ou l'on produit déjà des formes hybrides, pour des raisons de diminution de coûts, mais aussi chez les espèces autogames, comme par exemple le blé ou le riz, où les hybridations contrôlées à large échelle sont difficiles. La production de quelques plantes hybrides apomictiques serait suffisante pour initier la production à large échelle de graines hybrides, génétiquement stables.

Les plantes ou graines de plantes partiellement ou totalement apomictiques d'espèces cultivées telles que le maïs, riz et blé, caractérisées en ce qu'elles comprennent des allèles inactivés d'un gène, tel que défini ci-dessus entrent également dans le champ de l'invention.

Les plantes ou graines de plantes de l'invention sont avantageusement telles qu'obtenues pas inactivation du gène par mutagénèse ou selon la méthode telle que définie ci-dessus, pour induire dans les plantes cultivées un phénotype tout ou partiellement apomictique.

Ces plantes et ces graines, dont la protéine à motif DNA Méthyl Transférase est inactivée, produisent des gamètes non réduits et des sacs embryonnaires multiples.

D'autres caractéristiques et avantages de l'invention sont donnés dans les exemples qui suivent à titre illustratif.

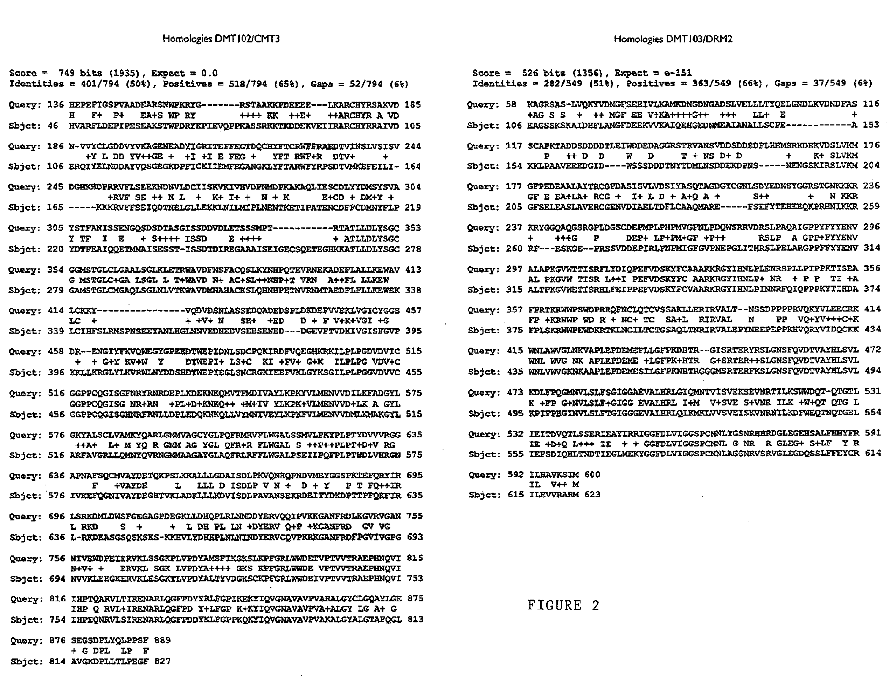

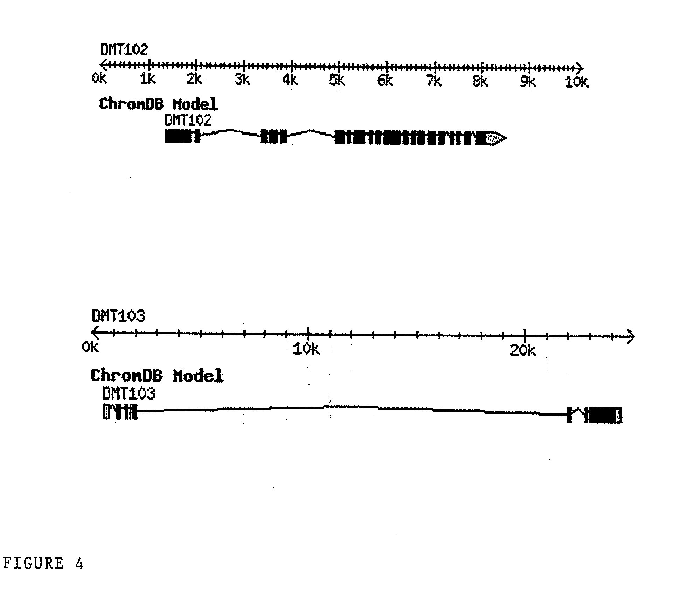

Ces exemples font référence aux figures 1 à 6, qui représentent, respectivement,
- la figure 1 : les profils comparés d'expression des régulateurs chromatidiens;
- la figure 2 : la comparaison de séquences protéiques avec l'alignement des séquences protéiques de DMT 102 et CMT3, et DMT 103 et DRM2;
- la figure 3 : le profil d'expression des deux gènes par mRNA in situ ;
- la figure 4 : la structure des deux gènes DMT 102 et DMT 103 ;
- la figure 5 : les phénotypes des plantes mutantes avec production de sacs embryonnaires multiples ; et
- la figure 6 : les phénotypes des plantes mutantes avec production de gamètes non-réduits.

Les séquences SEQ ID N°9, 10, 11 et 12 correspondent à celles de mutants ago 104-752, 770, 775 et 1352. Les séquences SEQ ID N°13 et 14 correspondent à celles de mutants de DMT 103-1042 et DMT 103-1342.

### DEFINITIONS

« Le gamétophyte » est la structure haploïde qui se développe à partir des produits de la méiose, et contient à maturité les gamètes. Il s'agit du sac embryonnaire côté femelle, et du grain de pollen côté mâle. Chez le maïs, l'ovule ne contient qu'un sac embryonnaire.

« L'apomixie gamétophytique » se réfère à une forme de reproduction asexuée par graines dans laquelle les gamètes produits dans les gamétophytes femelles n'ont pas subis de réduction méiotique, et présente donc la même ploïdie et la même constitution génétique que la plante mère. L'apomixie gamétophytique implique deux étapes successives : apoméiose et parthénogenèse.

« L'apoméiose » correspond aux mécanismes par lesquels les plantes apomictiques échappent à la méiose.

La « non-réduction » correspond à la formation de gamète en absence de réduction méiotique.

Des gamètes « non-réduits » sont donc des gamètes qui se développent en absence de méiose, ou au travers d'une méiose non réductionnelle. L'apoméiose est donc la forme spécifique de non réduction que l'on observe chez les plants apomictiques.

« La displosporie » est une forme spécifique d'apomixie gamétophytique, dans laquelle les gamètes apoméiotiques se développent à partir des mêmes cellules que celles qui participent au développement reproducteur sexué, c'est-à-dire l'archéspore.

« L'aposporie » est une forme spécifique d'apomixie gamétophytique, dans laquelle les gamétophytes femelles et les gamètes apoméiotiques se développent à partir des cellules somatiques de l'ovule. Chez les plantes aposporiques, la sexualité et l'apomixie coexiste fonctionnellement, et on retrouve donc typiquement plusieurs sacs embryonnaires dans un même ovule, issus soit de sexualité soit d'aposporie.

La « parthénogenèse » correspond au développement des embryons sans fécondation et sans contribution génétique paternelle.

### EXEMPLE 1 : Identification de régulateur de la structure de la chromatine dérégulée chez les plantes apomictiques

L'expérience décrite ci-après porte sur la comparaison du profil d'expression de gènes impliqués dans le déterminisme de la structure de la chromatine entre des plantes sexuées et des plantes apomictiques.

Les ARNs d'échantillons correspondant à des ovules de plantes apomictiques et sexuées aux stades de développement suivants ont été isolés ; ovules contenant une cellule mère de la mégaspore, ovules contenant une mégaspore fonctionnelle, et ovules au moment de la fécondation.

Les plantes sexuées utilisées sont deux lignées de maïs de référence, B73 et W23.

Les plantes apomictiques sont des formes hybrides obtenues par croisement d'une plante apomictique de l'espèce Tripsacum dactyloides, un apparenté sauvage du maïs chez qui on trouve des formes apomictiques, avec un maïs sexué ; ces plantes ont été ensuite rétro-croisées plusieurs fois sur du maïs, en sélectionnant les descendances apomictiques, jusqu'à l'obtention de plantes contenant un génome de maïs diploïde, et un génome de Tripsacum dactyloides haploïde.

Ces plantes ont été décrites dans la littérature précédemment, (Grimanelli et al, Genetics, 2003, Nov, 163(3) ; 1521-31) et se reproduisent par apomixie avec très forte pénétrance du caractère, proche de 95%. La taille relativement importante des organes reproducteurs chez le maïs permet une dissection fine des tissus utilisés, en utilisant un stéréomiscroscope. Un échantillon aléatoire des tissus échantillonnés est utilisé après chaque prélèvement pour vérifier les stages de développement.

Dans cette expérience, le profil d'expression de 386 gènes de maïs appartenant aux différentes familles de gènes connus pour affecter la structure de la chromatine a été précisé. Ces gènes ont été identifiés en utilisant la base de donnés CHROMDB, qui répertorie l'ensemble de gènes appartenant à ces familles chez les différentes espèces dont le génome est tout ou partiellement séquencé.

L'analyse s'est faite en deux étapes, avec tout d'abord la sélection des gènes s'exprimant spécifiquement dans les tissus reproducteurs ; puis, pour ces gènes sélectionnés, l'analyse de leur profil d'expression aux différents stades mentionnés précédemment pour les formes apomictiques et sexuées.

Sur 386 gènes analysés, 8 présentent un profil clairement altéré.

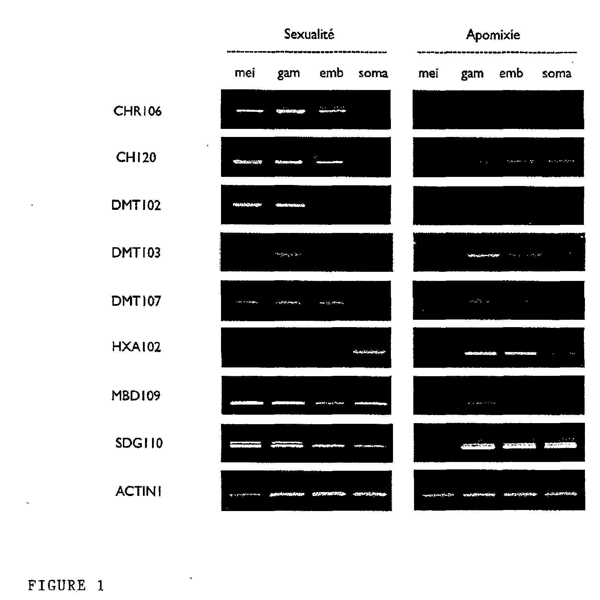

Ces résultats sont illustrés sur la Figure 1 : Légende :mei : ovule en méiose (sexualité) ou apoméiose (apomixie), gam : ovules en gamétogenèse ; emb : démarrage de l'embryogenèse, correspondant au moment de la fécondation ; soma ; tissus somatiques de la plante.

L'altération des profils d'expression peut impliquer l'absence totale d'expression chez une forme de reproduction par rapport à l'autre, c'est le cas par exemple de DMT 102 et CHR 106, dont l'expression est totalement abolie chez les plantes apomictiques.

La majorité des exemples, cependant, illustrent des dérégulations plus spécifiques dans le temps. C'est le cas de CHR120, DMT 103, DMT 107 HXA 102, MBD 109 ou SGD 110, dont l'expression n'est abolie qu'à cette étape spécifique du développement.

### EXEMPLE 2 : Fonction biologique des gènes, identifiée par homologie de séquence

La recherche d'homologies pour ces différents gènes, et en particulier l'utilisation de BLAST pour les comparer aux bases de données publiques, permet de leur attribuer une fonction biologique. Il apparaît clairement sur la base de ces homologies que l'ensemble des gènes identifiés sont impliqués directement ou indirectement dans les voies de silencing, et en particulier l'établissement ou de la maintenance de la méthylation de l'ADN :

CHR 106 est un homologue chez le maïs de DDM1 chez Arabidopsis, une enzyme impliquée dans la maintenance de la méthylation de l'ADN.

DMT102, DMT103 and DMT107 sont les homologues chez le maïs de respectivement CMT3, et DRM2 ou DRM1 chez Arabidopsis. DRM1 et 2 et CMT3 agissent de manière partiellement redondante dans le contrôle de la méthylation asymétrique (au site CHH ou CHG).

MBD109 est une protéine à motif d'attachement au groupe méthyle (Methyl Bindind Domain), de fonction inconnue, mais agit donc probablement sur des sites méthylés.

CHR120 est un homologue chez le maïs de MOM1 chez Arabidopsis, un gène impliqué dans les mécanismes de silencing.

HXA102 est un homologue chez le maïs de AtADA2 chez Arabidopsis, un composant du complexe histone acetyltransférase ADA.

SDG110 est une histone méthyl transférase, de fonction inconnue.

### EXEMPLE 3 : Profil d'expression des gènes DMT102 et DMT103 chez le maïs sexué.

On rapporte ci-après les résultats relatifs à DMT102 et DMT103. Ils correspondent à une voie extrêmement bien caractérisée chez Arabidopsis, appelée voie RdDM (pour RNA dependent DNA methylation).

DMT102 et DMT103 sont respectivement les homologues de CMT3 (CHROMOMETHYLASE 3) et DRM1 et 2 (DOMAIN REARRANGED METHYL TRANSFERASE 1 et 2) chez Arabidopsis. Les profils d'expression tissulaires de ces deux gènes (DMT102 et DMT103) ont été analysés par hybridation in situ de sondes ARN chez un maïs sexué.

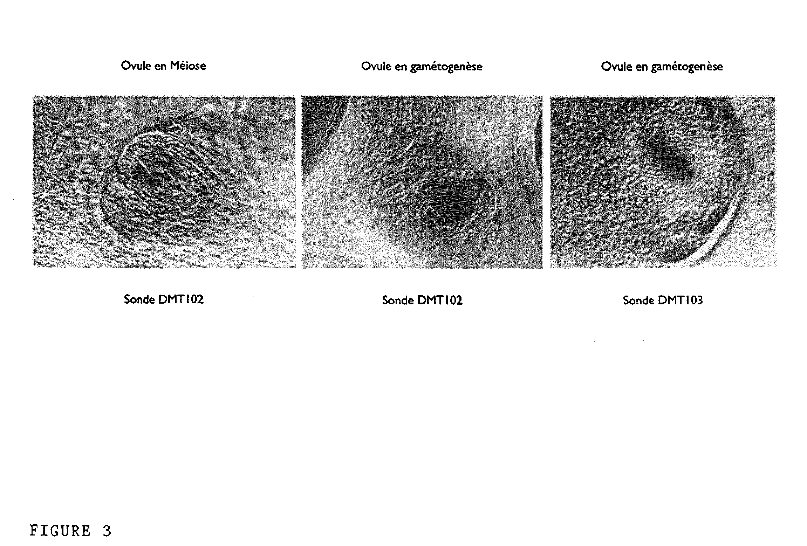

Les résultats sont illustrés Figure 3. Les profils d'hybridation obtenus confirment la grande spécificité d'expression détectée par RT-PCR: les deux gènes s'expriment de manière très spécifique au cours d'étapes ciblées du développement reproducteur. Ainsi, un signal est détectable pour DMT102 immédiatement avant, puis pendant la méiose. DMT103 n'est détecté qu'au cours de la gamétogenèse. Les données in situ montrent par ailleurs qu'au niveau tissulaire, ces deux gènes ne s'expriment que dans un nombre très limité de cellules, correspondant pour chaque ovule d'une part à la cellule reproductrice (l'archéspore, la cellule mère de la mégaspore et les méiocytes durant la sporogenèse; le gamétophyte au cours de la gamétogenèse), et d'autre part à un petit nombre de cellules entourant la cellule reproductrice.

L'action de ces gènes est donc limitée par un profil d'expression extrêmement ciblé d'un point de vue spatial et temporel.

Ce profil indique une différence importante entre les membres de la voie RdDM identifiés ici et la voie RdDM telle qu'elle a été décrite chez Arabidopsis: chez cette espèce, la voie RdDM est essentielle dans les tissus somatiques de la plantes, où ils jouent un rôle dans le maintien des profils de méthylation des séquences répétées. Leur rôle reproducteur chez Arabidopsis est inconnu, et des mutations dans les gènes correspondant n'ont pas de phénotype reproducteur particulier, ou d'effets notables sur la fertilité des plantes. Les gènes identifiés ici, par opposition, possèdent un profil d'expression essentiellement reproducteur.

### EXEMPLE 4 : Phénotype de plantes mutantes pour lesquelles la fonction de DMT102 et DMT103 est abolie

Pour démontrer le rôle potentiel de DMT102 et DMT103 dans l'expression de l'apomixie des expérimentations ont été réalisées pour vérifier si l'inactivation de leur fonction chez une plante sexuée conduit au même phénotype que celui de plantes apomictiques. Il s'agit de vérifier qu'en manipulant l'expression de ces gènes de telle façon que leur expression soit similaire à ce qu'elle est chez une plante apomictique, on récupère bien un réponse phénotypique équivalente.

Les plantes de maïs mutées spécifiquement dans ces deux gènes ont donc été analysées, les mutations correspondantes abolissant leur fonction. Pour DMT102, la lignée mutante correspond à l'insertion d'un transposon de la famille Mutator dans le domaine methyltransférase de la protéine. Pour DMT103, la mutation découle de la substitution de plusieurs acides aminés dans des sites essentiels: R-49-I, R-272-Q, C-182-Y (résidu dans la forme sauvage - position - résidu dans le mutant).

L'analyse de ces plantes a porté sur l'expression de deux caractéristiques propres aux plantes apomictiques: l'apoméiose, et donc la capacité à produire des gamètes non réduits, et l'aposporie, et donc la capacité à produire plusieurs sacs embryonnaires dans les ovules, mais avec une seule archéspore.

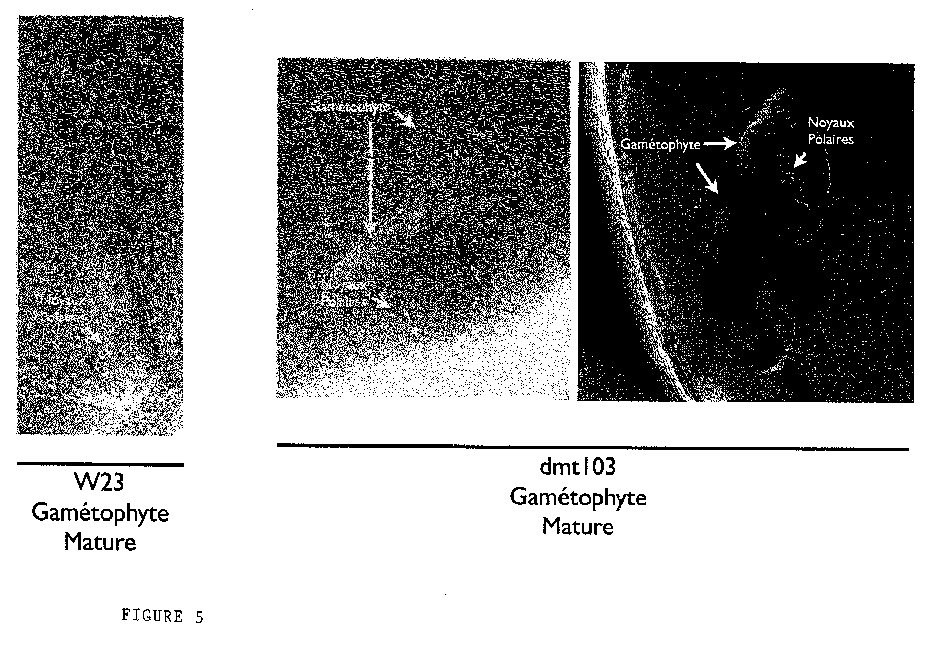

Comme montré sur la figure 5, les formes mutantes de DMT103 produisent des sacs embryonnaires multiples dans un unique ovule.

Ce phénotype n'a jamais été décrit dans la littérature en dehors des plantes apomictiques.

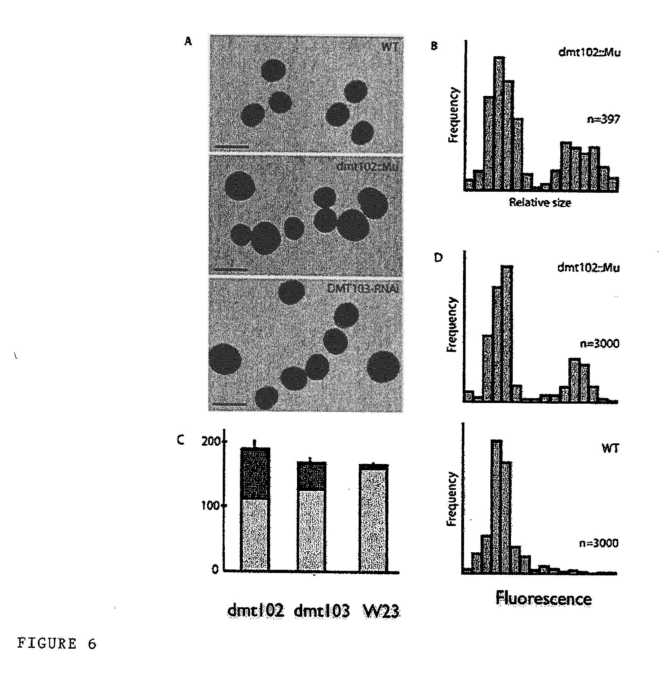

La figure 6 donne les phénotypes des plantes mutantes avec production de gamètes non-réduits : A) la taille des gamétophytes mâles est chez les plantes très corrélée au niveau de ploïdie des gamètes qu'ils contiennent. On peut rapidement évaluer la capacité d'une plante à produire des gamètes non-réduits par l'observation sous microscope des gamètophytes matures, ici les grains de pollen.

WT correspond à une plante sauvage, la lignée W23.

dmt102-mu et dmt103 sont deux formes mutantes de DMT102 et DMT103, respectivement, et produisent clairement des gamétophytes de tailles variables, telles qu'illustrées par le graphe de fréquence B). La fréquence de ces gamètes dans les plantes sauvage W23, et les deux formes mutantes est quantifiée en C). La relation entre taille des graines et le niveau de ploïdie est démontrée par une analyse en cytométrie de flux (D).

Cette figure montre que les deux gènes, sous leurs formes mutantes respectives, produisent une proportion élevée de gamètes non-réduits, de 30% chez DMT103 à 50% chez DMT102. Ces formes sont donc fortement apoméiotiques.

## Revendications

1. Utilisation pour produire des plantes partiellement ou totalement apomictiques d'un gène, d'un transcrit de ce gène, ou de son ORF, codant pour une protéine à motif DNA Méthyl Tranférase.

2. Utilisation selon la revendication 1, d'un gène codant pour une protéine de séquence SEQ ID N°1 ou SEQ ID N° 5.

3. Utilisation selon la revendication 2, **caractérisée en ce qu'**il s'agit du gène DMT 103 répondant à la séquence SEQ ID N°2, ou d'un transcrit d'un tel gène répondant à la séquence SEQ ID N°3, ou de son ORF de séquence SEQ ID N°4.

4. Utilisation selon la revendication 2, **caractérisée en ce qu'**il s'agit du gène DMT 102 répondant à la séquence SEQ ID N°6, ou du transcrit d'un tel gène répondant à la séquence SEQ ID N°7, ou de son ORF de séquence SEQ ID N°8.

5. Utilisation selon l'une quelconque des revendications 1 à 3, **caractérisée en ce que** le gène est inactivé par mutagénèse.

6. Méthode pour induire dans les espèces cultivées telles que le maïs, riz ou blé un phénotype tout ou partiellement apomictique, **caractérisée en ce qu'**elle comprend l'inactivation ciblée, par un élément transposable, par exemple de type Mutator, d'un gène tel que défini dans l'une quelconque des revendications là 4, d'un transcrit de ce gène selon la revendication 1 ou 4, ou de son ORF selon la revendication 1 ou 5, et l'identification du locus muté.

7. Application de la méthode selon la revendication 6, pour la propagation de génotypes instables, le contrôle de contaminations polliniques, l'amélioration des plantes et la production commerciale de graines.

8. Plantes ou graines de plantes génétiquement transformées, totalement ou partiellement apomictiques d'espèces cultivées telles que le maïs, riz ou blé **caractérisées en ce qu'**elles comprennent des allèles inactivés d'un gène, ce gène n'étant plus transcrit ou la transcription résultant en une protéine non fonctionnelle, tel que défini dans l'une quelconque des revendications 1 à 4.

9. Plantes ou graines de plantes génétiquement transformées, totalement ou partiellement apomictiques d'espèces cultivées telles que le maïs, riz ou blé obtenues selon la revendication 6.

## Patentansprüche

1. Verwendung eines Gens, das für ein Protein mit DNA-Methyltransferase-Motiv codiert, eines Transkripts des Gens oder seines offenen Leserahmens (open readingframe - ORF) zur Herstellung von teilweise oder vollständig apomiktischen Pflanzen.

2. Verwendung nach Anspruch 1 eines Gens, das für ein Protein mit der Sequenz SEQ ID Nr. 1 oder SEQ ID Nr. 5 codiert.

3. Verwendung nach Anspruch 2, **dadurch gekennzeichnet, dass** es sich um das Gen DMT 103 handelt, das der Sequenz SEQ ID Nr. 2 entspricht, oder um ein Transkrpit eines solchen Gens, das der Sequenz SEQ ID Nr. 3 entspricht, oder um seinen ORF mit der Sequenz SEQ ID Nr. 4.

4. Verwendung nach Anspruch 2, **dadurch gekennzeichnet, dass** es sich um das Gen DMT 102 handelt, das der Sequenz SEQ ID Nr. 6 entspricht, oder um das Transkript eines solchen Gens, das der Sequenz SEQ ID Nr. 7 entspricht, oder um seinen ORF mit der Sequenz SEQ ID Nr. 8.

5. Verwendung nach einem der Ansprüche 1 bis 3, **dadurch gekennzeichnet, dass** das Gen durch Mutagenese inaktiviert ist.

6. Verfahren zum Induzieren eines vollständig oder teilweise apomiktischen Phänotyps bei Zuchtpflanzen wie Mais, Reis oder Weizen, **dadurch gekennzeichnet, dass** das Verfahren Folgendes umfasst: das gezielte Inaktivieren eines Gens, wie es in einem der Ansprüche 1 bis 4 bestimmt ist, eines Transkript dieses Gens nach Anspruch 1 oder 4 oder seines ORFs nach Anspruch 1 oder 5 mittels eines transponierbaren Elements, beispielsweise eines Mutagens, und das Identifizieren der mutierten Stelle.

7. Anwenden des Verfahrens nach Anspruch 6 zum Vermehren instabiler Genotypen, zum Steuern von Verunreinigungen durch Pollen, zum Verbessern der Pflanzen und zum kommerziellenErzeugen von Samen.

8. Vollständig oder teilweise apomiktische genetisch transformierte Pflanzen oder Pflanzensamen von Zuchtpflanzen wie Mais, Reis oder Weizen, **dadurch gekennzeichnet, dass** sie inaktive Allele eines Gens umfassen, wobei das Gen nicht mehr transkribiert wird oder durch die Transkription ein nicht funktionsfähiges Protein gebildet wird, wie es in einem der Ansprüche 1 bis 4 bestimmt ist.

9. Vollständig oder teilweise apomiktische genetisch transformierte Pflanzen oder Pflanzensamen von Zuchtpflanzen wie Mais, Reis oder Weizen, die nach Anspruch 6 erhalten wurden.

## Claims

1. A use, for producing partially or totally apomictic plants, of a gene, of a transcript of this gene, or of its ORF, coding for a protein with a DNA methyltransferase motif.

2. The use according to claim 1, of a gene coding for a protein of sequence SEQ ID N°1 or SEQ ID N°.5.

3. The use according to claim 2, **characterized in that** it is the DMT103 gene corresponding to sequence SEQ ID N°2, or of a transcript of said gene corresponding to sequence SEQ ID N°3, or of its ORF of sequence SEQ ID N°4.

4. The use according to claim 2, **characterized in that** it is the DMT102 gene corresponding to sequence SEQ ID N°6, or of the transcript of said gene corresponding to sequence SEQ ID N°7, or of its ORF of sequence SEQ ID N°8.

5. The use according to any one of claims 1 to 3, **characterized in that** the gene is inactivated by mutagenesis.

6. A method for inducing, in cultivated species such as maize, rice or wheat, a totally or partially apomictic phenotype, **characterized in that** it comprises the targeted inactivation, by a transposable element, for example of the Mutator type, of a gene as defined in any one of claims 1 to 4, of a transcript of this gene according to claim 1 or 4, or of its ORF according to claim 1 or 5, and identification of the mutated locus.

7. An application of the method as claimed in claim 6, for the propagation of unstable genotypes, control of pollen contaminations, improvement of plants and commercial seed production.

8. Totally or partially apomictic genetically transformed plants of cultivated species such as maize, rice or wheat, or seeds thereof, **characterized in that** they comprise inactivated alleles of a gene, said gene not being transcribed or the transcription resulting in a non functional protein, as defined in any one of claims 1 to 4.

9. Totally or partially apomictic genetically transformed plants of cultivated species such as maize, rice or wheat, or seeds thereof, obtained as claimed in claim 6.


### SEQUENCE LISTING

<110> INSTITUT DE RECHERCHE POUR LE DEVELOPPEMENT (IRD)
<120> Induction de l'apomixie chez les plantes cultivées à reproduction sexuées et utilisation pour la production de plantes totalement ou partiellement apomictiques
<130> 63069-3595
<150> FR09/05375
   <151> 2009-11-09
<160> 14
<170> PatentIn version 3.5
<210> 1
   <211> 603
   <212> PRT
   <213> Artificial sequence
<220>
   <223> Methyltransferases
<400> 1
   Met Val His Trp Val Ser Asp Ser Asp Gly Ser Asp Asn Phe Glu Trp
   1               5                   10                  15
   Asp Ser Asp Gly Asn Gly Glu Gln Thr Val Ser Phe Asn Ala Ala Gly
               20                  25                  30
   Ala Gly Ser Ser Ala Leu Ala Ala Thr Asn Thr Asp Ala Pro Gly Pro
           35                  40                  45
   Ser Thr Arg Val Ala Asn Gly Asn Gly Lys Ala Gly Arg Ser Ala Ser
       50                  55                  60
   Leu Val Gln Lys Tyr Val Asp Met Gly Phe Ser Glu Glu Ile Val Leu
   65                  70                  75                  80
   Lys Ala Met Lys Asp Asn Gly Asp Asn Gly Ala Asp Ser Leu Val Glu
                   85                  90                  95
   Leu Leu Leu Thr Tyr Gln Glu Leu Gly Asn Asp Leu Lys Val Asp Asn
               100                 105                 110
   Asp Phe Ala Ser Ser Cys Ala Pro Lys Thr Ala Asp Asp Ser Asp Asp
           115                 120                 125
   Asp Asp Thr Leu Glu Ile Trp Asp Asp Glu Asp Ala Gly Gly Arg Ser
       130                 135                 140
   Thr Arg Val Ala Asn Ser Val Asp Asp Ser Asp Asp Glu Asp Phe Leu
   145                 150                 155                 160
   His Glu Met Ser Arg Lys Asp Glu Lys Val Asp Ser Leu Val Lys Met
   165 170 175
   Gly Phe Pro Glu Asp Glu Ala Ala Leu Ala Ile Thr Arg Cys Gly Pro
   180 185 190
   Asp Ala Ser Ile Ser Val Leu Val Asp Ser Ile Tyr Ala Ser Gln Thr
   195 200 205
   Ala Gly Asp Gly Tyr Cys Gly Asn Leu Ser Asp Tyr Glu Asp Asn Ser
   210 215 220
   Tyr Gly Gly Arg Ser Thr Gly Asn Lys Lys Lys Arg Lys Arg Tyr Gly
   225 230 235 240
   Gly Gln Ala Gln Gly Ser Arg Gly Pro Leu Asp Gly Ser Cys Asp Glu
   245 250 255
   Pro Met Pro Leu Pro His Pro Met Val Gly Phe Asn Leu Pro Asp Gln
   260 265 270
   Trp Ser Arg Arg Val Asp Arg Ser Leu Pro Ala Gln Ala Ile Gly Pro
   275 280 285
   Pro Tyr Phe Tyr Tyr Glu Asn Val Ala Leu Ala Pro Lys Gly Val Trp
   290 295 300
   Thr Thr Ile Ser Arg Phe Leu Tyr Asp Ile Gln Pro Glu Phe Val Asp
   305 310 315 320
   Ser Lys Tyr Phe Cys Ala Ala Ala Arg Lys Arg Gly Tyr Ile His Asn
   325 330 335
   Leu Pro Leu Glu Asn Arg Ser Pro Leu Leu Pro Ile Pro Pro Lys Thr
   340 345 350
   Ile Ser Glu Ala Phe Pro Arg Thr Lys Arg Trp Trp Pro Ser Trp Asp
   355 360 365
   Pro Arg Arg Gln Phe Asn Cys Leu Gln Thr Cys Val Ser Ser Ala Lys
   370 375 380
   Leu Leu Glu Arg Ile Arg Val Ala Leu Thr Asn Ser Ser Asp Pro Pro
   385 390 395 400
   Pro Pro Arg Val Gln Lys Tyr Val Leu Glu Glu Cys Arg Lys Trp Asn
   405 410 415
   Leu Ala Trp Val Gly Leu Asn Lys Val Ala Pro Leu Glu Pro Asp Glu
   420 425 430
   Met Glu Phe Leu Leu Gly Phe Pro Lys Asp His Thr Arg Gly Ile Ser
   435 440 445
   Arg Thr Glu Arg Tyr Arg Ser Leu Gly Asn Ser Phe Gln Val Asp Thr
   450 455 460
   Val Ala Tyr His Leu Ser Val Leu Lys Asp Leu Phe Pro Gln Gly Met
   465 470 475 480
   Asn Val Leu Ser Leu Phe Ser Gly Ile Gly Gly Ala Glu Val Ala Leu
   485 490 495
   His Arg Leu Gly Ile Gln Met Asn Thr Val Ile Ser Val Glu Lys Ser
   500 505 510
   Glu Val Asn Arg Thr Ile Leu Lys Ser Trp Trp Asp Gln Thr Gln Thr
   515 520 525
   Gly Thr Leu Ile Glu Ile Thr Asp Val Gln Thr Leu Ser Ser Glu Arg
   530 535 540
   Ile Glu Ala Tyr Ile Arg Arg Ile Gly Gly Phe Asp Leu Val Ile Gly
   545 550 555 560
   Gly Ser Pro Cys Asn Asn Leu Thr Gly Ser Asn Arg His His Arg Asp
   565 570 575
   Gly Leu Glu Gly Glu His Ser Ala Leu Phe His His Tyr Phe Arg Ile
   580 585 590
   Leu His Ala Val Lys Ser Ile Met Glu Arg Leu
   595 600
<210> 2
   <211> 25017
   <212> DNA
   <213> Artificial sequence
<220>
   <223> Methyltransferases
<400> 2
   ccaacaaact gactgatact cctttcgtcc acaaatctct gtcgcccgat cttttttcat        60
   ttttctatta gtcttcgttg tattagcatg ttcctctgtc ttctcagtat gtctgtcgct        120
   tcattacttc tgcgcattct ttaagaatct ttattgatat cgggtattat gaaaacttgt        180
   agtgattaaa agcaaatcga cgctccttgg tcactgcacc aatgcctaat acgctggaga        240
   tttaggaatg gatggagtat aatatactta gaaattaatt atataaacat agttaaaaat 300
   taatatatat gaagagtcgt gtaaagataa acttttaaat atagttgaga gtcaatatat 360
   ttgagaagac catcttttat cttttttatt tacattaaat gatattgtac ctgctctgac 420
   taaagcggtc agacggtgga gacagacaga ggacgactat cttttatatt tttatttaca 480
   ttaaatgata ttgtatcttt attgtcttgc cctaaagcgg tcagtgggtg gaggcagcca 540
   agagtcaccc tgtcgccgtt gccgtcgccg aggcaggcag agtctccctg tcgccgttgc 600
   cgtcgccggc ctcctcctcc tctgccactt cctcccaact cccagccgca ggggccgacg 660
   gcgacgggag ggaagaggcg gcagaggtac tccgaggggc gcggagagag gctgtgccag 720
   gccacgccgt ccagtgcctc cgggtccgtc gccgcagctt ccggccgcgt cggagaggta 780
   gcccccggag ctcttcgcgg aggctcggga atggtggtga gagtttccct tcgcgtcccc 840
   tcgtcttctc tgctctgcct ccttccgtgt actgtgaaga gatgctatct cgcgcaatga 900
   attcttgacg ctaataatat ggtccgctgc tacggatatt ccgcgcgtta gattggggtc 960
   tttgatgtgg cgaggtcatt acggtgtcct agccctcata ttcagcgctc agtgttgggg 1020
   ttttcttaac actggattga gctagagctt gcgttttgga tgtttttttc gagattgatg 1080
   gtggtttggt cctgttgttg atcgatgtgg ctgatatgta cttcttaatt cgtgtcacag 1140
   cactgggtta gcgacagtga tggcagtgat aacttcgaat gggacagtga tggtaacggg 1200
   gagcagacag tgagcttcaa cgctgctggt gctggttcat cagctctggc agcgacgaac 1260
   actgatgctc ctggcccatc gacacgggta agccagctaa cggatccata catatatgca 1320
   acaatgtgtg agttcagcag ctagttcgtg ttactcaggg gtgttcaatt taaaccactt 1380
   gtgccaaact ggccggggtg gggttgggtg gtggatataa taatcttgaa caaacgcagg 1440
   atcatgatta gtctacagtg acacggacac cctcaatgtt actgttacct tgtttctagt 1500
   ggctacgatt gtaaggtttc tatttgtttg tggggttggt tttaaggttg ctaatggcaa 1560
   tgggaaggct gggcgatctg cctctttggt tcagaagtat gtggacatgg gtttctcaga 1620
   agagattgtt ctgaaggcca tgaaggacaa tggtacttaa ctaatagctc atctcttctc 1680
   atttatttag attctatgtg gtttaatata ccctatatca cagttttttt ttctttcagg 1740
   ggataatgga gcagattcat tagttgagct ccttcttact taccaggtat tcatttgtgt 1800
   tcctgcttct atgttattct ttgatgatgt tgttgcatgg tatcaaagag aatttatttt 1860
   acaaagtgaa taagtgatgc caaatgcagg aactaggcaa tgacctcaaa gtggataatg 1920
   actttgcttc tagttgtgcc cccaaaactg ctgacgatag tgatgatgat gacacactgg 1980
   aaatctggga tgatgaggat gctggaggga gaagcaccag ggttgctaac tctgttgatg 2040
   attctgatga cgaggtattt tcatgctccc cttggtgatt gtaagcgttt actttattct 2100
   tttttgagaa acctactatg catttacttt gcttcaatca tatatacaac gacaaacagc 2160
   aacaacaaaa tcttttattc caaacaagtt gcgtagacta gagttaaaaa attaatagaa 2220
   caacaagtca tggttcggcc acatggatag ttgtttttta tgaactcata ttcaaggcta 2280
   aatctttggg ttgggtatat cccatcctta tctccattta ctgtctcttc ccatgtcaac 2340
   tttagtcttc ctcttcctcc cttctcaatt ctcataacta aggcgcctta gaattccact 2400
   acgcacttgc gtctatggag gtctacgttg gacatgtcca aaccatctca aacgttgttg 2460
   gacaagtttt tcttcaactg gtgctactcc tagcctatca cgtatatcat cattccggat 2520
   ttgatctctt cttgtatggc cacaaattaa acacaacata cacattttcg ggacacttaa 2580
   ttgttgaaca tgtcgtcttt tgtagggcaa cattctgcac catacaacat agaaggttta 2640
   atcattgtcc tataaatctt ttcttttagc ttctgtggta ccctcttgtc atatagaact 2700
   ccagatgctt gacgccactt catccatccg cttcgattct atggctaaca tcttcattaa 2760
   tatccccgtc tctctatagc attgatccta aatacctaaa tgtgtccttc taggcactgc 2820
   ctgactttcc aaactaacat ctcctttctc atgtgtagtc ccgaaatcac atttcatata 2880
   ttttgtttta gttgtactga gtctaaaacc tttggattct agagtctctc gccacaactc 2940
   tagtttccta tttactcttg cccaccacat aacattcctt ggtatcttgt cataattttc 3000
   catgtcaatg aaatgtcaca cccggattta agggataaag ccgggtgtgt ctcatatgtg 3060
   cgccaaaaaa gaacaacaca tataatgata aagtgtatag agataaatgt cacaatatca 3120
   taaaagtaat tattacatag cggaagtctt acaaaaataa atgataaata taaaacgaac 3180
   taatgtccat ccatggcgcc ataacgttga ctgggagacg ccacctagtt caactcgtat 3240
   gcttcattgt agggcggctc ctcttcgacc acctgctatt aacctgtggg gggggggggg 3300
   gtttatatat cgcaagagtg agctcacaaa atatcatagc ttaagtgatt aagtgagaac 3360
   tcaagaagaa aaatcaatca caataaaaga tgacacaaga gacacagtga ttttatcccg 3420
   tggttcagcc aatgcctact ccacgttgtg gtgacctcct tcggtcaagg attgcactca 3480
   atccctctca agtgatccaa caatcaaact tgagtatcac agttttcttc cttagtgatg 3540
   tttcccgttt gtgaggaatc tccacaagtt ggagcctctc acccttacaa tgttgatcac 3600
   aagaaaagca caagagtaag ggagggaaag aaacacacac aagactagaa tcgcaacaac 3660
   aatacacaca caagccaaga agagagcaca caaaaacaat gccacaagtt cacaacacac 3720
   ttagtgctca aatctcagtc acgatgatcc aaatgcgtat ttgcggagtc taggcgtctt 3780
   aggatgttta gagaatgctt ggtgtagtgc tccatgcgcc taggggttcc ttttatagcc 3840
   ccaaggcagc taggagccgt tggaattcca tttggaaagg taattcttgc ctttagaagc 3900
   ttatgaaggt tcttcattcc aacatgtgct agatggcggt gccagagcca gcccatatta 3960
   gtcttagcga ttaagcatgc atctagatcg acattctctt ttgagaaatc aactaaatag 4020
   agcttgccgt gtaatacagc cttaaaagtt aatgaacaat cactccttct aaagacagat 4080
   acatcaatat ttgtaaataa acaattatag cccatatgac ataattgact tacaaacaat 4140
   aaattatacc cgagcgattc tactaaaaac acatttgaaa ttgaatgctc ggttgtgatg 4200
   gctatcttgc ttaagccctt aaccttgcct tggtttccat ctccaaaaat gatagtatct 4260
   tgggaatccc tgttcttgat gtaagaggtg aacattttct tctcccccgt catgtggttt 4320
   ttgcatccgc tatcaataat ccagcttgag ctcccggatg cataaacctg caaggtaatt 4380
   aagcttggct tttaggtacc caagtcttgt tgggtcctac aaggttagtt acaacaaatt 4440
   ttggaaccca aatgcatgtt tctcccttgc atttagatcc caattttcta gcaactactt 4500
   tggcattttt acatgacaat acaaaagaaa cattacatgt ttgatacata gtagttggac 4560
   cagtgtatac tttcctaggt gcatgagaat cactatgatt acgcctaggc ctacttctac 4620
   catgaacata tgtggagcta gatgcaaaca tagcatgaga attatgataa gtaggtagag 4680
   caacatgatt ataacactta tcatgatggg caacactgga aattttcttg acatgagcat 4740
   aaagataagc atgattcttt tgaccatgtg aagaatacaa gctactagcc ataggagcct 4800
   tccctttctc tttgttgaga ctggaagtcc tttggcttgt taagttcttg gtttcttatc 4860
   ggaaaccaag tccatcctta attgaggggt gtctaccaat gatgtaggca tccctagcaa 4920
   atttgagttt ctcataatta tctttgcaag tcttaagttg agcattaaga cttgcaacat 4980
   cattgtttaa cttaataata gtagaattat gttcattaca agcatcaacg ttaaaatttt 5040
   tgcatctatt gcaaataaca acatgctcta tggatgaact agcagaattt gcttctttta 5100
   acttagcatt taaatcatca tttaaattct ttaggctaga gatagattca ttataagcag 5160
   ataattcaga agtcaatagt tcattcttct tagtttctag agcaagggct ttttgaacat 5220
   taatgaattt atcatgttct tcataaagaa tatcttcttg cttttctaga agcctatcct 5280
   tttcattaag agcatcaatt aattcattta ttttgtctac tttagtttta tctaatcctt 5340
   taaacaaatc agtatagtcc acttcatcat cagatgattc ctcatcacta gaagaagaat 5400
   acttgggggt atctcgaata cgtacctttt tctccttagc catgaggcac gtgtgtcgtt 5460
   cgttggagaa gaggaaagac ttgttgaagg tcgaggctag tccttcatca tcgaagtcgg 5520
   atgaagaaca gtcggagtcc cactcttttt caatgtgcgc ctcgcctttc gccttcctgt 5580
   agttcttctt tctttccttc ttcccatgct tctcttgtgc ctggtcatta tcgttatcga 5640
   ggcgttgtgc tataaaatga ccagacttac cgcatttgaa gcaggagcat tttccccttg 5700
   atttgctctt gttagggtac tccttgcgtc cttttaaggc ggtcttgaag cgcttgatga 5760
   tgagtgccat ctcatcctcg ttgagcccgg catcctccac ttgtgctagc ttgctaggta 5820
   gtacctcctt gctgctggtt gctttgagag caacgggctg cgattcgtgt agaggaagcg 5880
   gtccgtttaa tgcatcatcc acatacctcg cttcctttac catcatgcgc ccgctaacga 5940
   attttccgat gatttcctcg ggcgtcatct tggtgtacct ggggttctca cgattaaggt 6000
   ttataacatg aggatcaata acagtaaatg acctgagcat gagtcggacg acgtcatgat 6060
   ccgtccatct tgtactcccg tagcttcgga ttttgttgac aagggtcttg agcctgttgt 6120
   acgtttgtgt tggctcttct ccccttatca tcgcgaatct ccctagctcg ccttccacca 6180
   gttccatttt agggattatg gtggcatcat ttccctcgtg agaattttga gggtgtctca 6240
   tatttgtttg gcattatcta ggccactttg ttgtattcat ccctgcatag agatgctaat 6300
   aaaatagtag tagcttgtgt atttttgtgt atttgctcat tgataaacac agggttgtta 6360
   gagctatcaa aatgcattca attttcaaca atctcccaga tactaggatg gagggagaat 6420
   aaatgactac gcattttatg actccaaaaa gaataatcct ctccatcaaa gtgtgggggt 6480
   tttccaagtg gaatagagag caaatgggca tttgaattgt aggggatgcg agaataatca 6540
   aaggaataat tttgattaag cgttttcttt ttcgatgaag agtcttcgtc gtcgtcatcc 6600
   cttggtgaag aggaggcgtc actgtcgtag tagatgatct tctttatgtg cctcttcttc 6660
   tttctgtcct tcttcttgtg acttgagcct gagtcagtgg gcttgtcatc tcttggctcg 6720
   ttgaagaggg actccttctc cttgtcgttg atcaccatcc ccttaccctt aggatccatc 6780
   tctttggtcg gttagtccct aaatgaagag tacgactctg ctaccaattg agagcaccta 6840
   gaggggggtg aataggtgat cctgtaaaat caagtactaa atagtccaaa cttggttacg 6900
   aaatgttagt tagatcaaaa ccaagatgct atagcgagaa ctcttcactt gattgttcct 6960
   ttagaatgag tattaaactt aggaacaaga gcacaagtga ttaagcggcc tatgatggtt 7020
   tctttattgg ctcaacaacc tttaggtctt ggaggaggat ttggaagagt tgggctcctg 7080
   gaaaatgtaa attcttcatg tggctggtgg cgcacggccg ctgctggacc gctgaccggc 7140
   tggcaaggag aggcctgcct catcctcaaa gttgtctgtt atgtgaccag gaggaagaaa 7200
   ctatgaatca tctgctagtt tcctgtgtct tctcaaggga agtctggttt aatatcctga 7260
   aaacggtcgg tttgcagcac ctctctccca gtctcgattc ttcttctttt gatgattggt 7320
   gggagaatgt aagcatgacc cttattagcc cctccgataa gaagctgcgc aaggggctga 7380
   actcccttat cattttagga gcttgggcca tttggattca tcgcaatcaa tgtgtcttca 7440
   acggagagca gcctagggct agcaaggtca taaattgggt catggctgag tctcatctct 7500
   ggtgtagggc tggggctcgc ggattatctg atatcctttt tacctcggca tactatttga 7560
   ggagttggtc tctagtatgc ttagtgcact ggtgctcctg ctcattttct tttgttgggt 7620
   ggtgtgttga gttgtatgag gttctttaac acctcacccc atctttttct acttaatata 7680
   atgatacgca gctctcctgc gtattcgaga aaaaaaagaa gaaaaaccaa tcacaataaa 7740
   agatgacaca agagacacgg tgattttatc ccgtgtttga ccaatgccta ctccacattg 7800
   tggtgacctc cttcggtcca agaattgcaa ctcaatccct ctcaaatgat ccaacgatca 7860
   aacttgagta ccaccgtttt cttccttagt gatgtttccc gtttgtgagg aatctctaca 7920
   agttggagcc tctcaccctt acaatgttga tcacaagaaa agcacaagag taagggaggg 7980
   aaagaaacac acacaaaaca agaatcgcag caacaataca cacacaagcc aagaagagag 8040
   cacacaagaa caatgccaca agttcacaac acactcagtg gtcaaatctc aatcatgatg 8100
   atccaaatgc gtatttgcgg agtttaggcg tcttaggatg tttagagaat gtttggtgta 8160
   gtgctccatg cgcttagggg tcccttttat agccccaagg cagctatgag ccattggaat 8220
   tccatttgga aggcaattct tgccttctgt cgggcggcac accggacagt ctggtgcacc 8280
   accggacatg aacagtcctt gttcggtgcc cgatctcctt ccttttctgg cgaagccgat 8340
   cgttgagccc ttggtcccct tgtcgcaccg gacattgtcc ggtgcgacca agttaccgtt 8400
   ggcccaggcc acgcgtcacc cgctgattgc gctgccgacc gttggcgagg gcgccgttgg 8460
   ctcaccggac agtccggtga attatagccg cagcgccctc ggcgaattcc caagaacgac 8520
   gagttcgtcg ccgagccagc ctgggcaccg gacagtccgg tgcacctcag gctggttcaa 8580
   gtttggctgc acttagccaa acttctccaa tccaatctta gttgattaac caaggttcct 8640
   agcacttaga gaaatatgtt agtactcaaa aacaatttac taaggccaga gtcatacctt 8700
   gattctttga tttgcatctc ttccacactt agtacatttg aacctaagta atttgtgttg 8760
   ggcatctaat caccaaaaca tttatagaaa tggcccaagg gcacatttcc ctttcacaaa 8820
   accataaaga gcaatagcaa aaactaccca aatagttcag gggtaaacaa ggtgaaaaga 8880
   taaccagact agggtgacct attgggtccc atcaaaatta aacctatgca tgaataaatg 8940
   attataaaga acattattgg gtaataaaag tggtaaaggg cacaacttgc cttcaatgag 9000
   ctcctgctca gtaacttcta cctgctaaac accctgatcc tcggccactg gctcttctac 9060
   tcgtcacaat acaaacaagc atggtataag gaagaaatta acatcacacc aaacatgtat 9120
   acaaaataca taataataat ctacacatta aaacgagatc ataggaaaaa gaatcactaa 9180
   attcggagtt acagatttca agttatgatt ttccgaagta tttatgtgct tggtacaaaa 9240
   ttgattgtgt gatcaattct aatgtgggtt tcatggtaaa acagtaacac taagtgataa 9300
   acaatattat tacaaaatta taggaactgg aatggattaa tttggactta atatgaatta 9360
   gttttgtact aaaaatcaat ttccataatt atttatccaa tttctctgtt ctctgggccg 9420
   agcactaaat ataaggaaac acgggggcag aatcataaga tttccccaga ctcagagcaa 9480
   ccctggggtg gacggcgggt tgattttaag tattcctaag ggctcttttg agatattgcc 9540
   acgcgaaggg gtactgtgtg atctgagccg tccgatcaat tccagacgct cttgatttaa 9600
   tttacccgag cgtgaaacgg tacgctatgg cagccattag atcgccgatc gacgacacgg 9660
   attcaaacac cccacgccgc caccgagacg tccgatcggg atctagcggc gcggatctgt 9720
   ccaatcgaag gggtacgcgg cgtctaatct aagccgtcca tttagcatct aacggttcac 9780
   actcagtcgt ctacctctca gtcgtaatag cgcgacggcg gcgaattctc ccgtgacgga 9840
   gatctctccg gcgagcaccc acaggcgcac caacgccccg aagccgaaat tgactaacac 9900
   atacacccta cgaggacatg gcgaataaac tggggagtga atttacctcg gttcacggcg 9960
   gcaaggtctc tgttcacgac acaaggcggt gcggcaccca agcgaagcac cgacgagcaa 10020
   ttaaggttgc tctgcttgtc gattcggcca gcaagtgctt cggtctccct cgcagcaact 10080
   cggcgcaggt tcctgactcg atctccggca ctggacggtg acgggaacgg gaacccgtgt 10140
   ctccccactt ctgccacccc tggcttttct ctccctaagc tctatacgcg acggcgaggg 10200
   tttggcacgg aagcgagagg cgcataaggt tcggtgcagc agagttttat ggccgggtga 10260
   ggttgggcgg ttagcggcca gctgatccac aaaccccgcg tggtggcgga catctcggcc 10320
   atggccgttg cggaggtgag ggtgatgttt tgacacgggg ggcccagacg cagtgagggc 10380
   gcaacgcgac gtgttgagtc ggtctctcgg ggcccacggg acagaagctt cgtgcgaccg 10440
   agttacgtag actggcaagg gggccccgca agtcagtcat ggattccggc tgggccttgc 10500
   agagaacgtg ctggttgggc cggctaagcc ggattaggcc caggcgcaca gttcctttct 10560
   tcctttttct tttctatttt atttttccta tttctttcat tttcagtttt aagtttcgaa 10620
   tttgaattca tatgtgtgga gctcatactt aggttaaatg ttttcattca aataatagta 10680
   tgaatcgaat ttatttattt aattaattta atttgtatag tatttttctt cttttttctc 10740
   agatcctagt attccaatta gatctttaat tccaaattgg gccagtaata ttttgctatt 10800
   aatattattt ttattgttac caaatgcaca cacaaagaaa ctccgacatg atgcacgatt 10860
   tttttattgg ttattggtta attacttact taaaataagg ttgttcgcat gtgatatcaa 10920
   atagaggaaa atcatataga aaaaaattat ctcctttact ttgggttaca agtttgggta 10980
   ttacatgaaa atcatgtgta gatccttctt ttgctcccta tactgctcta tcacttgtct 11040
   tgttaagaaa actagaacat gaggcgcccg taagggcgcc ctatcaggac taccgtatac 11100
   aaagctgaac aatagttata aggacactaa agaaaggttc atgtatgtga atcacatggt 11160
   aacaggagta gtgatgatcg ttctttattt taccaagatc gtcatccgta gccttggact 11220
   tctctagagg caacctaaac cacaacttga aagaggcaga tgaaaaatat atcattcaga 11280
   tcagatataa gcatatctag aacaggataa actgaggaaa cgaggagtga gacatgaaag 11340
   gcaatacatt catatttgtc aggccttgca aaatttgttt cacaactcta gcaagatact 11400
   cgatagggag ggactacctc tgtgcaaaca tggggttgtt tgcataagta gcagaccata 11460
   ccagtccatt taacatgtgt ttagaaatta ggatttcctt tgcacaagaa ttgatcaaca 11520
   tgtacattgg ccacccgtag caattaaaac ttcacctaga gacaaattgt agaatgtgat 11580
   ttatttatat gtagagtaga gctataagtt tctcgttgga tactttatct gtcctaaaat 11640
   aggtgtatat cttgcactcc gagtcgacaa attatatatt tgattaagta tatgaaaaaa 11700
   cattaatatt tatgatatca aaacattaat atttatgata tcaaatagac atcattaggt 11760
   gagtcacatt gtgtgtagta aagatatttt aagatagaaa tattaatact atttttctaa 11820
   aaatctagtc aaagttgaga atgtctggct tctcaaaatg tgaggtctaa gatgtgcacc 11880
   tattgtagga tggagtaact atatagggta cataaaacaa acaaacaacg tagatatacc 11940
   attttaaatc gaaacacaag taaaaacaaa cacctcgatt gacgaatccc atttcttagt 12000
   aaattagttg gtggatgtgt catgtacggt aagccgtaca caaggcgcag gcgcgctcgc 12060
   gacatccggc gggcagcgga gcgcgccaac agtgcagcag cgacgcagat ggcaggcact 12120
   caagtttagg aaagcagcag ccacctatgg caagggataa ataggaaagt atcaatttgg 12180
   ctcttatggc aaggaataag taggaaagta gcaatttagc acttatggca aggaataagt 12240
   aggaaagtag tattttggca gggaagataa ttagggagct tgctccattt gtgtggccgc 12300
   cggccctata tatccccaag tgcgcagcac tgttgagggc aataaagtat caagtcatct 12360
   cccacctctc tctctcaagc ctgcggttag gaggaatact cctaccggag aagacggccg 12420
   aaggctacga gttacgtagc cgaggcccgg cgagtcaagg gttcgacgct cttgacagct 12480
   tggtatcccg agtccggaga tccttgcctc acccagtccc gcccaccgat ccactaacca 12540
   caccaccatc cccgaccacg cccaccagcg gttcgccatc ctccatccac atggccgagc 12600
   ccactttagc cgacgtcatc gagatgatga aggccatgca ggccgacatc accaggctga 12660
   aggagaagtc cgactcctcc tccgcaagcg gcgcccagcc cgagcgtcat cgcgacatcg 12720
   atcgcccgcc aaggttccag aaatgggact ttccgcggtt tgatggcaag tccgatcccc 12780
   tcctcttcat caacaaatgt gagtcgtatt ttcgccagca gcgtacaatg gtggaggagc 12840
   aagtgtggat ggcctcgtat aatctcgagg gcatcgcaca gcactggtac atacaactac 12900
   aggaagacga gggcaccccg tcctggagca gattcaggga actgctcaac ctgcgcttcg 12960
   ggccagccct ccgttcagcg ccgttcttcg agctcaccga gtgtcgacgc accggcacgg 13020
   tcgaggagta ctccaaccga ttccaggagc tcctaccccg tgctggcagg atgacggagg 13080
   agcaacgcgt ccaactctac accggcggcc tgctgccacc gctcagccac gcagtgcgga 13140
   tactcaaccc cggcaacctc aacactgcta tgagcctagc gcggcaggtg gagcagatgg 13200
   agctcgccaa gatcccggca ccagccaaga cagggccgcg cggcatcctg ccgccgccac 13260
   ctccgcggcc agcgactcag gcgctcccgg cgccacaggc ccccttggcg ctgccagcac 13320
   cgccactgtg cgcagcaacg ggccgtggcg acggctccag gcgattatct ccagaggaga 13380
   tggcggatcg gcgccgccaa ggactgtgtt ttaattgtaa cgagaaatat tctcgtggcc 13440
   acaaccgttt ttgcaggcgc atcttcttct tggatggcgt cgaaattgag gaggccgagg 13500
   cggaaacggg tgccgcggag accgacgctc cctgtttttc cctccaggca gtggccgggg 13560
   tcgcgccagg ggacaccatg cagatcgccg tcaccctcgg ttcagtctcc ctggtggcac 13620
   tgctcgactc gggaagcacc cacaatttta tatccgcagc ggcggcgcac cagtcgggca 13680
   tccccatcca gcgccgaccc cgcctcacgg ccatggtggc caatggcgag cggatcacct 13740
   gcgcgggcgt ccttcgcaat gcgccgctcc acgtcaaggg cgaactctac ccggctgacc 13800
   tcttcgtcat gccattggcc gggtacgacg tcgtcctcgg cacacgatgg cttggtgcgt 13860
   tggggccaat cgtgtggaac ctcgccaatc gccgcatgac gttccagcgc caggggcgct   13920
   ccatctcctg gacgggcacg cccagcccca aggagccggc gctgcgcgcc accaacggaa   13980
   cggacgccct actggaggcc cttctcgcct cgttcggggg actcttcgca gctccgatgg   14040
   gtttgccccc caagcgcgcc cacgaccatc gcattctcct caagcccgat gcgtcaccgg   14100
   tggctgttcg gccgtaccgc tacccggctg cacacaagga cgagctggag cggcagtgca   14160
   acgccatgat tgaccagggg atcgtgcgtc gcagcgactc cccattctca tcgcccgtgc   14220
   tcctggtcaa gaagcccgat ggctcgtggc gcttctgcgt cgactacagg gccctcaacg   14280
   ccctcaccat caaggacgcg ttccccatcc cggtcgtgga ggagcttctc gacgagctcc   14340
   acggcgccaa attcttcacc aaactggatc tacggtcggg ctatcatcag gtccggatgc   14400
   ggccagaaga cgtgcacaag acagcgttcc gcacacacga tggcttatat gagttcctcg   14460
   tgatggcgtt cgggctctgc aacgccccag ccacgttcca ggcactgatg aatgacgtgc   14520
   tccggccctt cctccgacgg ttcgtcctgg tattctttga tgacatcctc atttacagca   14580
   gcacgtgggc cgatcacctc cgccatctac gggccgtcct cgacgagcta caacgccatc   14640
   agctgttcat caagcgttcg aagtgttctt tcggagcaac atctgtcgcc tacctcggcc   14700
   acatcatctc ggccgcaggg gtggccatgg acccggccaa ggtggctgcc attcacgact   14760
   ggccgccgcc gcgctcggtc cgggccgttc gagggttcct cggcctcacc gggtactacc   14820
   gccgcttcgt ccacaactac ggcacggtgg ctgccccctt aacggccctc ctcaaaaagg    14880
   atggcttcgt ctgggatgac gcggccatgg ctgcctttac ggcgctcaag gcagccgtgt   14940
   cgtctgctcc tgtcctcgcc atgccagatt tcggcaagcg gttcgtggtg gagtgcgatg   15000
   catcctccac aggcttcggt ggggtgctcg tccaggaggg acacccggtc gcgttcttca   15060
   gccgacctat tgcaccccgc caccgcgacc tcgccgccta cgagcgcgaa cttatcggcc   15120
   tggtccaggc tgtccggcac tggcggccgt acctatgggg acgacacttc atcgtcaaga   15180
   cggaccactt cagtctcaag tatctcctcg accagaggtt ggccacgatt ccccaacacc   15240
   attgggtggg caaacttctg ggattcgatt ttacggtcga ataccgctcg ggcgccacga   15300
   acaccgtcgc cgacgcacta tccaggaggg accccgacga gggcacactg atggccattt   15360
   cagcaccccg tttcgacttc gtcgagcgcc tgcgccaggc acaggctacg gatccggctc   15420
   tcgtcgccct ctacgaagag atccgcgtcg gcacgcgcgc tgcaccatgg tccatcatcg   15480
   gtgggatggt cgccatggag gggcgcctct atatcccagc cacttcaccc ctattacggg    15540
   agatcgtggc cgctgtacat gatgacggcc acgagggcgt ccaccgcacc ctgcatcgac   15600
   tacggcgcga ctttcatttt cccaacatgc gtcgtattgt gcaggaattc gtcagggcgt   15660
   gcggcacatg ccaaacatac aagtccgatc accttcgtcc agctggtctc ctacagcccc   15720
   tgccgatccc aaatggggtg tggtcggaca tcggcatcga cttcatcgaa gctctgcccc   15780
   gcgtgcaagg caagacggtc attctcactg tcgtcgaccg tttcagcaag tactgccact   15840
   tcatcccact ggcgcaccca tacacggcgg agtcggtggc gcaagcattc tgcgccgagg 15900
   tggtgaggct gcacggcatc ccgcagtcca tcgtatcgga ccgagacccc gtcttcacgt 15960
   cggccttctg gagggagcta atggccctga ctggaaccaa gttgttcatg tcctccgcct 16020
   tccatccgca aactgacggc cagtcggagg cagccaaccg cgtcatcatg atgtaccttc 16080
   gctgcttcac cggcgatcga ccccgacagt ggctccggtg gcttccatgg gcggagtaca 16140
   cctacaacac cgcataccag tcctcactcc gcgagacacc tttccgggtg gtctacggcc 16200
   gcgacccacc gaccatccgg tcctacgagc cgggtgacgc acgcgtgccc gcggtggcac 16260
   aggagatgga ggaacgggct gccttcctcg acgacgttcg ctaccgtctc catcaggcgc 16320
   aagaatccca gaaacgcgcc tacgatcagc atcacaggca ggtcgtctac aatgtcggtg 16380
   actgggccct tcttcgtctc cgtcaacgac catcggcatc cctgccacgt tccaccacgg 16440
   gcaagttgaa gccccgctac gtcggcccct accgtgtcaa ggagatcatc aacgaggcgg 16500
   ccgtacgcct tcagcttcca tccgacgcca aaatccacga cgtcttccat gtgggcatcc 16560
   tcaagaagtt cgttggggcg ccaccaacgg aaccacctgc gctaccggcc acactcaacg 16620
   gcgcagtggt gtccacacct tcccgtgtcc tggcgggtcg cctggctcgc ggcattcggc 16680
   aagttctggt acagtggcac ggtgaaccag tgtcgtctgc gacctgggag gattttgagg 16740
   atttcagggc aagcttcccc gctttccagc tcgaggacga gctgactttc gaggccggga 16800
   gagatgtcat gtacggtaag ccgtacacaa ggcgcaggcg cgctcgcgac atccggcggg 16860
   cagcggagcg cgccaacagt gcagcagcga cgcagatggc aggcactcaa gtttaggaaa 16920
   gcagcagcca cctatggcaa gggataaata ggaaagtatc aatttggctc ttatggcaag 16980
   gaataagtag gaaagtagca atttagcact tatggcaagg aataagtagg aaagtagtat 17040
   tttggcaggg aagataatta gggagcttgc tccatttgtg tggccgccgg ccctatatat 17100
   ccccaagtgc gcagcactgt tgagggcaat aaagtatcaa gtcatctccc acctctctct 17160
   ctcaagcctg cggttaggag gaatactcct accggagaag acggccgaag gctacgagtt 17220
   acgtagccga ggcccggcga gtcaagggtt cgacgctctt gacaggatgc gagtcaaaat 17280
   catgtcatag tagattccat ctaactcacc agcacagctc aaacaaaaaa tgtaatggac 17340
   aataaggtta ttccaaaatt ctaattccag agtctgatca ccttcgtatg ggtagtcccg 17400
   atagcaacga aactctcctt ggtggtggcc tcatcgtctg gtgtcttgca tggcagcatg 17460
   ctgaatgtcc agccttcaag gatgcatgtc ctctgggtca tccccttaaa caccatcagt 17520
   tgtaggaaga tgaacctcag aagcatcaac tctgcatcac acagagacaa agaggtaagg 17580
   tttgtgccgt aaacatatgt atcttatccg gaatcataga gaaaaggaaa ggaagactga 17640
   gggtgtgttt ggattattgc atacctgaaa aaagttacgt cttgttgctg tcaacatttt 17700
   taaatagcca accgctcagt ttcattttgg ttcaattgta cgaaggacaa tcagtgtccg 17760
   gtgttcttga tagtcaaggc ttagggcata caagttttat cctcgtaaaa ctgactttac 17820
   cttacattgt tttgccatcc ttttttatct gtattatatc gttatctatg atttacttat 17880
   cttgaagcga aaatataaag attgggaaat ggaggcacct tctttgacct tgagcagctc 17940
   ctcgaagagc ggcttttggt tcttcctctt gagcgtctac aggacacatc atcgaagcgc 18000
   tcctcgaagg cgacccctta tcttgaagcg aaaatataaa gattgggaaa tggaggcacc 18060
   ttctttgacc ttgagcagtt cctcgaagag cggcctctgg ttcttcctct tgagcgcctg 18120
   caggacacat catcaaagcg ttcctcgaag atgacctcac cggaagcgag gaacaacaac 18180
   gacaagaggg cgaggaggca atagagcacg ccgccacgct ggggcgtgga gcccgcggtg 18240
   gccatgcgcc cgcacgtgct caaggtccca agcagtgggg atgtcgcgcg ccgccgcggc 18300
   ctcgggatct gcatgctcgt ggggacgggc gccacgtcgt cgtctcgcgc tccggctccg 18360
   tccgcggagg aggctatgcg accgtctttc gtggccagta cgagatccta tccacctcgg 18420
   tcgccgttgc cgccatcccc tgttccctag agcgttccac gaaggtgaag gaaacccttg 18480
   atgcaggtgg gaagagatcg cagacatcaa ggtaggtgga gggagcaata gcactgtagc 18540
   agtagcatac tactcggctt ggctgttgac tgttgaggag gctagaactt ttcggcacgg 18600
   caagtttttt aatataagga ttcatgagag gacatgcatg ccgaaatgtt tgaccagctg 18660
   aacaattcgc gttcgtggtc gctttccaca cgtcgggacc ggtgtgggga aaatcacgcg 18720
   aaacgcggag agcgttccaa aacccatcgt attttaatac tttagatggc tttcattgtt 18780
   gaccttccag gcatgaaacc aaattggttc aaagagatcc tcgttattgc tttcaaacga 18840
   tgcttgataa ctctccccca tagcttcata gtatgactca tcaacttaat tccttggtaa 18900
   ttattacagc tttaaatatc tcccttattc ttgtaaatcg gtacaaataa tacttctcca 18960
   cttgttaggc gtattgttcg atcgaaagat atggttgaac aacttggtta gccatactat 19020
   agcaatatcc tcgaggcatc ttcatacctc aattggaata tcattaggat ccatcacctt 19080
   accccctttc attcttttca aagcctccct aaccttagat tcttgaatct tacgcacaaa 19140
   gagcctattg gtgtcatcaa aagagtcatc caactgaaag gttgtgtccc cattctcccc 19200
   attgattaat ttgtcaaagt actcttacca tatatgcctg atctcgtcct ccttcaccaa 19260
   gaggcgctct gtttcatcct taatgcactt aactcgattg aggtctcttg tcttcctgtc 19320
   acgaaccctg gctatcctat atctgccctt ctctcctttc ttcgtactca agcattggta 19380
   aagatcctta gacgcactat tctttgctac catgtatttt tctatgctat ccacactcct 19440
   acctactctt ataaagcacc agtttccttg gtttccacca ttgtccccaa ggttttaaag 19500
   tctggcaact agtcgagaag tcgatctgaa cgactcgact ttctctacta gtcgctcgac 19560
   tcgttgacta gtcacgagta gtaacgacta gtcgcagcga ttgagtcggg tgacttattt 19620
   gggcctaggt ataaaaatgt gagcccatcc agccatctca accctagtag gtatgagatg 19680
   gaatgagatg gtatgaaatg ctaatagaca agttttctac cttatttatc tatgatatgg 19740
   aatgattcat gacttgatga ggccaagaca aatggttgag atcttgtgag gcatgcaact 19800
   atgttttgca actactatgt ttgcaacatc cttccttaca ttggctaata tgtggaaaca 19860
   aagacaagga tcgaaagctc tctttgttag taatgaatgg tgtgagagca aaatgaagaa 19920
   cactgaagta ggaaatgcac gtgttgtttg tcactctgtt gcattttggc aagcagtaga 19980
   agattgcatg agagcttcac aacctattct aatattcctg aggatagttg atggtgatga 20040
   gagactggca atggctatat ggtgggctgc catggaccat gaaaaaagag catcaaaaaa 20100
   gctttggagc acatagaatc atagaaagaa taatagatga agtaatttct attgttgata 20160
   aggggtggga cagtcagatg gataccaatc tgtatagagc aacattgttt ttggatccaa 20220
   acaagttctt tgaaaccaaa gagaacgata gaagacttac tacaatgcta tattcaatat 20280
   ttaatgatat attgtagaag atggagatgg atgaaaatat actaaacaag gtgtttttag 20340
   tgttctagca tgtttttagt cttagcgtat gtagaaagtt ataaaaatat aatcactgac 20400
   ttatgttcga ctagtcacga ctagtttgtc ctagtctcga gtctctacta gtcagtagtt 20460
   gcacgactcg actcgaaaac atcgattgtg tgggaaaccc taaccctaac aaggggttgg 20520
   gtgatgtatt aatagactgg gtagttgggc ctgacccatt acacaggggt gagaaggtaa 20580
   atacacgggg tagaccccaa accctaaacg agtactctaa tactctaaca gattgtccct 20640
   tttgcaaaaa aaggtcctta tacttctttc taatctaact gtagcccaac ttatagatct 20700
   tgatttcctc ccccatgtct tgcctaacaa ccaaataagt gtgtgggtgt gtcacgctcg 20760
   aagcgtttag atctaatttc ctctccttgt ctggcgtaac aactagataa gataagtctt 20820
   accccttgca tgcacaacca accaccacga gtgcttcctc ctcctcgtta ttggtgtcgt 20880
   atattaaccc ctaacatcac atctctttcc ccatccctgc ctttctcacc caaattcttc 20940
   cgtggaggac atgcagtagg ccatcctgct ggcctccaac tcgccagtcg ctgtctccgc 21000
   gccctaccgg cgccgccgcc tttaccttcc cccgtcggca agcgcctcga tcttgtagcg 21060
   cctcctctct ttgccgccat gcgcctttgc agtcagcggg gcaagaacaa ccgcgggaga 21120
   gtaggaggcg ttgccgccgc tggcggtgcg tggccggaca attgagggag aggaggcgct 21180
   ctagattccg agtttagttt tgtattggtt gttggcaaaa aaattgtgag atattaaggt 21240
   cgatgataat atatgtattg ctcgttttca tgtaatcgtt gtgcgctaat attttatcga 21300
   atatttttgt gccgtcacaa cacacgtgca ttatactagt catggtacaa tgttagcatt 21360
   tttttcttct ccttaatagc cctttagact tcctcattcc actaccaagt atctttagct 21420
   tcgcctctat tctctctagt tactccacac acctcttagg tggcctttcg aatgcaggtt 21480
   ttcatcttct cccacatgtt gtttgtgtca tcttccttcc aagtgtcctc tttgagaagc 21540
   aagatgacac aaacattgaa cttgcattgt attttatggt gtttggcatg tgaattcatt 21600
   tagctgagag ttgattgaat tggactacaa ttggacttcc gtttttttac acacatttgg 21660
   gaggggggcg ggagggcaga cccacccagt ggtggaggtt cccacatgag tggggttggg 21720
   gaagggataa accaaggcaa gccttcccct cgctaatacg gagatgctga ttcgaacccg 21780
   tgtcctcgtg actcactggg acatctctca ccactgcact aggcctgcac tttatttgtt 21840
   tttctcattt ttttttgttt tcctaacttg ttcatagtta ttgtacttcc cgctttttta 21900
   cacgttacag gaattgttgt tttttgttct tttgaaggta attgcaatag tagtttatgt 21960
   agcttgtcaa ctgtattctc actgcttaca tgtgctttct acacaggatt tcttacatga 22020
   gatgtcacgg aaggacgaaa aagttgattc cttagttaaa atggggtttc ctgaagacga 22080
   ggctgcactg gctattacca gatgcggtgt gctctctgag actttgttcc attctcgtct 22140
   ctttgcatta gattcctcag caaaataaac atcaatttcc aaccttgaaa gtaggatatg 22200
   ctctcatata gaataaatca gttgaataat aacattgcca tgtttaatta tatgtcaagc 22260
   tttctattgg tttactctct ggattttgag aattctcttc aaatgctgta acttggaact 22320
   cgtaaattgg ctgcaatctt gacctgaggt aacctagaat ggttgaatct ttccaagctt 22380
   gatttactgt ttgcatcaag tttgggttaa ccagttatat tcaaatctga agacaaagca 22440
   tttaggcatg cccataataa tctgtagaaa gcaaggcgta tgataaatat tatttcttac 22500
   aagaataact gggtgtttag gaagttatcc atccaagtag tacatgtaaa cctttgtttc 22560
   tatgagtagc aagcattcag atgctccttc aaagcagtca acgaacagtt tattgttcgt 22620
   ttggggtcag aatattttaa tggcatttgc tgttaaataa aacctttttt tgcatcttgc 22680
   atgcaacttg taccgctcag attctgcccc cgtccctgtt ttattttaac tggccatgca 22740
   ccttacgcca tctactgtgg tctgtggttg aaaattactc agggccggat gcatctattt 22800
   ctgttctggt ggattcaatc tatgcttcac agaccgcagg agatggttac tgtggcaatc 22860
   tgtctgacta tgaggtttgt cttactgtcc atacaagtct gcaataagat ttagttctat 22920
   tataatggcc tggtaatgga actgtgtctg caatactgca ggataattcc tatggaggga 22980
   gaagcacagg gaacaagaaa aagagaaaaa gatatggagg ccaagcacag ggaagtagag 23040
   gcccattaga tggtagctgt gatgaaccca tgcctctccc acatccaatg gttggattta 23100
   acttgccaga ccagtggtca agacgagtgg acagatcgtt gcctgcacaa gctattggtc 23160
   cgccgtactt ctactacgag aacgttgctc ttgctccaaa aggtgtctgg actaccatat 23220
   caagattctt gtatgatatt caaccagagt ttgtggactc aaagtacttc tgtgctgctg 23280
   ccaggaaaag gggttacata cacaacctgc cacttgagaa caggtcacct ctcctcccca 23340
   tacccccaaa gacgatatcg gaagcatttc ctcggaccaa gaggtggtgg ccttcatggg 23400
   acccaagacg acagttcaat tgcctccaga cttgcgtgtc tagtgcaaaa ttgttagaga 23460
   ggattcgcgt agccctcaca aacagttcag acccacctcc tccaagagtt cagaagtatg 23520
   tgttggagga gtgtaggaaa tggaacctgg catgggttgg cttaaacaag gttgctcctc 23580
   tagagcctga cgagatggag tttctactcg gctttccgaa ggatcacacg agaggtatca 23640
   gcaggacaga gaggtatcga tctctaggaa attcatttca ggtcgatact gttgcttacc    23700
   atctctcagt tctgaaggat ctgttcccac aaggcatgaa tgtgctgtct ttattctctg    23760
   gtattggagg agcagaggtg gctctccaca ggcttggcat acagatgaac acggttattt    23820
   cagtggagaa gtccgaggtc aaccggacga ttctgaagag ttggtgggat cagacgcaga    23880
   cgggtactct gattgagatc actgatgtgc agacactgtc atctgagagg atcgaggcgt    23940
   atattagaag aattgggggc ttcgatcttg tgattggtgg aagtccctgt aacaacctca    24000
   ctgggagcaa ccgtcaccac agagatggtt tggagggcga gcattctgca ttgttccatc    24060
   attattttag gatcttacac gccgtcaagt ccatcatgga gcgtttgtag ttcttagaat    24120
   ctttactgtt gttttggttc tagtagaata ctttgtgacc gaacttgtaa atggtttgca    24180
   ggttgactgg tttagttctc ttgccactct aatttaggct agtttttttt ttatttaatc    24240
   ttctttggtg atcttggtgc agttctgtgg cactgttggg tattcaaatg cagattgatc    24300
   cgagaggctg ccctgttgca ttttcttttt aaaatttgta tttgtcaatg agagatcagg    24360
   tattctggtt gccaaaaaaa aatctgaagt tgtggagctg agaaaaggac ggtgctaagc    24420
   tacaaccacc cactgccaca ccatgcgcac ccccaaccca aaagatcctg cttttgctca    24480
   cgcttatcac cactcccatc tccatccaaa accataaaaa aaaccagtgc gagctgaagc    24540
   atccaggctc tggaatcatc gtcgggatgt ctgtgccagc atcagcccag gtgcctcaca    24600
   ccatacgatc tgctgggagc tgctgcttca ggtcgtcgca tcaactatct gcgcagaatc    24660
   tgggccgacg tgagttcccg atctcagctt tgagtggcct tctttttgaa agtgagcaca    24720
   ggttcttcca aagcccgttg tgttcgtgta gatcgcaggt ggcgggggac cggaaagaag    24780
   aagatccttt ccacccgtgg cgtgcttgag tccagaaacg gcgcacatcc tggtggcctt    24840
   atgaagagaa ggtgagaacc tggtgaaaga catgcggagc tgtagctaga cagctagctg    24900
   atctagtagc agcgcacgcc ttgcaggaag gtcgtggagc acatcatcct gctcagagcc    24960
   aagcccaaca tctcagacgc tgaagagaaa gacatgctgg actacctata cacatcg       25017
<210> 3
   <211> 2353
   <212> DNA
   <213> Artificial sequence
<220>
   <223> Methyltransferases
<400> 3
   gagtcaccct gtcgccgttg ccgtcgccga ggcaggcaga gtctccctgt cgccgttgcc      60
   gtcgccggcc tcctcctcct ctgccacttc ctcccaactc ccagccgcag gggccgacgg     120
   cgacgggagg gaagaggcgg cagaggtact ccgaggggcg cggagagagg ctgtgccagg     180
   ccacgccgtc cagtgcctcc gggtccgtcg ccgcagcttc cggccgcgtc ggagaggtag     240
   cccccggagc tcttcgcgga ggctcgggaa tggtgcactg ggttagcgac agtgatggca 300
   gtgataactt cgaatgggac agtgatggta acggggagca gacagtgagc ttcaacgctg 360
   ctggtgctgg ttcatcagct ctggcagcga cgaacactga tgctcctggc ccatcgacac 420
   gggttgctaa tggcaatggg aaggctgggc gatctgcctc tttggttcag aagtatgtgg 480
   acatgggttt ctcagaagag attgttctga aggccatgaa ggacaatggg gataatggag 540
   cagattcatt agttgagctc cttcttactt accaggaact aggcaatgac ctcaaagtgg 600
   ataatgactt tgcttctagt tgtgccccca aaactgctga cgatagtgat gatgatgaca 660
   cactggaaat ctgggatgat gaggatgctg gagggagaag caccagggtt gctaactctg 720
   ttgatgattc tgatgacgag gatttcttac atgagatgtc acggaaggac gaaaaagttg 780
   attccttagt taaaatgggg tttcctgaag acgaggctgc actggctatt accagatgcg 840
   ggccggatgc atctatttct gttctggtgg attcaatcta tgcttcacag accgcaggag 900
   atggttactg tggcaatctg tctgactatg aggataattc ctatggaggg agaagcacag 960
   ggaacaagaa aaagagaaaa agatatggag gccaagcaca gggaagtaga ggcccattag 1020
   atggtagctg tgatgaaccc atgcctctcc cacatccaat ggttggattt aacttgccag 1080
   accagtggtc aagacgagtg gacagatcgt tgcctgcaca agctattggt ccgccgtact 1140
   tctactacga gaacgttgct cttgctccaa aaggtgtctg gactaccata tcaagattct 1200
   tgtatgatat tcaaccagag tttgtggact caaagtactt ctgtgctgct gccaggaaaa 1260
   ggggttacat acacaacctg ccacttgaga acaggtcacc tctcctcccc atacccccaa 1320
   agacgatatc ggaagcattt cctcggacca agaggtggtg gccttcatgg gacccaagac 1380
   gacagttcaa ttgcctccag acttgcgtgt ctagtgcaaa attgttagag aggattcgcg 1440
   tagccctcac aaacagttca gacccacctc ctccaagagt tcagaagtat gtgttggagg 1500
   agtgtaggaa atggaacctg gcatgggttg gcttaaacaa ggttgctcct ctagagcctg 1560
   acgagatgga gtttctactc ggctttccga aggatcacac gagaggtatc agcaggacag 1620
   agaggtatcg atctctagga aattcatttc aggtcgatac tgttgcttac catctctcag 1680
   ttctgaagga tctgttccca caaggcatga atgtgctgtc tttattctct ggtattggag 1740
   gagcagaggt ggctctccac aggcttggca tacagatgaa cacggttatt tcagtggaga 1800
   agtccgaggt caaccggacg attctgaaga gttggtggga tcagacgcag acgggtactc 1860
   tgattgagat cactgatgtg cagacactgt catctgagag gatcgaggcg tatattagaa 1920
   gaattggggg cttcgatctt gtgattggtg gaagtccctg taacaacctc actgggagca 1980
   accgtcacca cagagatggt ttggagggcg agcattctgc attgttccat cattatttta 2040
   ggatcttaca cgccgtcaag tccatcatgg agcgtttgta gttcttagaa tctttactgt 2100
   tgttttggtt ctagtagaat actttgtgac cgaacttgta aatggtttgc aggttgactg 2160
   gtttagttct cttgccactc taatttaggc tagttttttt tttatttaat cttctttggt 2220
   gatcttggtg cagttctgtg gcactgttgg gtattcaaat gcagattgat ccgagaggct 2280
   gccctgttgc attttctttt taaaatttgt atttgtcaat gagagatcag gtattctggt 2340
   tgccaaaaaa aaa 2353
<210> 4
   <211> 1812
   <212> DNA
   <213> Artificial sequence
<220>
   <223> Methyltransferases
<400> 4
   atggtgcact gggttagcga cagtgatggc agtgataact tcgaatggga cagtgatggt      60
   aacggggagc agacagtgag cttcaacgct gctggtgctg gttcatcagc tctggcagcg     120
   acgaacactg atgctcctgg cccatcgaca cgggttgcta atggcaatgg gaaggctggg     180
   cgatctgcct ctttggttca gaagtatgtg gacatgggtt tctcagaaga gattgttctg     240
   aaggccatga aggacaatgg ggataatgga gcagattcat tagttgagct ccttcttact     300
   taccaggaac taggcaatga cctcaaagtg gataatgact ttgcttctag ttgtgccccc     360
   aaaactgctg acgatagtga tgatgatgac acactggaaa tctgggatga tgaggatgct     420
   ggagggagaa gcaccagggt tgctaactct gttgatgatt ctgatgacga ggatttctta     480
   catgagatgt cacggaagga cgaaaaagtt gattccttag ttaaaatggg gtttcctgaa     540
   gacgaggctg cactggctat taccagatgc gggccggatg catctatttc tgttctggtg     600
   gattcaatct atgcttcaca gaccgcagga gatggttact gtggcaatct gtctgactat     660
   gaggataatt cctatggagg gagaagcaca gggaacaaga aaaagagaaa aagatatgga     720
   ggccaagcac agggaagtag aggcccatta gatggtagct gtgatgaacc catgcctctc     780
   ccacatccaa tggttggatt taacttgcca gaccagtggt caagacgagt ggacagatcg     840
   ttgcctgcac aagctattgg tccgccgtac ttctactacg agaacgttgc tcttgctcca     900
   aaaggtgtct ggactaccat atcaagattc ttgtatgata ttcaaccaga gtttgtggac     960
   tcaaagtact tctgtgctgc tgccaggaaa aggggttaca tacacaacct gccacttgag    1020
   aacaggtcac ctctcctccc cataccccca aagacgatat cggaagcatt tcctcggacc    1080
   aagaggtggt ggccttcatg ggacccaaga cgacagttca attgcctcca gacttgcgtg    1140
   tctagtgcaa aattgttaga gaggattcgc gtagccctca caaacagttc agacccacct    1200
   cctccaagag ttcagaagta tgtgttggag gagtgtagga aatggaacct ggcatgggtt    1260
   ggcttaaaca aggttgctcc tctagagcct gacgagatgg agtttctact cggctttccg    1320
   aaggatcaca cgagaggtat cagcaggaca gagaggtatc gatctctagg aaattcattt    1380
   caggtcgata ctgttgctta ccatctctca gttctgaagg atctgttccc acaaggcatg    1440
   aatgtgctgt ctttattctc tggtattgga ggagcagagg tggctctcca caggcttggc 1500
   atacagatga acacggttat ttcagtggag aagtccgagg tcaaccggac gattctgaag 1560
   agttggtggg atcagacgca gacgggtact ctgattgaga tcactgatgt gcagacactg 1620
   tcatctgaga ggatcgaggc gtatattaga agaattgggg gcttcgatct tgtgattggt 1680
   ggaagtccct gtaacaacct cactgggagc aaccgtcacc acagagatgg tttggagggc 1740
   gagcattctg cattgttcca tcattatttt aggatcttac acgccgtcaa gtccatcatg 1800
   gagcgtttgt ag 1812
<210> 5
   <211> 915
   <212> PRT
   <213> Artificial sequence
<220>
   <223> Methyltransferases
<400> 5
   Met Ala Pro Ser Ser Pro Ser Pro Ala Ala Pro Thr Arg Val Ser Gly
   1               5                   10                  15
   Arg Lys Arg Ala Ala Lys Ala Glu Glu Ile His Gln Asn Lys Glu Glu
               20                  25                  30
   Glu Glu Glu Glu Glu Glu Val Val Ala Ala Ser Ser Ala Lys Arg Ser
           35                  40                  45
   Arg Lys Ala Ala Ser Ser Gly Lys Lys Pro Lys Ser Pro Pro Lys Gln
       50                  55                  60
   Ala Lys Pro Gly Arg Lys Lys Lys Gly Asp Ala Glu Met Lys Glu Pro
   65                  70                  75                  80
   Val Glu Asp Asp Val Cys Ala Glu Glu Pro Asp Glu Glu Glu Leu Ala
                   85                  90                  95
   Met Gly Glu Glu Glu Ala Glu Glu Gln Ala Met Arg Glu Glu Val Val
               100                 105                 110
   Ala Val Ala Ala Gly Ser Pro Gly Lys Lys Arg Val Gly Arg Arg Asn
           115                 120                 125
   Ala Ala Ala Ala Ala Gly Asp His Glu Pro Glu Phe Ile Gly Ser Pro
       130                 135                 140
   Val Ala Ala Asp Glu Ala Arg Ser Asn Trp Pro Lys Arg Tyr Gly Arg
   145                 150                 155                 160
   Ser Thr Ala Ala Lys Lys Pro Asp Glu Glu Glu Glu Leu Lys Ala Arg
   165 170 175
   Cys His Tyr Arg Ser Ala Lys Val Asp Asn Val Val Tyr Cys Leu Gly
   180 185 190
   Asp Asp Val Tyr Val Lys Ala Gly Glu Asn Glu Ala Asp Tyr Ile Gly
   195 200 205
   Arg Ile Thr Glu Phe Phe Glu Gly Thr Asp Gln Cys His Tyr Phe Thr
   210 215 220
   Cys Arg Trp Phe Phe Arg Ala Glu Asp Thr Val Ile Asn Ser Leu Val
   225 230 235 240
   Ser Ile Ser Val Asp Gly His Lys His Asp Pro Arg Arg Val Phe Leu
   245 250 255
   Ser Glu Glu Lys Asn Asp Asn Val Leu Asp Cys Ile Ile Ser Lys Val
   260 265 270
   Lys Ile Val His Val Asp Pro Asn Met Asp Pro Lys Ala Lys Ala Gln
   275 280 285
   Leu Ile Glu Ser Cys Asp Leu Tyr Tyr Asp Met Ser Tyr Ser Val Ala
   290 295 300
   Tyr Ser Thr Phe Ala Asn Ile Ser Ser Glu Asn Gly Gln Ser Asp Ser
   305 310 315 320
   Asp Thr Ala Ser Gly Ile Ser Ser Asp Asp Val Asp Leu Glu Thr Ser
   325 330 335
   Ser Ser Met Pro Thr Arg Thr Ala Thr Leu Leu Asp Leu Tyr Ser Gly
   340 345 350
   Cys Gly Gly Met Ser Thr Gly Leu Cys Leu Gly Ala Ala Leu Ser Gly
   355 360 365
   Leu Lys Leu Glu Thr Arg Trp Ala Val Asp Phe Asn Ser Phe Ala Cys
   370 375 380
   Gln Ser Leu Lys Tyr Asn His Pro Gln Thr Glu Val Arg Asn Glu Lys
   385 390 395 400
   Ala Asp Glu Phe Leu Ala Leu Leu Lys Glu Trp Ala Val Leu Cys Lys
   405 410 415
   Lys Tyr Val Gln Asp Val Asp Ser Asn Leu Ala Ser Ser Glu Asp Gln
   420 425 430
   Ala Asp Glu Asp Ser Pro Leu Asp Lys Asp Glu Phe Val Val Glu Lys
   435 440 445
   Leu Val Gly Ile Cys Tyr Gly Gly Ser Asp Arg Glu Asn Gly Ile Tyr
   450 455 460
   Phe Lys Val Gln Trp Glu Gly Tyr Gly Pro Glu Glu Asp Thr Trp Glu
   465 470 475 480
   Pro Ile Asp Asn Leu Ser Asp Cys Pro Gln Lys Ile Arg Asp Phe Val
   485 490 495
   Gln Glu Gly His Lys Arg Lys Ile Leu Pro Leu Pro Gly Asp Val Asp
   500 505 510
   Val Ile Cys Gly Gly Pro Pro Cys Gln Gly Ile Ser Gly Phe Asn Arg
   515 520 525
   Tyr Arg Asn Arg Asp Glu Pro Leu Lys Asp Glu Lys Asn Lys Gln Met
   530 535 540
   Val Thr Phe Met Asp Ile Val Ala Tyr Leu Lys Pro Lys Tyr Val Leu
   545 550 555 560
   Met Glu Asn Val Val Asp Ile Leu Lys Phe Ala Asp Gly Tyr Leu Gly
   565 570 575
   Lys Tyr Ala Leu Ser Cys Leu Val Ala Met Lys Tyr Gln Ala Arg Leu
   580 585 590
   Gly Met Met Val Ala Gly Cys Tyr Gly Leu Pro Gln Phe Arg Met Arg
   595 600 605
   Val Phe Leu Trp Gly Ala Leu Ser Ser Met Val Leu Pro Lys Tyr Pro
   610 615 620
   Leu Pro Thr Tyr Asp Val Val Val Arg Gly Gly Ala Pro Asn Ala Phe
   625 630 635 640
   Ser Gln Cys Met Val Ala Tyr Asp Glu Thr Gln Lys Pro Ser Leu Lys
   645 650 655
   Lys Ala Leu Leu Leu Gly Asp Ala Ile Ser Asp Leu Pro Lys Val Gln
   660 665 670
   Asn His Gln Pro Asn Asp Val Met Glu Tyr Gly Gly Ser Pro Lys Thr
   675 680 685
   Glu Phe Gln Arg Tyr Ile Arg Leu Ser Arg Lys Asp Met Leu Asp Trp
   690 695 700
   Ser Phe Gly Glu Gly Ala Gly Pro Asp Glu Gly Lys Leu Leu Asp His
   705 710 715 720
   Gln Pro Leu Arg Leu Asn Asn Asp Asp Tyr Glu Arg Val Gln Gln Ile
   725 730 735
   Pro Val Lys Lys Gly Ala Asn Phe Arg Asp Leu Lys Gly Val Arg Val
   740 745 750
   Gly Ala Asn Asn Ile Val Glu Trp Asp Pro Glu Ile Glu Arg Val Lys
   755 760 765
   Leu Ser Ser Gly Lys Pro Leu Val Pro Asp Tyr Ala Met Ser Phe Ile
   770 775 780
   Lys Gly Lys Ser Leu Lys Pro Phe Gly Arg Leu Trp Trp Asp Glu Thr
   785 790 795 800
   Val Pro Thr Val Val Thr Arg Ala Glu Pro His Asn Gln Val Ile Ile
   805 810 815
   His Pro Thr Gln Ala Arg Val Leu Thr Ile Arg Glu Asn Ala Arg Leu
   820 825 830
   Gln Gly Phe Pro Asp Tyr Tyr Arg Leu Phe Gly Pro Ile Lys Glu Lys
   835 840 845
   Tyr Ile Gln Val Gly Asn Ala Val Ala Val Pro Val Ala Arg Ala Leu
   850 855 860
   Gly Tyr Cys Leu Gly Gln Ala Tyr Leu Gly Glu Ser Glu Gly Ser Asp
   865 870 875 880
   Pro Leu Tyr Gln Leu Pro Pro Ser Phe Thr Ser Val Gly Gly Arg Thr
   885 890 895
   Ala Gly Gln Ala Arg Ala Ser Pro Val Gly Thr Pro Ala Gly Glu Val
   900 905 910
   Val Glu Gln
   915
<210> 6
   <211> 10088
   <212> DNA
<213> Artificial sequence
<220>
   <223> Methyltransferases
<400> 6
   ccggtgtgca cccgacaggg ggacaatttg tttttcaaaa caaactagtg tatgctccta 60
   ctctgttagc tgtacttaac attgttaagt gttgataaaa atacaagaga taattgcgcc 120
   aatgcccctg ttttagcgca attgtattct actatatttt tagacttaca tgttttatga 180
   caattcaaat taatcccaca acgtttcaaa ttattttaac aacaattcaa actatttgca 240
   acaatttaaa ttatttttaa agatttaaaa atattgacaa attattccat caacatttta 300
   aattattttg cctcatcaca ctaatttaat tagttagata ttttttaact ttgaaaataa 360
   taatttgaat tgtcataaaa gcggacaaag tctaaaaaaa gcggtaaaac tatagttaca 420
   caacaaaaca gtgacaatgg cgcaattgtt tcttgtcttc tttatcatga cttaacactg 480
   ttaagtataa ccaacggagt atgggcatac atcgttttat tttgaaaaac aggggcaaac 540
   gcgcaaagtt ttaaaaaggg ggtaattcta cagttacacc ctaaaacagg gacaccatca 600
   caattgttcc ttgttttttt agtaaagttt tgttcatgtc acagctgatg tatgaatatc 660
   aattgtaagt actaaacata gtataattac aaaactaatt acacaaacaa agactaaatt 720
   gtaagacgaa cctgttaagt ctaattaatt catgattaac aaatgtttac tgtaatatca 780
   tgttaccgaa tcataaacta atcagattta atatgttttc cttattatgt gtgtaattag 840
   ttttataatt atattatatt taatatttga aatgtgacaa tactaaagct aagcctctta 900
   agcccaacaa tccacatatt ttagatttaa aatttgaaaa cagacgtatc ggctagaaga 960
   gccctgtcac tgtcagctaa tcaattacaa gaagtggccc atactagttc catcaccagt 1020
   ccagtagtcc accaccccac cctacagctg ggtcatctgg cacgggtgga ggggccaacg 1080
   gccgaaagcg ccgcgcactt ccggcgggca ccctcgcgga gtcgcgggtg acagcgaaat 1140
   ttcaaatcca taccctcccg ctgcagacgg gtcccacgcc gtcaaaattt ggacgctccc 1200
   gctccctcga tcttttgggt ttcgttttcc cagttcccac cctctcttcc accctgccct 1260
   gtttccagat ttgaccgatc cccttcgatt cgatttctac acctccagag cactcactct 1320
   cctggcaaac cccttcgtct cccaacccta gagagcagca gcagctaccg cagcccctgc 1380
   catggcgccg agctccccgt cacccgccgc gcccacacgc gtctctgggc ggaagcgcgc 1440
   cgccaaggcc gaggagatcc accagaacaa ggaggaggag gaggaggagg aggaggtcgt 1500
   ggcggcgtcc tccgccaagc gcagccgcaa ggcggcatct tctgggaaga agcccaagtc 1560
   gccccccaag caggccaagc cggggaggaa gaagaagggg gatgccgaga tgaaggagcc 1620
   cgtggaggac gacgtgtgcg ccgaggagcc cgacgaggag gagttggcca tgggcgagga 1680
   ggaggccgag gagcaggcca tgcgggagga ggtggttgcg gtcgcggcgg ggtcacccgg 1740
   gaagaagagg gtggggagaa ggaacgccgc cgccgccgcc ggcgaccacg agccggagtt 1800
   catcggcagc cctgttgccg cggacgaggc gcgcagcaac tggcccaagc gctacggccg 1860
   cagcactgcc gcaaagaagt acagtttttc tcccagctct ggttttgatt tgaccagatt 1920
   tttgctccat gtctattagt acttgtgagc cgagcaatct gctatttgct gatttattgt 1980
   gcgtgcagac cggatgagga ggaagagctc aaggccagat gtcactaccg gagcgctaag 2040
   gtggacaacg tcgtctactg ccttggggat gacgtctatg tcaaggtcct tgttcatcgc 2100
   tttctgttgc ttctgctctc atttatgatg tgcatatgtg tttgttaagg aagcaagaat 2160
   tgcttgattt ttgttgccga ctcgcatttc cgtgacgagt tctgcgtatg gtcaccggta 2220
   cgtggcactg atacacaacg tggtatgctg gaagtctggt agtatatttt gcatcgacca 2280
   ggaggtccag atcgatatgt gcggtatagt gcttatttga ttgcaccctg ttcggagatt 2340
   catgcatgat ggcgtgttta gatgacgcct cccagacagc tgcctgctag gcagctgatt 2400
   ctggcccagg cgtccggaat ggtgaagttg cgctggcaag attctcaggc cacctaccaa 2460
   atatgccctg gagcatattg catgcttctt tttttgttct ctttccttct atatttatct 2520
   cattgttagt gaagtttcac attgcgcgtg tcatggaata tttactttca aatcaacgag 2580
   gagatgctag cattgaggtg tgtgataatt attacatact agaagatatc gtgcatgttg 2640
   ccattgggat tgcgaagaat gtggaaatga tgttggtagc ttgtattaag aggttaacag 2700
   ttagtgggat gacatgacta ttagttagag atgatgtgga tagtaagtgg gatatgatgt 2760
   agatgacttg tgtgttgaga cagaactata acatggagtt ggaaatggga gcagcatggt 2820
   caaacatacc ctaaatgcct gtctctacac aatgtggtga ttggtgtata gtctggtgtt 2880
   aaaagctgga tactttgatt ctgttgaaga ttgtcacacc cgaatttaag gacaaatcta 2940
   gatacatctc atatgttcac caggatagtg tatagatacc aatgtcataa tctttattac 3000
   acgacgataa tgtcttacaa aatatctggt gttacaagat gcacctttca acatgtttaa 3060
   tgctgcaaac tgttttaatt aaacagaatg cagtgttttg aacaaaaaaa tgctgcttta 3120
   tcctgcatct tgttttgcat gtgtcagcaa ttgcttacat tccattatga tctctgagat 3180
   tctttaaatt tctagcctga tgaaagtatt tactaattca actgaacaca aacattgttt 3240
   gaatgaacaa ggcaacacgg atgcttggaa taatggttgt gtataatatc acttagtggt 3300
   tttgctctca caccacatct ttcatgggtt ctataataat agttactgac tttaagtttc 3360
   ttattccttt ttgtctatct taggctggag aaaacgaggc agattacatt ggccgcatta 3420
   ctgaattttt tgaggggact gaccagtgtc actattttac ttgccgttgg ttcttccgag 3480
   cagaggacac ggtgtgtatt tagtattttg tcattctatg catgtcaagt ggcatagcac 3540
   tcacatttgt catggcaggt tatcaattct ttggtgtcca taagtgtgga tggccacaag 3600
   catgacccta gacgtgtttt tctttctgag gaaaagaacg acaatgtgct tgattgcatt 3660
   atctccaagg tcaagatagt ccatgttgat ccaaatgtaa gtttgctgca gtttgctgag 3720
   agctttgtgg ttttgctata cacataatgt ttctgactac cattgttttg ttgcctactt    3780
   gccttagatg gatccaaaag ccaaggctca gctgatagag agttgcgacc tatactatga    3840
   catgtcttac tctgttgcat attctacatt tgctaatatc tcgtctggta attccttctg    3900
   catcatcttt tttggttgac tagctgaatg cagttagctt tgccaaagag ttaaatacat    3960
   gagttgttcc tgcactcgtc aagggatgtc aataatgtcc acaaactctg aaaatgtatt    4020
   tttagatact taacttgtta agtcagtaaa acctgtcaga tacttgggtt ttgggtacga    4080
   ttaccatcct tatgtgagta aaactcgtca agggatgtca atgacgtgtt gattgtgtat    4140
   tagatattct gtttgttcga aagctaatct acacaaacag cttatgtaat gtaaaacctc    4200
   aaacaaactt gcctcttcat aagcttaggt ttataggatt agcgtttagt gcatgtaagg    4260
   cctatttgct tcacggcctc cctgccgagc tcctggctag acagccatcc tggccgtagg    4320
   tgcccgaaat cgaacacctg ggagccacgt ttgcactggc aggttttcct gggtgcaaac    4380
   caaacacgcc tatagtgttc aagtataact gaattggtgc tcacctttgt ctaatagctt    4440
   aagtttttgg ttttcatcgg tgcatgcaac tccatactca atagtcaata tgatatagtg    4500
   ttcaagcata gaactctcga gtttgaatcc tggcaggggc aatcaaataa aataattgca    4560
   gcttacccct atttccacgt ttgagcacat gagggagagt gttgaattat aagtgtgttc    4620
   tccatctttc tctaacagat tgaactggtt tgtgcatgca actcaatatg atatttgagt    4680
   caaatgttta ctttaaaatc atagttgatg caatttaata acatattttt ttggtctcgt    4740
   gtgagggagt gtacgtataa ctgaattgca cacatttcct tatcagctta ggtttttgac    4800
   tgcaactgtt ggtgcatgta gctcaataac taaagttgat ctggacagtc tacagtgaat    4860
   aagtttgaca cttgtaaaat gtgcatgtat ttttacaaac gctggcactt ttttcctaat    4920
   agaaaatggg cagtcagaca gtgataccgc ttcgggtatt tcttctgatg atgtggatct    4980
   ggagacgtca tctagtatgc caacgaggac agcaaccctt cttgatctgt attctggctg    5040
   tgggggcatg tctactggtc tttgcttggg tgcagctctt tctggcttga aacttgaaac    5100
   tgtaatcttc taactagtca tctgttggat agaatatgtt cacgatctca gaacttattc    5160
   tattgttctg gcttgcagcg atgggctgtt gatttcaaca gttttgcgtg ccaaagttta    5220
   aaatataatc atccacagac tgaggtatgg atagtaaact tcatcttgga ttccatctgt    5280
   tctgtcagct actcttacaa agtgtctgga tttttggatg taggtgcgaa atgagaaagc    5340
   cgatgagttt cttgccctcc ttaaggaatg ggcagttcta tgcaaaaaat atgtccaaga    5400
   tgtggattca aatttagcaa gctcagagga tcaagcggat gaagacagcc ctcttgacaa    5460
   ggacgaattt gttgtagaga agcttgtcgg gatatgttat ggtggcagtg acagggaaaa    5520
   tggcatctat tttaaggtac ttcagtgtca tttgttcatt tctacttgat tccaacaaaa    5580
   aaatcaatta cttaagcctg tcaaacgatg gatatttctg tatattttgc tgtaacgcta    5640
   gatttctgca ggtccagtgg gaaggatacg gccctgagga ggatacatgg gaaccgattg    5700
   ataacttgag gttagtgtat ggtatatcgt ctgcttgttg ccttgtatac ctatttgcat 5760
   ctaatccttg ttttgcaaac cagtgactgc ccgcagaaaa ttagagattt tgtacaagaa 5820
   gggcacaaaa gaaagattct cccactgcct gtgagtattt agttcgttgt gattttgctc 5880
   gctatttgtt tagctcccct tttttatttg gtgatatctg cctattttat tctttcaaag 5940
   ggtgatgttg atgtcatttg tggaggccca ccatgccaag gtatcagtgg gtttaatcgg 6000
   tacagaaacc gtgatgagcc actcaaagat gagaaaaaca aacaaatggt gactttcatg 6060
   gatattgtgg cgtacttgaa gcccaagtat gttctcatgg aaaatgtggt ggacatactc 6120
   aaatttgcgg atggttacct aggaaaatat gctttgagct gccttgttgc tatgaagtac 6180
   caagcgcggc ttggaatgat ggtggctggt tgctatggtc tgccacagtt caggatgcgt 6240
   gtgttcctct ggggtgctct ttcttccatg gtctgttctg taccttgctg ttttatatgc 6300
   ttcgctagat tcatattgca ctgttggctg ctggctaacc aggtgtacgt gtatttgaca 6360
   atttaggtgc tccctaagta tcctctgccc acctatgatg ttgtagtacg tggaggagcc 6420
   cctaatgcct tttcggtgag tgcaatcaca aaccactact atgaaatcat gtggaatgtg 6480
   taaaatacgc tgaccaactg aatttgttgc agcaatgtat ggttgcatat gacgagacac 6540
   aaaaaccatc cctgaaaaaa gccttgcttc ttggcgatgc aatttcagat ttaccaaagg 6600
   caagtgttct gtcaagttca tgcatttctc agtgagcatg ctatttaact cttctctgca 6660
   ggttcaaaat caccagccta acgatgtgat ggagtatggt ggttccccca agaccgaatt 6720
   ccagcgctac attcgactca gtcgtaaagg taaaaaaccc cgtgaactac tactggttgg 6780
   ccttcactac gaatatgtta ggatttaatt tcagaagaac cgcctttttt ttcttggtgc 6840
   ttcggtacta ctgcagcaag ctcactctta ttatcatgtc agacatgttg gattggtcct 6900
   tcggtgaggg ggctggtcca gatgaaggca agctcttgga tcaccagcct ttacggctta 6960
   acaacgatga ttatgagcgg gttcaacaga ttcctgtcaa gaaggttggt ggcttggtcg 7020
   catttgtgcc ttcctttgtt gtttttcccc cttctgaaac aatcatctct ctttcctatg 7080
   acagggagcc aacttccgcg acctaaaggg cgtgagggtt ggagcaaaca atattgttga 7140
   gtgggatcca gaaatcgagc gtgtgaaact ttcatctggg aaaccactgg tatgtgtgct 7200
   atttcgtgct gttgtttcta taactgtgca acatttactt tccatattca aactcataac 7260
   tgacgagatg ctgcaactac tgtaagattc atggctaacc catgacaaca ttttgcacac 7320
   atctttgtta tctaggttcc tgactatgca atgtcattca tcaagggcaa atcactcaag 7380
   taagtttcaa aacatttttt ttgttttttt ggggaaaagt aggttattgt ttacttgtgc 7440
   ttacatatga tgttgcaggc cgtttgggcg cctgtggtgg gacgagacag ttcctacagt 7500
   tgtaaccaga gcagagcctc acaaccaggt cagcttcaga aaggccactc cttttcgcca 7560
   atccctgcat ctgtatttac tattagcgtg tgttcccata tgatcattac cgaacatgtt 7620
   gtccacacag gttataattc atccgactca agcaagggtc ctcactatcc gggagaacgc 7680
   aaggttacag ggcttccccg attactaccg attgtttggc ccgatcaagg agaagtaagt 7740
   tcctgttttc aagttgcctg taccagatct agtcactatt gaaagttttc agcagcaagc 7800
   cattcatcag ttagttacag ctcttgaaag ccttacctct gaacatgtgt gctttctctg 7860
   atggtgatag gtacattcaa gtcgggaacg cagtggctgt ccctgttgcc cgggcactgg 7920
   gctactgtct ggggcaagcc tacctgggtg aatctgaggg gagtgaccct ctgtaccagc 7980
   tgcctccaag tttcacctct gttggaggac gcactgcggg gcaggcgagg gcctctcctg 8040
   ttggcacccc tgcaggggag gtagttgagc agtaaaagga tgacagatct gagctgagct 8100
   gggcaacatc cagcggcagg agcatttctg gttcggttcg attcgggctc acgagcgcat 8160
   tgtgctgatt cgcagggtgc actcgagctc gagtgcggag ttgttgtaat gtaggttgag 8220
   gtttcaaaac atccatccat ccatcggccc cgcctatttg tggatggtgt ttctcgtgcc 8280
   attttagttc agctgtattg atgcttcgtc atgtatgtgt attgtaccat tcgttcttgt 8340
   tacctactag taaggcaaca tttgatccat gttcaacgcg agcaaaattc catccacttt 8400
   aggtactgag atgctcaagt agttcagtca tcaggtaaca tttacaacct attattttgc 8460
   ctggtattat ctgaaaacga tttgctgagg atgttttttt tttgaagtaa aggtactgta 8520
   ggaaggtcct acagtaatat tattaaatca aaagaactaa gtttagggag acagaagaat 8580
   attacaaaga aataaaaggg agagtgagga gggggaagaa ttctggatac atgtatttac 8640
   tctatttcac ataaacatat ggacatcaac atagatgtga aggtatctaa tctaaataag 8700
   cgccgacatg ctttacaaga tagtttgatc tataaggtga gggatgttgt ggatgaagat 8760
   tgtgaccaac tagctcatgt gtagttttgt ttgtagtacc tgtcctaaaa aatatgtaat 8820
   tctaagttta atttgaatca gccttgtttt ttaaaatttt tttgaccaag tccgttaaaa 8880
   agtaataaca ttttttactt caaatagatt tactatgaaa ttttgtctta tggttatcta 8940
   atgacattta tttaaaactg taacaaaaac tggtttggtt aggggactga gggagtagtg 9000
   cttaaaatgg aagacgatag gagagatcac attagtggtg gcggtactga cagtgtcacg 9060
   cggtgatgtt ttgcagtgtg tttacttcgc cctctaatct aagaaaacaa gtattctgtc 9120
   ttcacctatt cagaatcttt tgatctaccg cttatggctg gtttaatgca tgtgtaccga 9180
   gttcaatata ggccttgttt ggttgtaatc ggatagagga tcgaaagaga ttaaatctct 9240
   ttttatttaa ttttgactag taacaacttc cttcaatcct cttcactcga tgaaccctta 9300
   catgattaac gtgtatctat gggggtgtgt tgaagtaaaa tattttgtgg agttttgagg 9360
   taatgatttc acgagatgtg gatgccgaag gcgcggttgc ccacgcaata accgttactg 9420
   gaggtaagtt tacgggagag catagaagaa ttagttcaca ttgtctatag gggtgcaaag 9480
   gcacttccca aaaaatgtaa aatttataaa tttagttgaa attttattat atatgcacct 9540
   ctagaacaaa ttattatgca cccacgacac atacacccct attaaattta cgcttgcttc 9600
   gccacggctt atgtagatac gtagctgtta tatgttttgt tagtatacac atgcagtttc 9660
   tcttatttct ctgaacttca ggctagttgc aactgccggc atccaatttg tcatgatttg 9720
   cacacatgtt gaggtgaacc atttgcatag agttgcaaca gtggggagac gcaaactgtt 9780
   aaatctttaa tatgaccctt ttaatcgaca acctgaatag acagcatgta aatatttggc 9840
   aatacgcacc tcttggttgg gatcccatac cgatctcaac tatttctaat acaaattgga 9900
   cgacaacagt aataaatatt tgaatttctt caactaaatg gactgctcag ccgcttaagg 9960
   ccatgttcgg ttctacccca atccatatgg attgagaggg ttttaatccc tagtaagtga 10020
   aaatctcccc caatccatat caatcccctc caatccatat ggattaaaaa taagcgaaca 10080
   agccctaa 10088
<210> 7
   <211> 3197
   <212> DNA
   <213> Artificial sequence
<220>
   <223> Methyltransferases
<400> 7
   agagcagcag cagctaccgc agcccctgcc atggcgccga gctccccgtc acccgccgcg      60
   cccacacgcg tctctgggcg gaagcgcgcc gccaaggccg aggagatcca ccagaacaag     120
   gaggaggagg aggaggagga ggaggtcgtg gcggcgtcct ccgccaagcg cagccgcaag     180
   gcggcatctt ctgggaagaa gcccaagtcg ccccccaagc aggccaagcc ggggaggaag     240
   aagaaggggg atgccgagat gaaggagccc gtggaggacg acgtgtgcgc cgaggagccc     300
   gacgaggagg agttggccat gggcgaggag gaggccgagg agcaggccat gcgggaggag     360
   gtggttgcgg tcgcggcggg gtcacccggg aagaagaggg tggggagaag gaacgccgcc     420
   gccgccgccg gcgaccacga gccggagttc atcggcagcc ctgttgccgc ggacgaggcg     480
   cgcagcaact ggcccaagcg ctacggccgc agcactgccg caaagaaacc ggatgaggag     540
   gaagagctca aggccagatg tcactaccgg agcgctaagg tggacaacgt cgtctactgc     600
   cttggggatg acgtctatgt caaggctgga gaaaacgagg cagattacat tggccgcatt     660
   actgaatttt ttgaggggac tgaccagtgt cactatttta cttgccgttg gttcttccga     720
   gcagaggaca cggttatcaa ttctttggtg tccataagtg tggatggcca caagcatgac     780
   cctagacgtg tttttctttc tgaggaaaag aacgacaatg tgcttgattg cattatctcc     840
   aaggtcaaga tagtccatgt tgatccaaat atggatccaa aagccaaggc tcagctgata     900
   gagagttgcg acctatacta tgacatgtct tactctgttg catattctac atttgctaat     960
   atctcgtctg aaaatgggca gtcagacagt gataccgctt cgggtatttc ttctgatgat    1020
   gtggatctgg agacgtcatc tagtatgcca acgaggacag caacccttct tgatctgtat    1080
   tctggctgtg ggggcatgtc tactggtctt tgcttgggtg cagctctttc tggcttgaaa      1140
   cttgaaactc gatgggctgt tgatttcaac agttttgcgt gccaaagttt aaaatataat      1200
   catccacaga ctgaggtgcg aaatgagaaa gccgatgagt ttcttgccct ccttaaggaa      1260
   tgggcagttc tatgcaaaaa atatgtccaa gatgtggatt caaatttagc aagctcagag      1320
   gatcaagcgg atgaagacag ccctcttgac aaggacgaat ttgttgtaga gaagcttgtc      1380
   gggatatgtt atggtggcag tgacagggaa aatggcatct attttaaggt ccagtgggaa      1440
   ggatacggcc ctgaggagga tacatgggaa ccgattgata acttgagtga ctgcccgcag      1500
   aaaattagag attttgtaca agaagggcac aaaagaaaga ttctcccact gcctggtgat      1560
   gttgatgtca tttgtggagg cccaccatgc caaggtatca gtgggtttaa tcggtacaga      1620
   aaccgtgatg agccactcaa agatgagaaa aacaaacaaa tggtgacttt catggatatt      1680
   gtggcgtact tgaagcccaa gtatgttctc atggaaaatg tggtggacat actcaaattt      1740
   gcggatggtt acctaggaaa atatgctttg agctgccttg ttgctatgaa gtaccaagcg      1800
   cggcttggaa tgatggtggc tggttgctat ggtctgccac agttcaggat gcgtgtgttc      1860
   ctctggggtg ctctttcttc catggtgctc cctaagtatc ctctgcccac ctatgatgtt      1920
   gtagtacgtg gaggagcccc taatgccttt tcgcaatgta tggttgcata tgacgagaca      1980
   caaaaaccat ccctgaaaaa agccttgctt cttggcgatg caatttcaga tttaccaaag      2040
   gttcaaaatc accagcctaa cgatgtgatg gagtatggtg gttcccccaa gaccgaattc      2100
   cagcgctaca ttcgactcag tcgtaaagac atgttggatt ggtccttcgg tgagggggct      2160
   ggtccagatg aaggcaagct cttggatcac cagcctttac ggcttaacaa cgatgattat      2220
   gagcgggttc aacagattcc tgtcaagaag ggagccaact tccgcgacct aaagggcgtg      2280
   agggttggag caaacaatat tgttgagtgg gatccagaaa tcgagcgtgt gaaactttca      2340
   tctgggaaac cactggttcc tgactatgca atgtcattca tcaagggcaa atcactcaag      2400
   ccgtttgggc gcctgtggtg ggacgagaca gttcctacag ttgtaaccag agcagagcct      2460
   cacaaccagg ttataattca tccgactcaa gcaagggtcc tcactatccg ggagaacgca      2520
   aggttacagg gcttccccga ttactaccga ttgtttggcc cgatcaagga gaagtacatt      2580
   caagtcggga acgcagtggc tgtccctgtt gcccgggcac tgggctactg tctggggcaa      2640
   gcctacctgg gtgaatctga ggggagtgac cctctgtacc agctgcctcc aagtttcacc      2700
   tctgttggag gacgcactgc ggggcaggcg agggcctctc ctgttggcac ccctgcaggg      2760
   gaggtagttg agcagtaaaa ggatgacaga tctgagctga gctgggcaac atccagcggc      2820
   aggagcattt ctggttcggt tcgattcggg ctcacgagcg cattgtgctg attcgcaggg      2880
   tgcactcgag ctcgagtgcg gagttgttgt aatgtaggtt gaggtttcaa aacatccatc      2940
   catccatcgg ccccgcctat ttgtggatgg tgtttctcgt gccattttag ttcagctgta      3000
   ttgatgcttc gtcatgtatg tgtattgtac cattcgttct tgttacctac tagtaaggca      3060
   acatttgatc catgttcaac gcgagcaaaa ttccatccac tttaggtact gagatgctca 3120
   agtagttcag tcatcaggta acatttacaa cctattattt tgcctggtat tatctgaaaa 3180
   cgatttgctg aggatgt 3197
<210> 8
   <211> 2748
   <212> DNA
   <213> Artificial sequence
<220>
   <223> Methyltransferases
<400> 8
<210> 9
   <211> 11129
   <212> DNA
   <213> Artificial sequence
<220>
   <223> Nucleic acid
<220>
   <221> misc_feature
   <222> (3102)..(3111)
   <223> n is a, c, g, or t
<220>
   <221> misc_feature
   <222> (6223)..(6232)
   <223> n is a, c, g, or t
<220>
<221> misc_feature
   <222> (6310)..(6319)
   <223> n is a, c, g, or t
<220>
   <221> misc_feature
   <222> (6325)..(6326)
   <223> n is a, c, g, or t
<400> 9
   tacacaagag gagcgaagca agtttcatcc aacaggcttc atcacggtga aacgggtata      60
   caacgtggct tcggtataac tcaaaggaag tataacgaag cacacttcat caagcaggct     120
   tcgtcataac gaaacaatga gatgcaagga gaaggtgtta ctcaagttat taaatacaac     180
   tgacgacatt gcccttatga catattgtaa actctgtatg taattattga gggtataaat     240
   gtaatagcgt acgaaccctt gcgattgtct ataaatagat gaacattacc ccgtactatt     300
   cacgttggat tgtaatcgct ctcgcgtcaa caccttcaaa caagccgaag atatcactgc     360
   aatataaatt ttgataatgt ttatatattt atcatggaat aaatatatga ctggatatca     420
   cactcatgta tttaaatcct tattgatata aatgatgagg atgtgttatt cacgaccttt     480
   gtctgaagat tattatatcc gaaaggagat attttttcga aggacgaatg ttcttaacat      540
   ttaataactg tgttaccttg ttcttgaaca tgtgacaaac attaggtttt gagtatttca     600
   attaatagtg aaattaaatt aaattttagt tgtccaaaat gacagacttc gatatttgtt     660
   ataattgttg agttaaattt taacagcatc cataaacaag taaacaacga ctatgtgtgc     720
   accagtgtct cctgtatcaa cggggtggtc cgagataagt gctattatgg acgaagaggg     780
   aaggggattc gacgaaatgg aggcgttggc gttggcttct gtgttttgga gacgcacgcg     840
   acagccaaac tccaaaacgg atacgagaca gctcttgggg ctgcgtaaac aggtattagt     900
   tttctgtccc cgtttaccgt tcccgtgcgc agacgccgtc acgcgtactc ttcttgtctc     960
   cgtcgccgcg ctctacggcg gtgctggcgt ccgtgctgta ctccgcgccg gcagaggccg    1020
   cgcgcgcgtc gcccgcggac ggcgccacgc cgctccacct cgccgcggcc gtgcacatcc    1080
   tcctcgccgc gggcgcgtcc gtggacgcgc gcgccttctc gggcctccgc gccggggacc    1140
   tcctcctccc gcgcgccaac gaggccgccg ccgccgcaga ccgggcgctc cgcgtgctcc    1200
   tgaagttccc cgcggcgtca ctgtcgtcct cgcccaagaa gtccgcctcg ccgccgccgg    1260
   ccccggaggc gaggaaggag tacccgcccg acctgacgct gccggacctc aagagcgggc    1320
   tgttcagcac cgacgagcaa cggacgcacc ttacagctgt cctagcacta attactccta    1380
   aattcatcat acaccaaagt ttcaattagt aaaaggtttt ggtattattt tctttacaag    1440
   actaaaagca tccactcgta tttgccatgg aaatattgcc aaaatggtta ccgaagaacc    1500
   aatgaaaaat ggcggtatcc gtccacctgt aacttgtcca aggctcttcc agcagagtag    1560
   tgccgccatg acgattgaca gagacacgag acgaaacaag cagaaggtcc ccgcggcgtc    1620
   actgtcgtcc tcgcccaaga agtccgcctc gccgccgccg gccccggagg cgaggaagga    1680
   gtacccgccc gacctgacgc tgccggacct caagagcggg ctgttcagca ccgacgagtt 1740
   ccgcatgtac agcttcaagg tgaagccctg ctcccgcacc tactcgcaca actggaccga 1800
   gtgcccttcg tgcacccggg cgagaacgcg cggcgccgtg acccgcgcgt actcctacag 1860
   ctgcgtgccc tgccccgagt tccgcaaggg ccggcgctgc cgcaagggcg acggctgcga 1920
   gtacgcgcac gacgtcttcg agtgctggct ccaccccggc gcagtaccgg acgcgcctct 1980
   gcaaggacga ggtgggctgc gcgcgccgca tctgcttctt cgcgcacaag cgcgaggagc 2040
   tccgcgccgt caacccctcc gcggtgttcg tcggcatgca gatgcagccc accgtgtcgc 2100
   cgccgccgcc caacggcctc ggcgacatgc tcagcccggc ggcctggccc tcctcccccg 2160
   cgagcaggct caataaggcc gcgctcggcg gccgggacct ggaccagtac cagcacatgt 2220
   tgttcgacaa ggtgtcgtcg ccgagggcca gttggagaca agaagagtac gcgtgacgga 2280
   gatgcgacgg agaaaaaggg tacgcgtgac ggcgtctgca cacgggaacg gtaaacgggg 2340
   acaagaaact aatacctgtt tacgcagccc caagtgctgt ctcgtatccg ttttggagtt 2400
   tggctgtcgc gtgcgtctcc aaaacagaga agccaacgcc aacgcctcca tttcgtcgaa 2460
   tccccttccc tcttcgtcca taatggcaat tatctcctcg tgttcgtctg gaggaggtac 2520
   cgaggtcccg ccgttcgaga gacgagctac gcatttaagc cggggaggtc ggagcagtga 2580
   gtgggagtag ttcagggact gggagaggag aggaagcagg acctgggaga cctcgcctcg 2640
   tccgtcctcc tgcctacttc cttgcttttg gtaagtgaaa gtgattgaag tggagatgcc 2700
   tccaatctgc agtgctgttt cacgcgtaaa ccctggcccc ctcgccgctg gaagccccat 2760
   tgttcctctt tcatttctta ccgctgatgc agcttatggt ttcctagggt tttcgagata 2820
   tttggtttct tgcatccaga ggacgaagag ctttcatagg gttttcgaga cattgtcctc 2880
   acaatcgcat ccattctgca gtgctgtttc acgcgtaaac cctagcccct cgtcgctgga 2940
   agcccacatg gttcctcttt catttcttac cgctgatgca gcttatgggt tcctagggtt 3000
   ttcgagacat ttggcttctt gcatccagag gacgaagagc tttcctaggg ttttcgagac 3060
   attgtcctca caatcgcaga tttggttcga gacatttgtt tnnnnnnnnn ncgtcccgcg 3120
   cttgagtcct tgtgatctgc acagcatgat gctgccacca aatgcatgca gttagacaag 3180
   cttaatgttg catctgataa gactgtacaa cagattcgtg tgccaaatgt gtccttgcta 3240
   ctcaaggagg tcatatggaa caagtaaaac gaaatttctg catcagcaat caggcacaag 3300
   actgaatgac gtggttagtg ccaatgaggt ggggacttgg gaatggaatt agagttatag 3360
   ctattcctat aattcatctc actacagagg ctctccatcc tactaggact gtacaacaga 3420
   ttcgtgtgcc aaatgtgtcc ttgctactca aggaggtcat atggaacaag taaaacaaaa 3480
   tttctgcatc agcaatcagg cacaagactg aatgacgtgg ttagtgccaa tgaggtggga 3540
   acttgggaat ggaattagag ttatagctat tctatagcta ttcctataat tcatctcact 3600
   acagaggctc tccatcctac tagaatttaa tctgataaag taggcagaaa catggaactt 3660
   ataatgtcga agattgcatg tatagaatct aagtaacgga aagattcaac tttgtgatta 3720
   tatgctgctg ttacatggta ttggtaaaac attctttcaa gtgctcattg tcttcccttg 3780
   caaccaattc attgttgtcc ttgtttcctt gcaggtaggt gctgcttgtt ttatcttgaa 3840
   atgggctctc atgatggcga ggatgaagag ttgccacccc cccctccggt gccaccagat 3900
   gtgattccca ttaaagctga agatgctgtg ggtgaatcac cagcaaacca tatattaaag 3960
   ccaaagagat tactgatgga caggcctggt ataggaagaa aagggcagcc gacccagctc 4020
   tattcaaatc actttaaagt cgctgtgaag agtacagaag acgtcttctt tcactactat 4080
   gtatgtctgc tgactgagtt catgatcctt tgttcaaaat atgtcattgt ctgtcccttg 4140
   tttataacta atctttggct gatgtgatat tgttatttta tttgactagg taaacctgaa 4200
   gtatgaggat gatcgacccg ttgatggtaa agggatcggc agaaaggtga ttgataaact 4260
   gcagcagaca tatcgtgcag agctttctaa caaggacttt gtatatgatg gagaaaagag 4320
   cctgtttaca gttggtggtc ttccacaaaa aaagaatgag ttcaccgttg tcttggagga 4380
   cgtatctact ggaaagttag ttttagtctt tgatctgctt tcttgttgtt tatgcttacc 4440
   cagaatcagt aattgccatg ttttttgttt gtggttgtag gactgctgcc aatgggagcc 4500
   ctggaggtaa tgacagtcct ggaggtggtg ataggaagag agtgaggagg ccataccaga 4560
   cgaaaacttt caaagtggag ataaattttg cagcagaggt tcctatgagt gctattggtc 4620
   aagtcattag aggcgaagaa tctgagaact ccctggaggc gcttcgtgtt cttgatatca 4680
   tactgaggca gcattccgca gaacagtatg tagctatgca tcttgatgga tgattatagg 4740
   tagatttgag gatcttctgc agttacgaca tgacaatttt tctcaatact tgcagaggct 4800
   gccttttggt taagcaatca tttttctaca acaacccttc atgctttgtt gacttgggtg 4860
   gtggtgtgat gggttgtcgt ggatttcatt caagcttccg tggcacacag agtggacttt 4920
   ccctcaatgt tggtgcgtgt tcgtcctgta tggctgtatt ggtgccatgt aactgcattg 4980
   tctattctat aatcttacat ctttcatgat catgatagcc tttttttttg aaatccacag 5040
   atgtctcaac aacaatgatc gtgaaacctg gccctgttat tgattttctt ctttctaacc 5100
   agaatgttaa tgatcctagc agaattgatt ggcaaaaggt aaatgccatc tttggatgaa 5160
   acttctcgaa taaccatgct agtgcattga aatatctatt ttgatagaat gataacacaa 5220
   ttttggggtg tgcatattca atacaggcca agcgtgctct caagggcttg aggattagaa 5280
   ccactcctgc aaattcagaa ttcaagattt ttggtctcag cgagaggatc tgcaaagaac 5340
   aaacgtgtgg acatgttttc ttaccgctct gtttattgtc atgcaagctt tactttacat 5400
   ttttaaaaaa ttgtacccta gtgtgttaag cttgtttcag aagaatttta tttaacaaat 5460
   attgccttta ttgattttgt tctgattcca atgccaagct tgcttgatgg tgatatctgt 5520
   gtgcttaatt gctttctcat tgacatatgc aattaggttt ccgctgaggc agagaaatgg 5580
   tagcaacgga gattgtgata ccattgaaat aactgtctat gactactatg caaagaaagg 5640
   aatcgatcta aagtattctg gtgatttccc ctgtataaat acagggaagg caaagcgccc 5700
   aacatatttt ccaatcgagg tttgtttcag ttttgttagt tacatcctgt caaatctctg 5760
   tttattaaat atatcttgca tctcatattc gatgggcagg cttggtgggg ctgtctcact 5820
   gagtcactag gtcgtgggtt caaagcagcc tctccacatt tgtggggggg ggaaggcttg 5880
   cttcggttta tcccttcctt agaccctagg tctgcccctt tttttgcatc tcattttttt 5940
   gtattgtgtt tctagctatg ctcgcttgtt ccgcttcaaa gatacaccaa agctttgtct 6000
   acgctacaaa ggtcatccct tgtggagaag tctagacaga agcctgaaga aaggatgacc 6060
   gttctaaatg atgtgagctg ttaccttgat tttagcatgc tgcgaccatt cataactgca 6120
   tgggatttat ttctacgact gaattaatca acagcttatg attaccctaa atgctaggca 6180
   ctgcaacgca gtaactacga ttctgacccc atgttgatgt gcnnnnnnnn nnaggttgag 6240
   ggcatgtggt gtttcagttg ctccaaaatt tacccaagtt gaaggaagga tccttcaagc 6300
   cccaaaggtn nnnnnnnnng cttgnnagag aatcctaggg tgtttggatg gaattctagg 6360
   atttaaagat ttttttccta tcctgattgt taatatctct atggatccaa acgctccctt 6420
   agttgttttc tctctaaatg ttgtaccaag tcattatctc agtaaaatgc atctgactaa 6480
   atctaatatc ttgtagctga aagccggcaa tggtgatgat atcttttcac gaaatggacg 6540
   gtggaatttc actaataggg tcagcagctc aacatgtttc ttttttaacc ttcttttttt 6600
   ctttattagg aaggattttt ctgatgttta tttgccaata ctttgcccct ttcagaagtt 6660
   ttatgaaacc tgctctgtga ataagtgggc ggtcgttaat ttctctgcac gttgtgatgt 6720
   tcggaatctt atccgtgacc tgatgaggaa tgcatctgca aagggaattg tatggtttag 6780
   cttttcacat atcttctatt gtagcagttt tctgctgctg acgttttctt ctggcagcaa 6840
   atggaggaac cttttgatgt gtttgaagag agtccctcta tgaggcgtgc acctgtgtca 6900
   agaagggtgg atgatatgtt tgggcagata aaatcaaaac ttcctggagc tcctaggttc 6960
   ctcttgtgcc ttctccctga gaggaaaaat tgtgaaatct atggttggtt tattttttgt 7020
   taccactcca cataaatatc tcacatcttg caataattat acctttttgg tttcaaccag 7080
   gtccttggaa gagaaagtgc ctggccgagt ttggtattgt cacacagtgt ctagctccat 7140
   taagagtcaa tgatccgtac ctgcttaatt tgctgatgaa gatcaatgca aaggtttggg 7200
   acatgatttt atctttcttt tgcactgtat tgttccctgg ttcatgatta tattatcctc 7260
   tttcctctgt ttttaactag cttggtggtc tgaactcgtt gctgcaagtt gaagcatctt 7320
   cgtcaatacc acatgtgtcg caagtaccca ccatcatctt aggtatggat gtttcacatg 7380
   gtcatccagg acaagataga ccttcggttg cagcggtaag atgggtgtac ctttacttgt 7440
   acaattcttc tgcccacatg gaatctgagt ggttttgtaa aatatgtgaa atggatgtct 7500
   gttaatctga tgtaaaccat acctttcttc tttgtttagg tggttagttc tcgtcaatgg 7560
   cctcttatct ctagatatag agcatcagtg cacacccaat ctgccagact agaaatgatg 7620
   tcctcgttgt ttaagccgcg gggtactgat gatgatggcc tcatccggta agagatatct 7680
   tcttagtatg ttgatgtatg gtaagcgtga tgacttttgg atgctaagtt tatcttcgta 7740
   tttgggactt ccagggaatc actgatcgac ttctacacta gctctggaaa gcgaaaacca 7800
   gaacacataa ttattttcag gtctgaagct ttggggttat tgttagcttg tatcactaaa 7860
   tctttttgta gaaacaaatt cttttttttg tttttgtttt gagaagtatg tatggtacat 7920
   tctttgctgg gatggattat tgatactttc ttagccgccg tggcgtaaat gtaaccattt 7980
   gctgggtttt agggatggag tcagtgaaag tcagtttacc caggtcatca acattgagct 8040
   ggatcagatc atcgaggtaa tacatttgtt tgattcgtcc tttgcacatt tgcttgctta 8100
   gttcaggaac gaattgcaag gtaacacatc cttgtaatta atgcaggcat gtaagtttct 8160
   ggatgagaag tggtcaccca agttcactgt gattgttgct caaaagaacc accacaccaa 8220
   gttctttcag acggcatcac cagacaatgt tcttcctggt aatatgttta tttttgtcat 8280
   actaaaggga gtgtggtgag atgcggtgac aataataaat gtgtttgttt gttacgcagg 8340
   aactgtggtg gatagtaaag tttgccatcc taagaacttc gacttctaca tgtgtgcaca 8400
   tgctgggatg attgtgagtt gatctcagaa ttgagcaaaa agatgctgct gtcttgttct 8460
   tgtctctgaa tcctagatgg ttcttcgtct cagggaacaa caaggccgac ccactatcat 8520
   gttctgcacg acgagatagg tttcagtgcc gacgagatgc aggagtttgt tcattcgctc 8580
   tcttacgtgt aagtagtagt agtaaaatgc tgttttggct gtgtgtgtgt gtgtgtgttt 8640
   tgtgcttacg ctgcgtgtcc tgctgtgcag gtaccagagg agcacgacag ccatctcagt 8700
   gggtaggccg cttgaatcta aagccatctt gtgcagcaca gggacagcag gttgattttt 8760
   ttctagtgtc tgtttttgtg tttgcagttg ctccagtgtg ctacgcccac ctcgctgcag 8820
   cccaggtgag cacgttcctg agattggagg agatgtcaga cgcgtcctcc agccagggag 8880
   gagggcatac ctcggctggc agtgctcctg tgccggagct gcctcgcctg catgacaaag 8940
   tcaggagctc catgttcttc tgctagctga tgtgcgtgcg catcaggatc gagctccatg 9000
   ttttgtgtta gtaaggccta gttagtaagg ctgtagaaag aatgtttaat gtttgcatgc 9060
   taaagtccaa acaatcaaaa ccactactat atctaccaga gcactgatcg atcaaacaac 9120
   aagagtcagc atcaatcaat caaaaccacc actattctat ctaccgatca atctactcta 9180
   tacctgaagc actcactcgc tcgcaaacaa ccaagggatc atggatcctt gagtagctcc 9240
   attgtccagt ccctgacctt gacgaagcat ttcatggaca gcctctcgta cgccatctcg 9300
   tacatccgga ggttcatgta ggcgaagctc aagtaggtgg cgatgcactc ttggtgcctg 9360
   tggtacagcg ccgggatggt cagagcggcc accacacttg cgtagcaggc ggtcgggaaa 9420
   tctgtaacgc tgccgacgat ggacacgctc cacaggcaga ggaaggctct gtagaagagc 9480
   agcgagtccc tgcccagagc gatgtcccgg aaagcgtccg aggcggcgtc cagagcagag 9540
   cgcagcagcg ccgccgcctc gtccacggcc tgctgcggaa cgcgcagctc cgggacggga 9600
   ggctgcggcc tgttcaggag gcgcgccgct ttggcccaca ggaagagcac ggtgaggagc 9660
   aggagcagca cgttggaggc gagggacacc aaggtgtagc cgccgtacaa gagcagccag 9720
   gaggagacgg tgcccgccag caggcaggcg ctcacgtcgg cgcggccccg gcgccacagc 9780
   agcacgtccc agactgctgc agcaatccga tccgattccg atccagatat attagttagt 9840
   agatccaatc tcgagcggcc aatccgtaat aataattggc tgagagaaaa ggtgggagag 9900
   agtaccgaat cctccgacgg cacatgtccc ggcgccgccg ccgccgccgc caacaccaac 9960
   accagcatcc accaccatcg tcgtagttaa tactaaggga agggaactag agaaggaagg 10020
   gaggggccga tgcaacggaa gaaatggatg gaattcgacc aacagctagc tagagcgaag 10080
   cagaacccaa tcccaatcaa aacccgagaa cgaacctgct tttactttcc caaccagacg 10140
   gtggctggct tggaatccca ctatccctca ttccctctac caacagggca ccggcctggc 10200
   ctccctcccc aatcatccaa ccaccacctt tttttttctt ccttttgctg tgttttctta 10260
   caaacttaaa tctactcgaa tattgattca agatattatg gagagtaatt gaatatttcg 10320
   atgaatcacg tcatgtatat atttcttaat tacaaacttc tatcactatc aaataaagta 10380
   aacataatgc tatttgcaat aatatatact atgtaaacat gactaaatca aaataaatgt 10440
   ttaatttaat agttaccaag tttgcaagaa aacatttcct tccaaagcac atatcatgat 10500
   gctggcaatt tagttttatc agctataata atatcattga caacacattt tgtaagtcca 10560
   cattctaaat aaattctgta cattataact aaaacattat atcatatcag cataaaaaat 10620
   aaattaagta ttctatgttt ctataaaaca ataaaccatg cattgtacgg tgactgcgtc 10680
   tagggaaaat tataccgcat gctagcaaaa cgtcctccag tatttcatta gacacattgg 10740
   tgtcaaccta atatcctatt tgttgtcgtt ccaactaacc ccctcttata gtgctacaac 10800
   actatcatct atgtgtgatg gttttactcg tgatacctca cttacaggtg aaaataagac 10860
   cctcaaaaag gaggcgaatg agctcattca cgtcttaatt aaagcctaca gtagtgaggc 10920
   tcacttgctt aaatgcttgg atagtcaaag attctaggta ttagactaag taactattct 10980
   gttttgctac aaatgtatta gactaatgct gaagggcaaa tgccgaaggt aggtttcgac 11040
   cagggcgcgc accaagactt tggtgatcat tgtgaacatg cttcggacga agctaattcc 11100
   gaaggtccta tgggcgacga caaactaag 11129
<210> 10
   <211> 11129
   <212> DNA
   <213> Artificial sequence
<220>
   <223> Nucleic acid
<220>
   <221> misc_feature
   <222> (3102)..(3111)
   <223> n is a, c, g, or t
<220>
   <221> misc_feature
   <222> (6223)..(6232)
   <223> n is a, c, g, or t
<220>
   <221> misc_feature
   <222> (6310)..(6319)
   <223> n is a, c, g, or t
<220>
   <221> misc_feature
   <222> (6325)..(6326)
   <223> n is a, c, g, or t
<400> 10
   tacacaagag gagcgaagca agtttcatcc aacaggcttc atcacggtga aacgggtata 60
   caacgtggct tcggtataac tcaaaggaag tataacgaag cacacttcat caagcaggct 120
   tcgtcataac gaaacaatga gatgcaagga gaaggtgtta ctcaagttat taaatacaac 180
   tgacgacatt gcccttatga catattgtaa actctgtatg taattattga gggtataaat 240
   gtaatagcgt acgaaccctt gcgattgtct ataaatagat gaacattacc ccgtactatt 300
   cacgttggat tgtaatcgct ctcgcgtcaa caccttcaaa caagccgaag atatcactgc 360
   aatataaatt ttgataatgt ttatatattt atcatggaat aaatatatga ctggatatca 420
   cactcatgta tttaaatcct tattgatata aatgatgagg atgtgttatt cacgaccttt 480
   gtctgaagat tattatatcc gaaaggagat attttttcga aggacgaatg ttcttaacat 540
   ttaataactg tgttaccttg ttcttgaaca tgtgacaaac attaggtttt gagtatttca 600
   attaatagtg aaattaaatt aaattttagt tgtccaaaat gacagacttc gatatttgtt 660
   ataattgttg agttaaattt taacagcatc cataaacaag taaacaacga ctatgtgtgc 720
   accagtgtct cctgtatcaa cggggtggtc cgtctggagg aggtaccgag agataagtgc 780
   tattatggac gaagagggaa ggggattcga cgaaatggag gcgttggcgt tggcttctgt 840
   gttttggaga cgcacgcgac agccaaactc caaaacggat acgagacagc tcttggggct 900
   gcgtaaacag gtattagttt tctgtccccg tttaccgttc ccgtgcgcag acgccgtcac 960
   gcgtactctt cttgtctccg tcgccgcgct ctacggcggt gctggcgtcc gtgctgtact 1020
   ccgcgccggc agaggccgcg cgcgcgtcgc ccgcggacgg cgccacgccg ctccacctcg 1080
   ccgcggccgt gcacatcctc ctcgccgcgg gcgcgtccgt ggacgcgcgc gccttctcgg 1140
   gcctccgcgc cggggacctc ctcctcccgc gcgccaacga ggccgccgcc gccgcagacc 1200
   gggcgctccg cgtgctcctg aagttccccg cggcgtcact gtcgtcctcg cccaagaagt 1260
   ccgcctcgcc gccgccggcc ccggaggcga ggaaggagta cccgcccgac ctgacgctgc 1320
   cggacctcaa gagcgggctg ttcagcaccg acgagcaacg gacgcacctt acagctgtcc 1380
   tagcactaat tactcctaaa ttcatcatac accaaagttt caattagtaa aaggttttgg 1440
   tattattttc tttacaagac taaaagcatc cactcgtatt tgccatggaa atattgccaa 1500
   aatggttacc gaagaaccaa tgaaaaatgg cggtatccgt ccacctgtaa cttgtccaag 1560
   gctcttccag cagagtagtg ccgccatgac gattgacaga gacacgagac gaaacaagca 1620
   gaaggtcccc gcggcgtcac tgtcgtcctc gcccaagaag tccgcctcgc cgccgccggc 1680
   cccggaggcg aggaaggagt acccgcccga cctgacgctg ccggacctca agagcgggct 1740
   gttcagcacc gacgagttcc gcatgtacag cttcaaggtg aagccctgct cccgcaccta 1800
   ctcgcacaac tggaccgagt gcccttcgtg cacccgggcg agaacgcgcg gcgccgtgac 1860
   ccgcgcgtac tcctacagct gcgtgccctg ccccgagttc cgcaagggcc ggcgctgccg 1920
   caagggcgac ggctgcgagt acgcgcacga cgtcttcgag tgctggctcc accccggcgc 1980
   agtaccggac gcgcctctgc aaggacgagg tgggctgcgc gcgccgcatc tgcttcttcg 2040
   cgcacaagcg cgaggagctc cgcgccgtca acccctccgc ggtgttcgtc ggcatgcaga 2100
   tgcagcccac cgtgtcgccg ccgccgccca acggcctcgg cgacatgctc agcccggcgg 2160
   cctggccctc ctcccccgcg agcaggctca ataaggccgc gctcggcggc cgggacctgg 2220
   accagtacca gcacatgttg ttcgacaagg tgtcgtcgcc gagggccagt tggagacaag 2280
   aagagtacgc gtgacggaga tgcgacggag aaaaagggta cgcgtgacgg cgtctgcaca 2340
   cgggaacggt aaacggggac aagaaactaa tacctgttta cgcagcccca agtgctgtct 2400
   cgtatccgtt ttggagtttg gctgtcgcgt gcgtctccaa aacagagaag ccaacgccaa 2460
   cgcctccatt tcgtcgaatc cccttccctc ttcgtccata atggcaatta tctcctcgtg 2520
   ttcggtcccg ccgttcgaga gacgagctac gcatttaagc cggggaggtc ggagcagtga 2580
   gtgggagtag ttcagggact gggagaggag aggaagcagg acctgggaga cctcgcctcg 2640
   tccgtcctcc tgcctacttc cttgcttttg gtaagtgaaa gtgattgaag tggagatgcc 2700
   tccaatctgc agtgctgttt cacgcgtaaa ccctggcccc ctcgccgctg gaagccccat 2760
   tgttcctctt tcatttctta ccgctgatgc agcttatggt ttcctagggt tttcgagata 2820
   tttggtttct tgcatccaga ggacgaagag ctttcatagg gttttcgaga cattgtcctc 2880
   acaatcgcat ccattctgca gtgctgtttc acgcgtaaac cctagcccct cgtcgctgga 2940
   agcccacatg ttcctctttc atttcttacc gctgatgcag cttatgggtt tcctagggtt 3000
   ttcgagacat ttggcttctt gcatccagag gacgaagagc tttcctaggg ttttcgagac 3060
   attgtcctca caatcgcaga tttggttcga gacatttgtt tnnnnnnnnn ncgtcccgcg 3120
   cttgagtcct tgtgatctgc acagcatgat gctgccacca aatgcatgca gttagacaag 3180
   cttaatgttg catctgataa gactgtacaa cagattcgtg tgccaaatgt gtccttgcta 3240
   ctcaaggagg tcatatggaa caagtaaaac gaaatttctg catcagcaat caggcacaag 3300
   actgaatgac gtggttagtg ccaatgaggt ggggacttgg gaatggaatt agagttatag 3360
   ctattcctat aattcatctc actacagagg ctctccatcc tactaggact gtacaacaga 3420
   ttcgtgtgcc aaatgtgtcc ttgctactca aggaggtcat atggaacaag taaaacaaaa 3480
   tttctgcatc agcaatcagg cacaagactg aatgacgtgg ttagtgccaa tgaggtggga 3540
   acttgggaat ggaattagag ttatagctat tctatagcta ttcctataat tcatctcact 3600
   acagaggctc tccatcctac tagaatttaa tctgataaag taggcagaaa catggaactt 3660
   ataatgtcga agattgcatg tatagaatct aagtaacgga aagattcaac tttgtgatta 3720
   tatgctgctg ttacatggta ttggtaaaac attctttcaa gtgctcattg tcttcccttg 3780
   caaccaattc attgttgtcc ttgtttcctt gcaggtaggt gctgcttgtt ttatcttgaa 3840
   atgggctctc atgatggcga ggatgaagag ttgccacccc cccctccggt gccaccagat 3900
   gtgattccca ttaaagctga agatgctgtg ggtgaatcac cagcaaacca tatattaaag 3960
   ccaaagagat tactgatgga caggcctggt ataggaagaa aagggcagcc gacccagctc 4020
   tattcaaatc actttaaagt cgctgtgaag agtacagaag acgtcttctt tcactactat 4080
   gtatgtctgc tgactgagtt catgatcctt tgttcaaaat atgtcattgt ctgtcccttg 4140
   tttataacta atctttggct gatgtgatat tgttatttta tttgactagg taaacctgaa 4200
   gtatgaggat gatcgacccg ttgatggtaa agggatcggc agaaaggtga ttgataaact 4260
   gcagcagaca tatcgtgcag agctttctaa caaggacttt gtatatgatg gagaaaagag 4320
   cctgtttaca gttggtggtc ttccacaaaa aaagaatgag ttcaccgttg tcttggagga 4380
   cgtatctact ggaaagttag ttttagtctt tgatctgctt tcttgttgtt tatgcttacc 4440
   cagaatcagt aattgccatg ttttttgttt gtggttgtag gactgctgcc aatgggagcc 4500
   ctggaggtaa tgacagtcct ggaggtggtg ataggaagag agtgaggagg ccataccaga 4560
   cgaaaacttt caaagtggag ataaattttg cagcagaggt tcctatgagt gctattggtc 4620
   aagtcattag aggcgaagaa tctgagaact ccctggaggc gcttcgtgtt cttgatatca 4680
   tactgaggca gcattccgca gaacagtatg tagctatgca tcttgatgga tgattatagg 4740
   tagatttgag gatcttctgc agttacgaca tgacaatttt tctcaatact tgcagaggct 4800
   gccttttggt taagcaatca tttttctaca acaacccttc atgctttgtt gacttgggtg 4860
   gtggtgtgat gggttgtcgt ggatttcatt caagcttccg tggcacacag agtggacttt 4920
   ccctcaatgt tggtgcgtgt tcgtcctgta tggctgtatt ggtgccatgt aactgcattg 4980
   tctattctat aatcttacat ctttcatgat catgatagcc tttttttttg aaatccacag 5040
   atgtctcaac aacaatgatc gtgaaacctg gccctgttat tgattttctt ctttctaacc 5100
   agaatgttaa tgatcctagc agaattgatt ggcaaaaggt aaatgccatc tttggatgaa 5160
   acttctcgaa taaccatgct agtgcattga aatatctatt ttgatagaat gataacacaa 5220
   ttttggggtg tgcatattca atacaggcca agcgtgctct caagggcttg aggattagaa 5280
   ccactcctgc aaattcagaa ttcaagattt ttggtctcag cgagaggatc tgcaaagaac 5340
   aaacgtgtgg acatgttttc ttaccgctct gtttattgtc atgcaagctt tactttacat 5400
   ttttaaaaaa ttgtacccta gtgtgttaag cttgtttcag aagaatttta tttaacaaat 5460
   attgccttta ttgattttgt tctgattcca atgccaagct tgcttgatgg tgatatctgt 5520
   gtgcttaatt gctttctcat tgacatatgc aattaggttt ccgctgaggc agagaaatgg 5580
   tagcaacgga gattgtgata ccattgaaat aactgtctat gactactatg caaagaaagg 5640
   aatcgatcta aagtattctg gtgatttccc ctgtataaat acagggaagg caaagcgccc 5700
   aacatatttt ccaatcgagg tttgtttcag ttttgttagt tacatcctgt caaatctctg 5760
   tttattaaat atatcttgca tctcatattc gatgggcagg cttggtgggg ctgtctcact 5820
   gagtcactag gtcgtgggtt caaagcagcc tctccacatt tgtggggggg ggaaggcttg 5880
   cttcggttta tcccttcctt agaccctagg tctgcccctt tttttgcatc tcattttttt 5940
   gtattgtgtt tctagctatg ctcgcttgtt ccgcttcaaa gatacaccaa agctttgtct 6000
   acgctacaaa ggtcatccct tgtggagaag tctagacaga agcctgaaga aaggatgacc 6060
   gttctaaatg atgtgagctg ttaccttgat tttagcatgc tgcgaccatt cataactgca 6120
   tgggatttat ttctacgact gaattaatca acagcttatg attaccctaa atgctaggca 6180
   ctgcaacgca gtaactacga ttctgacccc atgttgatgt gcnnnnnnnn nnaggttgag 6240
   ggcatgtggt gtttcagttg ctccaaaatt tacccaagtt gaaggaagga tccttcaagc 6300
   cccaaaggtn nnnnnnnnng cttgnnagag aatcctaggg tgtttggatg gaattctagg 6360
   atttaaagat ttttttccta tcctgattgt taatatctct atggatccaa acgctcccct 6420
   agttgttttc tctctaaatg ttgtaccaag tcattatctc agtaaaatgc atctgactaa 6480
   atctaatatc ttgtagctga aagccggcaa tggtgatgat atcttttcac gaaatggacg 6540
   gtggaatttc actaataggg tcagcagctc aacatgtttc ttttttaacc ttcttttttt 6600
   ctttattagg aaggattttt ctgatgttta tttgccaata ctttgcccct ttcagaagtt 6660
   ttatgaaacc tgctctgtga ataagtgggc ggtcgttaat ttctctgcac gttgtgatgt 6720
   tcggaatctt atccgtgacc tgatgaggaa tgcatctgca aagggaattg tatggtttag 6780
   cttttcacat atcttctatt gtagcagttt tctgctgctg acgttttctt ctggcagcaa 6840
   atggaggaac cttttgatgt gtttgaagag agtccctcta tgaggcgtgc acctgtgtca 6900
   agaagggtgg atgatatgtt tgggcagata aaatcaaaac ttcctggagc tcctaggttc 6960
   ctcttgtgcc ttctccctga gaggaaaaat tgtgaaatct atggttggtt tattttttgt 7020
   taccactcca cataaatatc tcacatcttg caataattat acctttttgg tttcaaccag 7080
   gtccttggaa gagaaagtgc ctggccgagt ttggtattgt cacacagtgt ctagctccat 7140
   taagagtcaa tgatccgtac ctgcttaatt tgctgatgaa gatcaatgca aaggtttggg 7200
   acatgatttt atctttcttt tgcactgtat tgttccctgg ttcatgatta tattatcctc 7260
   tttcctctgt ttttaactag cttggtggtc tgaactcgtt gctgcaagtt gaagcatctt 7320
   cgtcaatacc acatgtgtcg caagtaccca ccatcatctt aggtatggat gtttcacatg 7380
   gtcatccagg acaagataga ccttcggttg cagcggtaag atgggtgtac ctttacttgt 7440
   acaattcttc tgcccacatg gaatctgagt ggttttgtaa aatatgtgaa atggatgtct 7500
   gttaatctga tgtaaaccat acctttcttc tttgtttagg tggttagttc tcgtcaatgg 7560
   cctcttatct ctagatatag agcatcagtg cacacccaat ctgccagact agaaatgatg 7620
   tcctcgttgt ttaagccgcg gggtactgat gatgatggcc tcatccggta agagatatct 7680
   tcttagtatg ttgatgtatg gtaagcgtga tgacttttgg atgctaagtt tatcttcgta 7740
   tttgggactt ccagggaatc actgatcgac ttctacacta gctctggaaa gcgaaaacca 7800
   gaacacataa ttattttcag gtctgaagct ttggggttat tgttagcttg tatcactaaa 7860
   tctttttgta gaaacaaatt cttttttttg tttttgtttt gagaagtatg tatggtacat 7920
   tctttgctgg gatggattat tgatactttc ttagccgccg tggcgtaaat gtaaccattt 7980
   gctgggtttt agggatggag tcagtgaaag tcagtttacc caggtcatca acattgagct 8040
   ggatcagatc atcgaggtaa tacatttgtt tgattcgtcc tttgcacatt tgcttgctta 8100
   gttcaggaac gaattgcaag gtaacacatc cttgtaatta atgcaggcat gtaagtttct 8160
   ggatgagaag tggtcaccca agttcactgt gattgttgct caaaagaacc accacaccaa 8220
   gttctttcag acggcatcac cagacaatgt tcttcctggt aatatgttta tttttgtcat 8280
   actaaaggga gtgtggtgag atgcggtgac aataataaat gtgtttgttt gttacgcagg 8340
   aactgtggtg gatagtaaag tttgccatcc taagaacttc gacttctaca tgtgtgcaca 8400
   tgctgggatg attgtgagtt gatctcagaa ttgagcaaaa agatgctgct gtcttgttct 8460
   tgtctctgaa tcctagatgg ttcttcgtct cagggaacaa caaggccgac ccactatcat 8520
   gttctgcacg acgagatagg tttcagtgcc gacgagatgc aggagtttgt tcattcgctc 8580
   tcttacgtgt aagtagtagt agtaaaatgc tgttttggct gtgtgtgtgt gtgtgtgttt 8640
   tgtgcttacg ctgcgtgtcc tgctgtgcag gtaccagagg agcacgacag ccatctcagt 8700
   gggtaggccg cttgaatcta aagccatctt gtgcagcaca gggacagcag gttgattttt 8760
   ttctagtgtc tgtttttgtg tttgcagttg ctccagtgtg ctacgcccac ctcgctgcag 8820
   cccaggtgag cacgttcctg agattggagg agatgtcaga cgcgtcctcc agccagggag 8880
   gagggcatac ctcggctggc agtgctcctg tgccggagct gcctcgcctg catgacaaag 8940
   tcaggagctc catgttcttc tgctagctga tgtgcgtgcg catcaggatc gagctccatg 9000
   ttttgtgtta gtaaggccta gttagtaagg ctgtagaaag aatgtttaat gtttgcatgc 9060
   taaagtccaa acaatcaaaa ccactactat atctaccaga gcactgatcg atcaaacaac 9120
   aagagtcagc atcaatcaat caaaaccacc actattctat ctaccgatca atctactcta 9180
   tacctgaagc actcactcgc tcgcaaacaa ccaagggatc atggatcctt gagtagctcc 9240
   attgtccagt ccctgacctt gacgaagcat ttcatggaca gcctctcgta cgccatctcg 9300
   tacatccgga ggttcatgta ggcgaagctc aagtaggtgg cgatgcactc ttggtgcctg 9360
   tggtacagcg ccgggatggt cagagcggcc accacacttg cgtagcaggc ggtcgggaaa 9420
   tctgtaacgc tgccgacgat ggacacgctc cacaggcaga ggaaggctct gtagaagagc 9480
   agcgagtccc tgcccagagc gatgtcccgg aaagcgtccg aggcggcgtc cagagcagag 9540
   cgcagcagcg ccgccgcctc gtccacggcc tgctgcggaa cgcgcagctc cgggacggga 9600
   ggctgcggcc tgttcaggag gcgcgccgct ttggcccaca ggaagagcac ggtgaggagc 9660
   aggagcagca cgttggaggc gagggacacc aaggtgtagc cgccgtacaa gagcagccag 9720
   gaggagacgg tgcccgccag caggcaggcg ctcacgtcgg cgcggccccg gcgccacagc 9780
   agcacgtccc agactgctgc agcaatccga tccgattccg atccagatat attagttagt 9840
   agatccaatc tcgagcggcc aatccgtaat aataattggc tgagagaaaa ggtgggagag 9900
   agtaccgaat cctccgacgg cacatgtccc ggcgccgccg ccgccgccgc caacaccaac 9960
   accagcatcc accaccatcg tcgtagttaa tactaaggga agggaactag agaaggaagg 10020
   gaggggccga tgcaacggaa gaaatggatg gaattcgacc aacagctagc tagagcgaag    10080
   cagaacccaa tcccaatcaa aacccgagaa cgaacctgct tttactttcc caaccagacg    10140
   gtggctggct tggaatccca ctatccctca ttccctctac caacagggca ccggcctggc 10200
   ctccctcccc aatcatccaa ccaccacctt tttttttctt ccttttgctg tgttttctta 10260
   caaacttaaa tctactcgaa tattgattca agatattatg gagagtaatt gaatatttcg 10320
   atgaatcacg tcatgtatat atttcttaat tacaaacttc tatcactatc aaataaagta 10380
   aacataatgc tatttgcaat aatatatact atgtaaacat gactaaatca aaataaatgt 10440
   ttaatttaat agttaccaag tttgcaagaa aacatttcct tccaaagcac atatcatgat 10500
   gctggcaatt tagttttatc agctataata atatcattga caacacattt tgtaagtcca 10560
   cattctaaat aaattctgta cattataact aaaacattat atcatatcag cataaaaaat 10620
   aaattaagta ttctatgttt ctataaaaca ataaaccatg cattgtacgg tgactgcgtc 10680
   tagggaaaat tataccgcat gctagcaaaa cgtcctccag tatttcatta gacacattgg 10740
   tgtcaaccta atatcctatt tgttgtcgtt ccaactaacc ccctcttata gtgctacaac 10800
   actatcatct atgtgtgatg gttttactcg tgatacctca cttacaggtg aaaataagac 10860
   cctcaaaaag gaggcgaatg agctcattca cgtcttaatt aaagcctaca gtagtgaggc 10920
   tcacttgctt aaatgcttgg atagtcaaag attctaggta ttagactaag taactattct 10980
   gttttgctac aaatgtatta gactaatgct gaagggcaaa tgccgaaggt aggtttcgac 11040
   cagggcgcgc accaagactt tggtgatcat tgtgaacatg cttcggacga agctaattcc 11100
   gaaggtccta tgggcgacga caaactaag 11129
<210> 11
   <211> 11129
   <212> DNA
   <213> Artificial sequence
<220>
   <223> Nucleic acid
<220>
   <221> misc_feature
   <222> (3102)..(3111)
   <223> n is a, c, g, or t
<220>
   <221> misc_feature
   <222> (6223)..(6232)
   <223> n is a, c, g, or t
<220>
   <221> misc_feature
   <222> (6310)..(6319)
   <223> n is a, c, g, or t
<220>
   <221> misc_feature
   <222> (6325)..(6326)
   <223> n is a, c, g, or t
<400> 11
   tacacaagag gagcgaagca agtttcatcc aacaggcttc atcacggtga aacgggtata 60
   caacgtggct tcggtataac tcaaaggaag tataacgaag cacacttcat caagcaggct 120
   tcgtcataac gaaacaatga gatgcaagga gaaggtgtta ctcaagttat taaatacaac 180
   tgacgacatt gcccttatga catattgtaa actctgtatg taattattga gggtataaat 240
   gtaatagcgt acgaaccctt gcgattgtct ataaatagat gaacattacc ccgtactatt 300
   cacgttggat tgtaatcgct ctcgcgtcaa caccttcaaa caagccgaag atatcactgc 360
   aatataaatt ttgataatgt ttatatattt atcatggaat aaatatatga ctggatatca 420
   cactcatgta tttaaatcct tattgatata aatgatgagg atgtgttatt cacgaccttt 480
   gtctgaagat tattatatcc gaaaggagat attttttcga aggacgaatg ttcttaacat 540
   ttaataactg tgttaccttg ttcttgaaca tgtgacaaac attaggtttt gagtatttca 600
   attaatagtg aaattaaatt aaattttagt tgtccaaaat gacagacttc gatatttgtt 660
   ataattgttg agttaaattt taacagcatc cataaacaag taaacaacga ctatgtgtgc 720
   accagtgtct cctgtatcaa cggggtggtc cgtctggagg aggtaccgag gtccgagata 780
   agtgctatta tggacgaaga gggaagggga ttcgacgaaa tggaggcgtt ggcgttggct 840
   tctgtgtttt ggagacgcac gcgacagcca aactccaaaa cggatacgag acagctcttg 900
   gggctgcgta aacaggtatt agttttctgt ccccgtttac cgttcccgtg cgcagacgcc 960
   gtcacgcgta ctcttcttgt ctccgtcgcc gcgctctacg gcggtgctgg cgtccgtgct 1020
   gtactccgcg ccggcagagg ccgcgcgcgc gtcgcccgcg gacggcgcca cgccgctcca 1080
   cctcgccgcg gccgtgcaca tcctcctcgc cgcgggcgcg tccgtggacg cgcgcgcctt 1140
   ctcgggcctc cgcgccgggg acctcctcct cccgcgcgcc aacgaggccg ccgccgccgc 1200
   agaccgggcg ctccgcgtgc tcctgaagtt ccccgcggcg tcactgtcgt cctcgcccaa 1260
   gaagtccgcc tcgccgccgc cggccccgga ggcgaggaag gagtacccgc ccgacctgac 1320
   gctgccggac ctcaagagcg ggctgttcag caccgacgag caacggacgc accttacagc 1380
   tgtcctagca ctaattactc ctaaattcat catacaccaa agtttcaatt agtaaaaggt 1440
   tttggtatta ttttctttac aagactaaaa gcatccactc gtatttgcca tggaaatatt 1500
   gccaaaatgg ttaccgaaga accaatgaaa aatggcggta tccgtccacc tgtaacttgt 1560
   ccaaggctct tccagcagag tagtgccgcc atgacgattg acagagacac gagacgaaac 1620
   aagcagaagg tccccgcggc gtcactgtcg tcctcgccca agaagtccgc ctcgccgccg 1680
   ccggccccgg aggcgaggaa ggagtacccg cccgacctga cgctgccgga cctcaagagc 1740
   gggctgttca gcaccgacga gttccgcatg tacagcttca aggtgaagcc ctgctcccgc 1800
   acctactcgc acaactggac cgagtgccct tcgtgcaccc gggcgagaac gcgcggcgcc 1860
   gtgacccgcg cgtactccta cagctgcgtg ccctgccccg agttccgcaa gggccggcgc 1920
   tgccgcaagg gcgacggctg cgagtacgcg cacgacgtct tcgagtgctg gctccacccc 1980
   ggcgcagtac cggacgcgcc tctgcaagga cgaggtgggc tgcgcgcgcc gcatctgctt 2040
   cttcgcgcac aagcgcgagg agctccgcgc cgtcaacccc tccgcggtgt tcgtcggcat 2100
   gcagatgcag cccaccgtgt cgccgccgcc gcccaacggc ctcggcgaca tgctcagccc 2160
   ggcggcctgg ccctcctccc ccgcgagcag gctcaataag gccgcgctcg gcggccggga 2220
   cctggaccag taccagcaca tgttgttcga caaggtgtcg tcgccgaggg ccagttggag 2280
   acaagaagag tacgcgtgac ggagatgcga cggagaaaaa gggtacgcgt gacggcgtct 2340
   gcacacggga acggtaaacg gggacaagaa actaatacct gtttacgcag ccccaagtgc 2400
   tgtctcgtat ccgttttgga gtttggctgt cgcgtgcgtc tccaaaacag agaagccaac 2460
   gccaacgcct ccatttcgtc gaatcccctt ccctcttcgt ccataatggc aattatctcc 2520
   tcgtgttccg ccgttcgaga gacgagctac gcatttaagc cggggaggtc ggagcagtga 2580
   gtgggagtag ttcagggact gggagaggag aggaagcagg acctgggaga cctcgcctcg 2640
   tccgtcctcc tgcctacttc cttgcttttg gtaagtgaaa gtgattgaag tggagatgcc 2700
   tccaatctgc agtgctgttt cacgcgtaaa ccctggcccc ctcgccgctg gaagccccat 2760
   tgttcctctt tcatttctta ccgctgatgc agcttatggt ttcctagggt tttcgagata 2820
   tttggtttct tgcatccaga ggacgaagag ctttcatagg gttttcgaga cattgtcctc 2880
   acaatcgcat ccattctgca gtgctgtttc acgcgtaaac cctagcccct cgtcgctgga 2940
   agcccacatg gttcctcttt catttcttac cgctgatgca gcttatgggt tcctagggtt 3000
   ttcgagacat ttggcttctt gcatccagag gacgaagagc tttcctaggg ttttcgagac 3060
   attgtcctca caatcgcaga tttggttcga gacatttgtt tnnnnnnnnn ncgtcccgcg 3120
   cttgagtcct tgtgatctgc acagcatgat gctgccacca aatgcatgca gttagacaag 3180
   cttaatgttg catctgataa gactgtacaa cagattcgtg tgccaaatgt gtccttgcta 3240
   ctcaaggagg tcatatggaa caagtaaaac gaaatttctg catcagcaat caggcacaag 3300
   actgaatgac gtggttagtg ccaatgaggt ggggacttgg gaatggaatt agagttatag 3360
   ctattcctat aattcatctc actacagagg ctctccatcc tactaggact gtacaacaga 3420
   ttcgtgtgcc aaatgtgtcc ttgctactca aggaggtcat atggaacaag taaaacaaaa 3480
   tttctgcatc agcaatcagg cacaagactg aatgacgtgg ttagtgccaa tgaggtggga 3540
   acttgggaat ggaattagag ttatagctat tctatagcta ttcctataat tcatctcact 3600
   acagaggctc tccatcctac tagaatttaa tctgataaag taggcagaaa catggaactt 3660
   ataatgtcga agattgcatg tatagaatct aagtaacgga aagattcaac tttgtgatta 3720
   tatgctgctg ttacatggta ttggtaaaac attctttcaa gtgctcattg tcttcccttg 3780
   caaccaattc attgttgtcc ttgtttcctt gcaggtaggt gctgcttgtt ttatcttgaa 3840
   atgggctctc atgatggcga ggatgaagag ttgccacccc cccctccggt gccaccagat 3900
   gtgattccca ttaaagctga agatgctgtg ggtgaatcac cagcaaacca tatattaaag 3960
   ccaaagagat tactgatgga caggcctggt ataggaagaa aagggcagcc gacccagctc 4020
   tattcaaatc actttaaagt cgctgtgaag agtacagaag acgtcttctt tcactactat 4080
   gtatgtctgc tgactgagtt catgatcctt tgttcaaaat atgtcattgt ctgtcccttg 4140
   tttataacta atctttggct gatgtgatat tgttatttta tttgactagg taaacctgaa 4200
   gtatgaggat gatcgacccg ttgatggtaa agggatcggc agaaaggtga ttgataaact 4260
   gcagcagaca tatcgtgcag agctttctaa caaggacttt gtatatgatg gagaaaagag 4320
   cctgtttaca gttggtggtc ttccacaaaa aaagaatgag ttcaccgttg tcttggagga 4380
   cgtatctact ggaaagttag ttttagtctt tgatctgctt tcttgttgtt tatgcttacc 4440
   cagaatcagt aattgccatg ttttttgttt gtggttgtag gactgctgcc aatgggagcc 4500
   ctggaggtaa tgacagtcct ggaggtggtg ataggaagag agtgaggagg ccataccaga 4560
   cgaaaacttt caaagtggag ataaattttg cagcagaggt tcctatgagt gctattggtc 4620
   aagtcattag aggcgaagaa tctgagaact ccctggaggc gcttcgtgtt cttgatatca 4680
   tactgaggca gcattccgca gaacagtatg tagctatgca tcttgatgga tgattatagg 4740
   tagatttgag gatcttctgc agttacgaca tgacaatttt tctcaatact tgcagaggct 4800
   gccttttggt taagcaatca tttttctaca acaacccttc atgctttgtt gacttgggtg 4860
   gtggtgtgat gggttgtcgt ggatttcatt caagcttccg tggcacacag agtggacttt 4920
   ccctcaatgt tggtgcgtgt tcgtcctgta tggctgtatt ggtgccatgt aactgcattg 4980
   tctattctat aatcttacat ctttcatgat catgatagcc tttttttttg aaatccacag 5040
   atgtctcaac aacaatgatc gtgaaacctg gccctgttat tgattttctt ctttctaacc 5100
   agaatgttaa tgatcctagc agaattgatt ggcaaaaggt aaatgccatc tttggatgaa 5160
   acttctcgaa taaccatgct agtgcattga aatatctatt ttgatagaat gataacacaa 5220
   ttttggggtg tgcatattca atacaggcca agcgtgctct caagggcttg aggattagaa 5280
   ccactcctgc aaattcagaa ttcaagattt ttggtctcag cgagaggatc tgcaaagaac 5340
   aaacgtgtgg acatgttttc ttaccgctct gtttattgtc atgcaagctt tactttacat 5400
   ttttaaaaaa ttgtacccta gtgtgttaag cttgtttcag aagaatttta tttaacaaat 5460
   attgccttta ttgattttgt tctgattcca atgccaagct tgcttgatgg tgatatctgt 5520
   gtgcttaatt gctttctcat tgacatatgc aattaggttt ccgctgaggc agagaaatgg 5580
   tagcaacgga gattgtgata ccattgaaat aactgtctat gactactatg caaagaaagg 5640
   aatcgatcta aagtattctg gtgatttccc ctgtataaat acagggaagg caaagcgccc 5700
   aacatatttt ccaatcgagg tttgtttcag ttttgttagt tacatcctgt caaatctctg 5760
   tttattaaat atatcttgca tctcatattc gatgggcagg cttggtgggg ctgtctcact 5820
   gagtcactag gtcgtgggtt caaagcagcc tctccacatt tgtggggggg ggaaggcttg 5880
   cttcggttta tcccttcctt agaccctagg tctgcccctt tttttgcatc tcattttttt 5940
   gtattgtgtt tctagctatg ctcgcttgtt ccgcttcaaa gatacaccaa agctttgtct 6000
   acgctacaaa ggtcatccct tgtggagaag tctagacaga agcctgaaga aaggatgacc 6060
   gttctaaatg atgtgagctg ttaccttgat tttagcatgc tgcgaccatt cataactgca 6120
   tgggatttat ttctacgact gaattaatca acagcttatg attaccctaa atgctaggca 6180
   ctgcaacgca gtaactacga ttctgacccc atgttgatgt gcnnnnnnnn nnaggttgag 6240
   ggcatgtggt gtttcagttg ctccaaaatt tacccaagtt gaaggaagga tccttcaagc 6300
   cccaaaggtn nnnnnnnnng cttgnnagag aatcctaggg tgtttggatg gaattctagg 6360
   atttaaagat ttttttccta tcctgattgt taatatctct atggatccaa acgctccctt 6420
   agttgttttc tctctaaatg ttgtaccaag tcattatctc agtaaaatgc atctgactaa 6480
   atctaatatc ttgtagctga aagccggcaa tggtgatgat atcttttcac gaaatggacg 6540
   gtggaatttc actaataggg tcagcagctc aacatgtttc ttttttaacc ttcttttttt 6600
   ctttattagg aaggattttt ctgatgttta tttgccaata ctttgcccct ttcagaagtt 6660
   ttatgaaacc tgctctgtga ataagtgggc ggtcgttaat ttctctgcac gttgtgatgt 6720
   tcggaatctt atccgtgacc tgatgaggaa tgcatctgca aagggaattg tatggtttag 6780
   cttttcacat atcttctatt gtagcagttt tctgctgctg acgttttctt ctggcagcaa 6840
   atggaggaac cttttgatgt gtttgaagag agtccctcta tgaggcgtgc acctgtgtca 6900
   agaagggtgg atgatatgtt tgggcagata aaatcaaaac ttcctggagc tcctaggttc 6960
   ctcttgtgcc ttctccctga gaggaaaaat tgtgaaatct atggttggtt tattttttgt 7020
   taccactcca cataaatatc tcacatcttg caataattat acctttttgg tttcaaccag 7080
   gtccttggaa gagaaagtgc ctggccgagt ttggtattgt cacacagtgt ctagctccat 7140
   taagagtcaa tgatccgtac ctgcttaatt tgctgatgaa gatcaatgca aaggtttggg 7200
   acatgatttt atctttcttt tgcactgtat tgttccctgg ttcatgatta tattatcctc 7260
   tttcctctgt ttttaactag cttggtggtc tgaactcgtt gctgcaagtt gaagcatctt 7320
   cgtcaatacc acatgtgtcg caagtaccca ccatcatctt aggtatggat gtttcacatg 7380
   gtcatccagg acaagataga ccttcggttg cagcggtaag atgggtgtac ctttacttgt 7440
   acaattcttc tgcccacatg gaatctgagt ggttttgtaa aatatgtgaa atggatgtct 7500
   gttaatctga tgtaaaccat acctttcttc tttgtttagg tggttagttc tcgtcaatgg 7560
   cctcttatct ctagatatag agcatcagtg cacacccaat ctgccagact agaaatgatg 7620
   tcctcgttgt ttaagccgcg gggtactgat gatgatggcc tcatccggta agagatatct 7680
   tcttagtatg ttgatgtatg gtaagcgtga tgacttttgg atgctaagtt tatcttcgta 7740
   tttgggactt ccagggaatc actgatcgac ttctacacta gctctggaaa gcgaaaacca 7800
   gaacacataa ttattttcag gtctgaagct ttggggttat tgttagcttg tatcactaaa 7860
   tctttttgta gaaacaaatt cttttttttg tttttgtttt gagaagtatg tatggtacat 7920
   tctttgctgg gatggattat tgatactttc ttagccgccg tggcgtaaat gtaaccattt 7980
   gctgggtttt agggatggag tcagtgaaag tcagtttacc caggtcatca acattgagct 8040
   ggatcagatc atcgaggtaa tacatttgtt tgattcgtcc tttgcacatt tgcttgctta 8100
   gttcaggaac gaattgcaag gtaacacatc cttgtaatta atgcaggcat gtaagtttct 8160
   ggatgagaag tggtcaccca agttcactgt gattgttgct caaaagaacc accacaccaa 8220
   gttctttcag acggcatcac cagacaatgt tcttcctggt aatatgttta tttttgtcat 8280
   actaaaggga gtgtggtgag atgcggtgac aataataaat gtgtttgttt gttacgcagg 8340
   aactgtggtg gatagtaaag tttgccatcc taagaacttc gacttctaca tgtgtgcaca 8400
   tgctgggatg attgtgagtt gatctcagaa ttgagcaaaa agatgctgct gtcttgttct 8460
   tgtctctgaa tcctagatgg ttcttcgtct cagggaacaa caaggccgac ccactatcat 8520
   gttctgcacg acgagatagg tttcagtgcc gacgagatgc aggagtttgt tcattcgctc 8580
   tcttacgtgt aagtagtagt agtaaaatgc tgttttggct gtgtgtgtgt gtgtgtgttt 8640
   tgtgcttacg ctgcgtgtcc tgctgtgcag gtaccagagg agcacgacag ccatctcagt 8700
   gggtaggccg cttgaatcta aagccatctt gtgcagcaca gggacagcag gttgattttt 8760
   ttctagtgtc tgtttttgtg tttgcagttg ctccagtgtg ctacgcccac ctcgctgcag 8820
   cccaggtgag cacgttcctg agattggagg agatgtcaga cgcgtcctcc agccagggag 8880
   gagggcatac ctcggctggc agtgctcctg tgccggagct gcctcgcctg catgacaaag 8940
   tcaggagctc catgttcttc tgctagctga tgtgcgtgcg catcaggatc gagctccatg 9000
   ttttgtgtta gtaaggccta gttagtaagg ctgtagaaag aatgtttaat gtttgcatgc 9060
   taaagtccaa acaatcaaaa ccactactat atctaccaga gcactgatcg atcaaacaac 9120
   aagagtcagc atcaatcaat caaaaccacc actattctat ctaccgatca atctactcta 9180
   tacctgaagc actcactcgc tcgcaaacaa ccaagggatc atggatcctt gagtagctcc 9240
   attgtccagt ccctgacctt gacgaagcat ttcatggaca gcctctcgta cgccatctcg 9300
   tacatccgga ggttcatgta ggcgaagctc aagtaggtgg cgatgcactc ttggtgcctg 9360
   tggtacagcg ccgggatggt cagagcggcc accacacttg cgtagcaggc ggtcgggaaa 9420
   tctgtaacgc tgccgacgat ggacacgctc cacaggcaga ggaaggctct gtagaagagc 9480
   agcgagtccc tgcccagagc gatgtcccgg aaagcgtccg aggcggcgtc cagagcagag 9540
   cgcagcagcg ccgccgcctc gtccacggcc tgctgcggaa cgcgcagctc cgggacggga 9600
   ggctgcggcc tgttcaggag gcgcgccgct ttggcccaca ggaagagcac ggtgaggagc 9660
   aggagcagca cgttggaggc gagggacacc aaggtgtagc cgccgtacaa gagcagccag 9720
   gaggagacgg tgcccgccag caggcaggcg ctcacgtcgg cgcggccccg gcgccacagc 9780
   agcacgtccc agactgctgc agcaatccga tccgattccg atccagatat attagttagt 9840
   agatccaatc tcgagcggcc aatccgtaat aataattggc tgagagaaaa ggtgggagag 9900
   agtaccgaat cctccgacgg cacatgtccc ggcgccgccg ccgccgccgc caacaccaac 9960
   accagcatcc accaccatcg tcgtagttaa tactaaggga agggaactag agaaggaagg 10020
   gaggggccga tgcaacggaa gaaatggatg gaattcgacc aacagctagc tagagcgaag 10080
   cagaacccaa tcccaatcaa aacccgagaa cgaacctgct tttactttcc caaccagacg 10140
   gtggctggct tggaatccca ctatccctca ttccctctac caacagggca ccggcctggc 10200
   ctccctcccc aatcatccaa ccaccacctt tttttttctt ccttttgctg tgttttctta 10260
   caaacttaaa tctactcgaa tattgattca agatattatg gagagtaatt gaatatttcg 10320
   atgaatcacg tcatgtatat atttcttaat tacaaacttc tatcactatc aaataaagta 10380
   aacataatgc tatttgcaat aatatatact atgtaaacat gactaaatca aaataaatgt 10440
   ttaatttaat agttaccaag tttgcaagaa aacatttcct tccaaagcac atatcatgat 10500
   gctggcaatt tagttttatc agctataata atatcattga caacacattt tgtaagtcca 10560
   cattctaaat aaattctgta cattataact aaaacattat atcatatcag cataaaaaat 10620
   aaattaagta ttctatgttt ctataaaaca ataaaccatg cattgtacgg tgactgcgtc    10680
   tagggaaaat tataccgcat gctagcaaaa cgtcctccag tatttcatta gacacattgg    10740
   tgtcaaccta atatcctatt tgttgtcgtt ccaactaacc ccctcttata gtgctacaac    10800
   actatcatct atgtgtgatg gttttactcg tgatacctca cttacaggtg aaaataagac    10860
   cctcaaaaag gaggcgaatg agctcattca cgtcttaatt aaagcctaca gtagtgaggc    10920
   tcacttgctt aaatgcttgg atagtcaaag attctaggta ttagactaag taactattct    10980
   gttttgctac aaatgtatta gactaatgct gaagggcaaa tgccgaaggt aggtttcgac    11040
   cagggcgcgc accaagactt tggtgatcat tgtgaacatg cttcggacga agctaattcc    11100
   gaaggtccta tgggcgacga caaactaag                                      11129
<210> 12
   <211> 11120
   <212> DNA
   <213> Artificial sequence
<220>
   <223> Nucleic acid
<220>
   <221> misc_feature
   <222> (3102)..(3102)
   <223> n is a, c, g, or t
<220>
   <221> misc_feature
   <222> (6214)..(6223)
   <223> n is a, c, g, or t
<220>
   <221> misc_feature
   <222> (6301)..(6310)
   <223> n is a, c, g, or t
<220>
   <221> misc_feature
   <222> (6316)..(6317)
   <223> n is a, c, g, or t
<400> 12
   tacacaagag gagcgaagca agtttcatcc aacaggcttc atcacggtga aacgggtata      60
   caacgtggct tcggtataac tcaaaggaag tataacgaag cacacttcat caagcaggct     120
   tcgtcataac gaaacaatga gatgcaagga gaaggtgtta ctcaagttat taaatacaac     180
   tgacgacatt gcccttatga catattgtaa actctgtatg taattattga gggtataaat     240
   gtaatagcgt acgaaccctt gcgattgtct ataaatagat gaacattacc ccgtactatt     300
   cacgttggat tgtaatcgct ctcgcgtcaa caccttcaaa caagccgaag atatcactgc     360
   aatataaatt ttgataatgt ttatatattt atcatggaat aaatatatga ctggatatca     420
   cactcatgta tttaaatcct tattgatata aatgatgagg atgtgttatt cacgaccttt     480
   gtctgaagat tattatatcc gaaaggagat attttttcga aggacgaatg ttcttaacat     540
   ttaataactg tgttaccttg ttcttgaaca tgtgacaaac attaggtttt gagtatttca     600
   attaatagtg aaattaaatt aaattttagt tgtccaaaat gacagacttc gatatttgtt     660
   ataattgttg agttaaattt taacagcatc cataaacaag taaacaacga ctatgtgtgc     720
   accagtgtct cctgtatcaa cggggtggtc cgtctggagg aggtaccgag gtcccgccgt     780
   tcgagagacg agctacgcat ttaagccggg gaggtcggag cagtgagtgg gagtagttca     840
   gggactggga gaggagagga agcaggacct gggagacctc gcctcgtccg tcctcctgcc     900
   tacttccttg cttttggtaa gtgaaagtga ttgaagtgga gatgcctcca atctgcagtg     960
   ctgtttcacg cgtaaaccct ggccccctcg ccgctggaag ccccattgtt cctctttcat     1020
   ttcttaccgc tgatgcagct tatggtttcc tagggttttc gagatatttg gtttcttgca    1080
   tccagaggac gaagagcttt catagggttt tcgagacatt gtcctcacaa tcgcatccat    1140
   tctgcagtgc tgtttcacgc gtaaacccta gcccctcgtc gctggaagcc cacatggttc    1200
   ctctttcatt tcttaccgct gatgcagctt atgggttcct agggttttcg agacatttgg    1260
   cttcttgcat ccagaggacg aagagctttc ctagggtttt cgagacattg tcctcacaat    1320
   cgcagatttg gttcgagaca tttgtttgag ataagtgcta ttatggacga agagggaagg    1380
   ggattcgacg aaatggaggc gttggcgttg gcttctgtgt tttggagacg cacgcgacag    1440
   ccaaactcca aaacggatac gagacagctc ttggggctgc gtaaacaggt attagttttc    1500
   tgtccccgtt taccgttccc gtgcgcagac gccgtcacgc gtactcttct tgtctccgtc    1560
   gccgcgctct acggcggtgc tggcgtccgt gctgtactcc gcgccggcag aggccgcgcg    1620
   cgcgtcgccc gcggacggcg ccacgccgct ccacctcgcc gcggccgtgc acatcctcct    1680
   cgccgcgggc gcgtccgtgg acgcgcgcgc cttctcgggc ctccgcgccg gggacctcct    1740
   cctcccgcgc gccaacgagg ccgccgccgc cgcagaccgg gcgctccgcg tgctcctgaa    1800
   gttccccgcg gcgtcactgt cgtcctcgcc caagaagtcc gcctcgccgc cgccggcccc    1860
   ggaggcgagg aaggagtacc cgcccgacct gacgctgccg gacctcaaga gcgggctgtt    1920
   cagcaccgac gagcaacgga cgcaccttac agctgtccta gcactaatta ctcctaaatt    1980
   catcatacac caaagtttca attagtaaaa ggttttggta ttattttctt tacaagacta    2040
   aaagcatcca ctcgtatttg ccatggaaat attgccaaaa tggttaccga agaaccaatg    2100
   aaaaatggcg gtatccgtcc acctgtaact tgtccaaggc tcttccagca gagtagtgcc    2160
   gccatgacga ttgacagaga cacgagacga aacaagcaga aggtccccgc ggcgtcactg    2220
   tcgtcctcgc ccaagaagtc cgcctcgccg ccgccggccc cggaggcgag gaaggagtac    2280
   ccgcccgacc tgacgctgcc ggacctcaag agcgggctgt tcagcaccga cgagttccgc    2340
   atgtacagct tcaaggtgaa gccctgctcc cgcacctact cgcacaactg gaccgagtgc    2400
   ccttcgtgca cccgggcgag aacgcgcggc gccgtgaccc gcgcgtactc ctacagctgc    2460
   gtgccctgcc ccgagttccg caagggccgg cgctgccgca agggcgacgg ctgcgagtac 2520
   gcgcacgacg tcttcgagtg ctggctccac cccggcgcag taccggacgc gcctctgcaa 2580
   ggacgaggtg ggctgcgcgc gccgcatctg cttcttcgcg cacaagcgcg aggagctccg 2640
   cgccgtcaac ccctccgcgg tgttcgtcgg catgcagatg cagcccaccg tgtcgccgcc 2700
   gccgcccaac ggcctcggcg acatgctcag cccggcggcc tggccctcct cccccgcgag 2760
   caggctcaat aaggccgcgc tcggcggccg ggacctggac cagtaccagc acatgttgtt 2820
   cgacaaggtg tcgtcgccga gggccagttg gagacaagaa gagtacgcgt gacggagatg 2880
   cgacggagaa aaagggtacg cgtgacggcg tctgcacacg ggaacggtaa acggggacaa 2940
   gaaactaata cctgtttacg cagccccaag tgctgtctcg tatccgtttt ggagtttggc 3000
   tgtcgcgtgc gtctccaaaa cagagaagcc aacgccaacg cctccatttc gtcgaatccc 3060
   cttccctctt cgtccataat ggcaattatc tcctcgtgtt cncgtcccgc gcttgagtcc 3120
   ttgtgatctg cacagcatga tgctgccacc aaatgcatgc agttagacaa gcttaatgtt 3180
   gcatctgata agactgtaca acagattcgt gtgccaaatg tgtccttgct actcaaggag 3240
   gtcatatgga acaagtaaaa cgaaatttct gcatcagcaa tcaggcacaa gactgaatga 3300
   cgtggttagt gccaatgagg tggggacttg ggaatggaat tagagttata gctattccta 3360
   taattcatct cactacagag gctctccatc ctactaggac tgtacaacag attcgtgtgc 3420
   caaatgtgtc cttgctactc aaggaggtca tatggaacaa gtaaaacaaa atttctgcat 3480
   cagcaatcag gcacaagact gaatgacgtg gttagtgcca atgaggtggg aacttgggaa 3540
   tggaattaga gttatagcta ttctatagct attcctataa ttcatctcac tacagaggct 3600
   ctccatccta ctagaattta atctgataaa gtaggcagaa acatggaact tataatgtcg 3660
   aagattgcat gtatagaatc taagtaacgg aaagattcaa ctttgtgatt atatgctgct 3720
   gttacatggt attggtaaaa cattctttca agtgctcatt gtcttccctt gcaaccaatt 3780
   cattgttgtc cttgtttcct tgcaggtagg tgctgcttgt tttatcttga aatgggctct 3840
   catgatggcg aggatgaaga gttgccaccc ccccctccgg tgccaccaga tgtgattccc 3900
   attaaagctg aagatgctgt gggtgaatca ccagcaaacc atatattaaa gccaaagaga 3960
   ttactgatgg acaggcctgg tataggaaga aaagggcagc cgacccagct ctattcaaat 4020
   cactttaaag tcgctgtgaa gagtacagaa gacgtcttct ttcactacta tgtatgtctg 4080
   ctgactgagt tcatgatcct ttgttcaaaa tatgtcattg tctgtccctt gtttataact 4140
   aatctttggc tgatgtgata ttgttatttt atttgactag gtaaacctga agtatgagga 4200
   tgatcgaccc gttgatggta aagggatcgg cagaaaggtg attgataaac tgcagcagac 4260
   atatcgtgca gagctttcta acaaggactt tgtatatgat ggagaaaaga gcctgtttac 4320
   agttggtggt cttccacaaa aaaagaatga gttcaccgtt gtcttggagg acgtatctac 4380
   tggaaagtta gttttagtct ttgatctgct ttcttgttgt ttatgcttac ccagaatcag 4440
   taattgccat gttttttgtt tgtggttgta ggactgctgc caatgggagc cctggaggta 4500
   atgacagtcc tggaggtggt gataggaaga gagtgaggag gccataccag acgaaaactt 4560
   tcaaagtgga gataaatttt gcagcagagg ttcctatgag tgctattggt caagtcatta 4620
   gaggcgaaga atctgagaac tccctggagg cgcttcgtgt tcttgatatc atactgaggc 4680
   agcattccgc agaacagtat gtagctatgc atcttgatgg atgattatag gtagatttga 4740
   ggatcttctg cagttacgac atgacaattt ttctcaatac ttgcagaggc tgccttttgg 4800
   ttaagcaatc atttttctac aacaaccctt catgctttgt tgacttgggt ggtggtgtga 4860
   tgggttgtcg tggatttcat tcaagcttcc gtggcacaca gagtggactt tccctcaatg 4920
   ttggtgcgtg ttcgtcctgt atggctgtat tggtgccatg taactgcatt gtctattcta 4980
   taatcttaca tctttcatga tcatgatagc cttttttttt gaaatccaca gatgtctcaa 5040
   caacaatgat cgtgaaacct ggccctgtta ttgattttct tctttctaac cagaatgtta 5100
   atgatcctag cagaattgat tggcaaaagg taaatgccat ctttggatga aacttctcga 5160
   ataaccatgc tagtgcattg aaatatctat tttgatagaa tgataacaca attttggggt 5220
   gtgcatattc aatacaggcc aagcgtgctc tcaagggctt gaggattaga accactcctg 5280
   caaattcaga attcaagatt tttggtctca gcgagaggat ctgcaaagaa caaacgtgtg 5340
   gacatgtttt cttaccgctc tgtttattgt catgcaagct ttactttaca tttttaaaaa 5400
   attgtaccct agtgtgttaa gcttgtttca gaagaatttt atttaacaaa tattgccttt 5460
   attgattttg ttctgattcc aatgccaagc ttgcttgatg gtgatatctg tgtgcttaat 5520
   tgctttctca ttgacatatg caattaggtt tccgctgagg cagagaaatg gtagcaacgg 5580
   agattgtgat accattgaaa taactgtcta tgactactat gcaaagaaag gaatcgatct 5640
   aaagtattct ggtgatttcc cctgtataaa tacagggaag gcaaagcgcc caacatattt 5700
   tccaatcgag gtttgtttca gttttgttag ttacatcctg tcaaatctct gtttattaaa 5760
   tatatcttgc atctcatatt cgatgggcag gcttggtggg gctgtctcac tgagtcacta 5820
   ggtcgtgggt tcaaagcagc ctctccacat ttgtgggggg gggaaggctt gcttcggttt 5880
   atcccttcct tagaccctag gtctgcccct ttttttgcat ctcatttttt tgtattgtgt 5940
   ttctagctat gctcgcttgt tccgcttcaa agatacacca aagctttgtc tacgctacaa 6000
   aggtcatccc ttgtggagaa gtctagacag aagcctgaag aaaggatgac cgttctaaat 6060
   gatgtgagct gttaccttga ttttagcatg ctgcgaccat tcataactgc atgggattta 6120
   tttctacgac tgaattaatc aacagcttat gattacccta aatgctaggc actgcaacgc 6180
   agtaactacg attctgaccc catgttgatg tgcnnnnnnn nnnaggttga gggcatgtgg 6240
   tgtttcagtt gctccaaaat ttacccaagt tgaaggaagg atccttcaag ccccaaaggt 6300
   nnnnnnnnnn gcttgnnaga gaatcctagg gtgtttggat ggaattctag gatttaaaga 6360
   tttttttcct atcctgattg ttaatatctc tatggatcca aacgctccct tagttgtttt 6420
   ctctctaaat gttgtaccaa gtcattatct cagtaaaatg catctgacta aatctaatat 6480
   cttgtagctg aaagccggca atggtgatga tatcttttca cgaaatggac ggtggaattt 6540
   cactaatagg gtcagcagct caacatgttt cttttttaac cttctttttt tctttattag 6600
   gaaggatttt tctgatgttt atttgccaat actttgcccc tttcagaagt tttatgaaac 6660
   ctgctctgtg aataagtggg cggtcgttaa tttctctgca cgttgtgatg ttcggaatct 6720
   tatccgtgac ctgatgagga atgcatctgc aaagggaatt gtatggttta gcttttcaca 6780
   tatcttctat tgtagcagtt ttctgctgct gacgttttct tctggcagca aatggaggaa 6840
   ccttttgatg tgtttgaaga gagtccctct atgaggcgtg cacctgtgtc aagaagggtg 6900
   gatgatatgt ttgggcagat aaaatcaaaa cttcctggag ctcctaggtt cctcttgtgc 6960
   cttctccctg agaggaaaaa ttgtgaaatc tatggttggt ttattttttg ttaccactcc 7020
   acataaatat ctcacatctt gcaataatta tacctttttg gtttcaacca ggtccttgga 7080
   agagaaagtg cctggccgag tttggtattg tcacacagtg tctagctcca ttaagagtca 7140
   atgatccgta cctgcttaat ttgctgatga agatcaatgc aaaggtttgg gacatgattt 7200
   tatctttctt ttgcactgta ttgttccctg gttcatgatt atattatcct ctttcctctg 7260
   tttttaacta gcttggtggt ctgaactcgt tgctgcaagt tgaagcatct tcgtcaatac 7320
   cacatgtgtc gcaagtaccc accatcatct taggtatgga tgtttcacat ggtcatccag 7380
   gacaagatag accttcggtt gcagcggtaa gatgggtgta cctttacttg tacaattctt 7440
   ctgcccacat ggaatctgag tggttttgta aaatatgtga aatggatgtc tgttaatctg 7500
   atgtaaacca tacctttctt ctttgtttag gtggttagtt ctcgtcaatg gcctcttatc 7560
   tctagatata gagcatcagt gcacacccaa tctgccagac tagaaatgat gtcctcgttg 7620
   tttaagccgc ggggtactga tgatgatggc ctcatccggt aagagatatc ttcttagtat 7680
   gttgatgtat ggtaagcgtg atgacttttg gatgctaagt ttatcttcgt atttgggact 7740
   tccagggaat cactgatcga cttctacact agctctggaa agcgaaaacc agaacacata 7800
   attattttca ggtctgaagc tttggggtta ttgttagctt gtatcactaa atctttttgt 7860
   agaaacaaat tctttttttt gtttttgttt tgagaagtat gtatggtaca ttctttgctg 7920
   ggatggatta ttgatacttt cttagccgcc gtggcgtaaa tgtaaccatt tgctgggttt 7980
   tagggatgga gtcagtgaaa gtcagtttac ccaggtcatc aacattgagc tggatcagat 8040
   catcgaggta atacatttgt ttgattcgtc ctttgcacat ttgcttgctt agttcaggaa 8100
   cgaattgcaa ggtaacacat ccttgtaatt aatgcaggca tgtaagtttc tggatgagaa 8160
   gtggtcaccc aagttcactg tgattgttgc tcaaaagaac caccacacca agttctttca 8220
   gacggcatca ccagacaatg ttcttcctgg taatatgttt atttttgtca tactaaaggg 8280
   agtgtggtga gatgcggtga caataataaa tgtgtttgtt tgttacgcag gaactgtggt 8340
   ggatagtaaa gtttgccatc ctaagaactt cgacttctac atgtgtgcac atgctgggat 8400
   gattgtgagt tgatctcaga attgagcaaa aagatgctgc tgtcttgttc ttgtctctga 8460
   atcctagatg gttcttcgtc tcagggaaca acaaggccga cccactatca tgttctgcac 8520
   gacgagatag gtttcagtgc cgacgagatg caggagtttg ttcattcgct ctcttacgtg 8580
   taagtagtag tagtaaaatg ctgttttggc tgtgtgtgtg tgtgtgtgtt ttgtgcttac 8640
   gctgcgtgtc ctgctgtgca ggtaccagag gagcacgaca gccatctcag tgggtaggcc 8700
   gcttgaatct aaagccatct tgtgcagcac agggacagca ggttgatttt tttctagtgt 8760
   ctgtttttgt gtttgcagtt gctccagtgt gctacgccca cctcgctgca gcccaggtga 8820
   gcacgttcct gagattggag gagatgtcag acgcgtcctc cagccaggga ggagggcata 8880
   cctcggctgg cagtgctcct gtgccggagc tgcctcgcct gcatgacaaa gtcaggagct 8940
   ccatgttctt ctgctagctg atgtgcgtgc gcatcaggat cgagctccat gttttgtgtt 9000
   agtaaggcct agttagtaag gctgtagaaa gaatgtttaa tgtttgcatg ctaaagtcca 9060
   aacaatcaaa accactacta tatctaccag agcactgatc gatcaaacaa caagagtcag 9120
   catcaatcaa tcaaaaccac cactattcta tctaccgatc aatctactct atacctgaag 9180
   cactcactcg ctcgcaaaca accaagggat catggatcct tgagtagctc cattgtccag 9240
   tccctgacct tgacgaagca tttcatggac agcctctcgt acgccatctc gtacatccgg 9300
   aggttcatgt aggcgaagct caagtaggtg gcgatgcact cttggtgcct gtggtacagc 9360
   gccgggatgg tcagagcggc caccacactt gcgtagcagg cggtcgggaa atctgtaacg 9420
   ctgccgacga tggacacgct ccacaggcag aggaaggctc tgtagaagag cagcgagtcc 9480
   ctgcccagag cgatgtcccg gaaagcgtcc gaggcggcgt ccagagcaga gcgcagcagc 9540
   gccgccgcct cgtccacggc ctgctgcgga acgcgcagct ccgggacggg aggctgcggc 9600
   ctgttcagga ggcgcgccgc tttggcccac aggaagagca cggtgaggag caggagcagc 9660
   acgttggagg cgagggacac caaggtgtag ccgccgtaca agagcagcca ggaggagacg 9720
   gtgcccgcca gcaggcaggc gctcacgtcg gcgcggcccc ggcgccacag cagcacgtcc 9780
   cagactgctg cagcaatccg atccgattcc gatccagata tattagttag tagatccaat 9840
   ctcgagcggc caatccgtaa taataattgg ctgagagaaa aggtgggaga gagtaccgaa 9900
   tcctccgacg gcacatgtcc cggcgccgcc gccgccgccg ccaacaccaa caccagcatc 9960
   caccaccatc gtcgtagtta atactaaggg aagggaacta gagaaggaag ggaggggccg 10020
   atgcaacgga agaaatggat ggaattcgac caacagctag ctagagcgaa gcagaaccca 10080
   atcccaatca aaacccgaga acgaacctgc ttttactttc ccaaccagac ggtggctggc 10140
   ttggaatccc actatccctc attccctcta ccaacagggc accggcctgg cctccctccc 10200
   caatcatcca accaccacct ttttttttct tccttttgct gtgttttctt acaaacttaa 10260
   atctactcga atattgattc aagatattat ggagagtaat tgaatatttc gatgaatcac 10320
   gtcatgtata tatttcttaa ttacaaactt ctatcactat caaataaagt aaacataatg 10380
   ctatttgcaa taatatatac tatgtaaaca tgactaaatc aaaataaatg tttaatttaa 10440
   tagttaccaa gtttgcaaga aaacatttcc ttccaaagca catatcatga tgctggcaat 10500
   ttagttttat cagctataat aatatcattg acaacacatt ttgtaagtcc acattctaaa 10560
   taaattctgt acattataac taaaacatta tatcatatca gcataaaaaa taaattaagt 10620
   attctatgtt tctataaaac aataaaccat gcattgtacg gtgactgcgt ctagggaaaa 10680
   ttataccgca tgctagcaaa acgtcctcca gtatttcatt agacacattg gtgtcaacct 10740
   aatatcctat ttgttgtcgt tccaactaac cccctcttat agtgctacaa cactatcatc 10800
   tatgtgtgat ggttttactc gtgatacctc acttacaggt gaaaataaga ccctcaaaaa 10860
   ggaggcgaat gagctcattc acgtcttaat taaagcctac agtagtgagg ctcacttgct 10920
   taaatgcttg gatagtcaaa gattctaggt attagactaa gtaactattc tgttttgcta 10980
   caaatgtatt agactaatgc tgaagggcaa atgccgaagg taggtttcga ccagggcgcg 11040
   caccaagact ttggtgatca ttgtgaacat gcttcggacg aagctaattc cgaaggtcct 11100
   atgggcgacg acaaactaag 11120
<210> 13
   <211> 28526
   <212> DNA
   <213> Artificial sequence
<220>
   <223> Nucleic acid
<400> 13
   ccaacaaact gactgatact cctttcgtcc acaaatctct gtcgcccgat cttttttcat      60
   ttttctatta gtcttcgttg tattagcatg ttcctctgtc ttctcagtat gtctgtcgct     120
   tcattacttc tgcgcattct ttaagaatct ttattgatat cgggtattat gaaaacttgt     180
   agtgattaaa agcaaatcga cgctccttgg tcactgcacc aatgcctaat acgctggaga     240
   tttaggaatg gatggagtat aatatactta gaaattaatt atataaacat agttaaaaat     300
   taatatatat gaagagtcgt gtaaagataa acttttaaat atagttgaga gtcaatatat     360
   ttgagaagac catcttttat cttttttatt tacattaaat gatattgtac ctgctctgac     420
   taaagcggtc agacggtgga gacagacaga ggacgactat cttttatatt tttatttaca     480
   ttaaatgata ttgtatcttt attgtcttgc cctaaagcgg tcagtgggtg gaggcagcca     540
   agagtcaccc tgtcgccgtt gccgtcgccg aggcaggcag agtctccctg tcgccgttgc     600
   cgtcgccggc ctcctcctcc tctgccactt cctcccaact cccagccgca ggggccgacg     660
   gcgacgggag ggaagaggcg gcagaggtac tccgaggggc gcggagagag gctgtgccag     720
   gccacgccgt ccagtgcctc cgggtccgtc gccgcagctt ccggccgcgt cggagaggta 780
   gcccccggag ctcttcgcgg aggctcggga atggtggtga gagtttccct tcgcgtcccc 840
   tcgtcttctc tgctctgcct ccttccgtgt actgtgaaga gatgctatct cgcgcaatga 900
   attcttgacg ctaataatat ggtccgctgc tacggatatt ccgcgcgtta gattggggtc 960
   tttgatgtgg cgaggtcatt acggtgtcct agccctcata ttcagcgctc agtgttgggg 1020
   ttttcttaac actggattga ggagataagt gctattatgg acgaagaggg aaggggattc 1080
   gacgaaatgg aggcgttggc gttggcttct gtgttttgga gacgcacgcg acagccaaac 1140
   tccaaaacgg atacgagaca gctcttgggg ctgcgtaaac aggtattagt tttctgtccc 1200
   cgtttaccgt tcccgtgcgc agacgccgtc acgcgtactc ttcttgtctc cgtcgccgcg 1260
   ctctacggcg gtgctggcgt ccgtgctgta ctccgcgccg gcagaggccg cgcgcgcgtc 1320
   gcccgcggac ggcgccacgc cgagataagt gctattatgg acgaagaggg aaggggattc 1380
   gacgaaatgg aggcgttggc gttggcttct gtgttttgga gacgcacgcg acagccaaac 1440
   tccaaaacgg atacgagaca gctcttgggg ctgcgtaaac aggtattagt tttctgtccc 1500
   cgtttaccgt tcccgtgcgc agacgccgtc acgcgtactc ttcttgtctc cgtcgccgcg 1560
   ctctacggcg gtgctggcgt ccgtgctgta ctccgcgccg gcagaggccg cgcgcgcgtc 1620
   gcccgcggac ggcgccacgc cgctccacct cgccgcggcc gtgcacatcc tcctcgccgc 1680
   gggcgcgtcc gtggacgcgc gcgccttctc gggcctccgc gccggggacc tcctcctccc 1740
   gcgcgccaac gaggccgccg ccgccgcaga ccgggcgctc cgcgtgctcc tgaagttccc 1800
   cgcggcgtca ctgtcgtcct cgcccaagaa gtccgcctcg ccgccgccgg ccccggaggc 1860
   gaggaaggag tacccgcccg acctgacgct gccggacctc aagagcgggc tgttcagcac 1920
   cgacgagcaa cggacgcacc ttacagctgt cctagcacta attactccta aattcatcat 1980
   acaccaaagt ttcaattagt aaaaggtttt ggtattattt tctttacaag actaaaagca 2040
   tccactcgta tttgccatgg aaatattgcc aaaatggtta ccgaagaacc aatgaaaaat 2100
   ggcggtatcc gtccacctgt aacttgtcca aggctcttcc agcagagtag tgccgccatg 2160
   acgattgaca gagacacgag acgaaacaag cagaaggtcc ccgcggcgtc actgtcgtcc 2220
   tcgcccaaga agtccgcctc gccgccgccg gccccggagg cgaggaagga gtacccgccc 2280
   gacctgacgc tgccggacct caagagcggg ctgttcagca ccgacgagtt ccgcatgtac 2340
   agcttcaagg tgaagccctg ctcccgcacc tactcgcaca actggaccga gtgcccttcg 2400
   tgcacccggg cgagaacgcg cggcgccgtg acccgcgcgt actcctacag ctgcgtgccc 2460
   tgccccgagt tccgcaaggg ccggcgctgc cgcaagggcg acggctgcga gtacgcgcac 2520
   gacgtcttcg agtgctggct ccaccccggc gcagtaccgg acgcgcctct gcaaggacga 2580
   ggtgggctgc gcgcgccgca tctgcttctt cgcgcacaag cgcgaggagc tccgcgccgt 2640
   caacccctcc gcggtgttcg tcggcatgca gatgcagccc accgtgtcgc cgccgccgcc 2700
   caacggcctc ggcgacatgc tcagcccggc ggcctggccc tcctcccccg cgagcaggct 2760
   caataaggcc gcgctcggcg gccgggacct ggaccagtac cagcacatgt tgttcgacaa 2820
   ggtgtcgtcg ccgagggcca gttggagaca agaagagtac gcgtgacgga gatgcgacgg 2880
   agaaaaaggg tacgcgtgac ggcgtctgca cacgggaacg gtaaacgggg acaagaaact 2940
   aatacctgtt tacgcagccc caagtgctgt ctcgtatccg ttttggagtt tggctgtcgc 3000
   gtgcgtctcc aaaacagaga agccaacgcc aacgcctcca tttcgtcgaa tccccttccc 3060
   tcttcgtcca taatggcaat tatctcctcg tgttcgctcc acctcgccgc ggccgtgcac 3120
   atcctcctcg ccgcgggcgc gtccgtggac gcgcgcgcct tctcgggcct ccgcgccggg 3180
   gacctcctcc tcccgcgcgc caacgaggcc gccgccgccg cagaccgggc gctccgcgtg 3240
   ctcctgaagt tccccgcggc gtcactgtcg tcctcgccca agaagtccgc ctcgccgccg 3300
   ccggccccgg aggcgaggaa ggagtacccg cccgacctga cgctgccgga cctcaagagc 3360
   gggctgttca gcaccgacga gcaacggacg caccttacag ctgtcctagc actaattact 3420
   cctaaattca tcatacacca aagtttcaat tagtaaaagg ttttggtatt attttcttta 3480
   caagactaaa agcatccact cgtatttgcc atggaaatat tgccaaaatg gttaccgaag 3540
   aaccaatgaa aaatggcggt atccgtccac ctgtaacttg tccaaggctc ttccagcaga 3600
   gtagtgccgc catgacgatt gacagagaca cgagacgaaa caagcagaag gtccccgcgg 3660
   cgtcactgtc gtcctcgccc aagaagtccg cctcgccgcc gccggccccg gaggcgagga 3720
   aggagtaccc gcccgacctg acgctgccgg acctcaagag cgggctgttc agcaccgacg 3780
   agttccgcat gtacagcttc aaggtgaagc cctgctcccg cacctactcg cacaactgga 3840
   ccgagtgccc ttcgtgcacc cgggcgagaa cgcgcggcgc cgtgacccgc gcgtactcct 3900
   acagctgcgt gccctgcccc gagttccgca agggccggcg ctgccgcaag ggcgacggct 3960
   gcgagtacgc gcacgacgtc ttcgagtgct ggctccaccc cggcgcagta ccggacgcgc 4020
   ctctgcaagg acgaggtggg ctgcgcgcgc cgcatctgct tcttcgcgca caagcgcgag 4080
   gagctccgcg ccgtcaaccc ctccgcggtg ttcgtcggca tgcagatgca gcccaccgtg 4140
   tcgccgccgc cgcccaacgg cctcggcgac atgctcagcc cggcggcctg gccctcctcc 4200
   cccgcgagca ggctcaataa ggccgcgctc ggcggccggg acctggacca gtaccagcac 4260
   atgttgttcg acaaggtgtc gtcgccgagg gccagttgga gacaagaaga gtacgcgtga 4320
   cggagatgcg acggagaaaa agggtacgcg tgacggcgtc tgcacacggg aacggtaaac 4380
   ggggacaaga aactaatacc tgtttacgca gccccaagtg ctgtctcgta tccgttttgg 4440
   agtttggctg tcgcgtgcgt ctccaaaaca gagaagccaa cgccaacgcc tccatttcgt 4500
   cgaatcccct tccctcttcg tccataatgg caattatctc ctcgtgttcc tagagcttgc 4560
   gttttggatg tttttttcga gattgatggt ggtttggtcc tgttgttgat cgatgtggct 4620
   gatatgtact tcttaattcg tgtcacagca ctgggttagc gacagtgatg gcagtgataa 4680
   cttcgaatgg gacagtgatg gtaacgggga gcagacagtg agcttcaacg ctgctggtgc 4740
   tggttcatca gctctggcag cgacgaacac tgatgctcct ggcccatcga cacgggtaag 4800
   ccagctaacg gatccataca tatatgcaac aatgtgtgag ttcagcagct agttcgtgtt 4860
   actcaggggt gttcaattta aaccacttgt gccaaactgg ccggggtggg gttgggtggt 4920
   ggatataata atcttgaaca aacgcaggat catgattagt ctacagtgac acggacaccc 4980
   tcaatgttac tgttaccttg tttctagtgg ctacgattgt aaggtttcta tttgtttgtg 5040
   gggttggttt taaggttgct aatggcaatg ggaaggctgg gcgatctgcc tctttggttc 5100
   agaagtatgt ggacatgggt ttctcagaag agattgttct gaaggccatg aaggacaatg 5160
   gtacttaact aatagctcat ctcttctcat ttatttagat tctatgtggt ttaatatacc 5220
   ctatatcaca gttttttttt ctttcagggg ataatggagc agattcatta gttgagctcc 5280
   ttcttactta ccaggtattc atttgtgttc ctgcttctat gttattcttt gatgatgttg 5340
   ttgcatggta tcaaagagaa tttattttac aaagtgaata agtgatgcca aatgcaggaa 5400
   ctaggcaatg acctcaaagt ggataatgac tttgcttcta gttgtgcccc caaaactgct 5460
   gacgatagtg atgatgatga cacactggaa atctgggatg atgaggatgc tggagggaga 5520
   agcaccaggg ttgctaactc tgttgatgat tctgatgacg aggtattttc atgctcccct 5580
   tggtgattgt aagcgtttac tttattcttt tttgagaaac ctactatgca tttactttgc 5640
   ttcaatcata tatacaacga caaacagcaa caacaaaatc ttttattcca aacaagttgc 5700
   gtagactaga gttaaaaaat taatagaaca acaagtcatg gttcggccac atggatagtt 5760
   gttttttatg aactcatatt caaggctaaa tctttgggtt gggtatatcc catccttatc 5820
   tccatttact gtctcttccc atgtcaactt tagtcttcct cttcctccct tctcaattct 5880
   cataactaag gcgccttaga attccactac gcacttgcgt ctatggaggt ctacgttgga 5940
   catgtccaaa ccatctcaaa cgttgttgga caagtttttc ttcaactggt gctactccta 6000
   gcctatcacg tatatcatca ttccggattt gatctcttct tgtatggcca caaattaaac 6060
   acaacataca cattttcggg acacttaatt gttgaacatg tcgtcttttg tagggcaaca 6120
   ttctgcacca tacaacatag aaggtttaat cattgtccta taaatctttt cttttagctt 6180
   ctgtggtacc ctcttgtcat atagaactcc agatgcttga cgccacttca tccatccgct 6240
   tcgattctat ggctaacatc ttcattaata tccccgtctc tctatagcat tgatcctaaa 6300
   tacctaaatg tgtccttcta ggcactgcct gactttccaa actaacatct cctttctcat 6360
   gtgtagtccc gaaatcacat ttcatatatt ttgttttagt tgtactgagt ctaaaacctt 6420
   tggattctag agtctctcgc cacaactcta gtttcctatt tactcttgcc caccacataa 6480
   cattccttgg tatcttgtca taattttcca tgtcaatgaa atgtcacacc cggatttaag 6540
   ggataaagcc gggtgtgtct catatgtgcg ccaaaaaaga acaacacata taatgataaa 6600
   gtgtatagag ataaatgtca caatatcata aaagtaatta ttacatagcg gaagtcttac 6660
   aaaaataaat gataaatata aaacgaacta atgtccatcc atggcgccat aacgttgact 6720
   gggagacgcc acctagttca actcgtatgc ttcattgtag ggcggctcct cttcgaccac 6780
   ctgctattaa cctgtggggg gggggggggt ttatatatcg caagagtgag ctcacaaaat 6840
   atcatagctt aagtgattaa gtgagaactc aagaagaaaa atcaatcaca ataaaagatg 6900
   acacaagaga cacagtgatt ttatcccgtg gttcagccaa tgcctactcc acgttgtggt 6960
   gacctccttc ggtcaaggat tgcactcaat ccctctcaag tgatccaaca atcaaacttg 7020
   agtatcacag ttttcttcct tagtgatgtt tcccgtttgt gaggaatctc cacaagttgg 7080
   agcctctcac ccttacaatg ttgatcacaa gaaaagcaca agagtaaggg agggaaagaa 7140
   acacacacaa gactagaatc gcaacaacaa tacacacaca agccaagaag agagcacaca 7200
   aaaacaatgc cacaagttca caacacactt agtgctcaaa tctcagtcac gatgatccaa 7260
   atgcgtattt gcggagtcta ggcgtcttag gatgtttaga gaatgcttgg tgtagtgctc 7320
   catgcgccta ggggttcctt ttatagcccc aaggcagcta ggagccgttg gaattccatt 7380
   tggaaaggta attcttgcct ttagaagctt atgaaggttc ttcattccaa catgtgctag 7440
   atggcggtgc cagagccagc ccatattagt cttagcgatt aagcatgcat ctagatcgac 7500
   attctctttt gagaaatcaa ctaaatagag cttgccgtgt aatacagcct taaaagttaa 7560
   tgaacaatca ctccttctaa agacagatac atcaatattt gtaaataaac aattatagcc 7620
   catatgacat aattgactta caaacaataa attatacccg agcgattcta ctaaaaacac 7680
   atttgaaatt gaatgctcgg ttgtgatggc tatcttgctt aagcccttaa ccttgccttg 7740
   gtttccatct ccaaaaatga tagtatcttg ggaatccctg ttcttgatgt aagaggtgaa 7800
   cattttcttc tcccccgtca tgtggttttt gcatccgcta tcaataatcc agcttgagct 7860
   cccggatgca taaacctgca aggtaattaa gcttggcttt taggtaccca agtcttgttg 7920
   ggtcctacaa ggttagttac aacaaatttt ggaacccaaa tgcatgtttc tcccttgcat 7980
   ttagatccca attttctagc aactactttg gcatttttac atgacaatac aaaagaaaca 8040
   ttacatgttt gatacatagt agttggacca gtgtatactt tcctaggtgc atgagaatca 8100
   ctatgattac gcctaggcct acttctacca tgaacatatg tggagctaga tgcaaacata 8160
   gcatgagaat tatgataagt aggtagagca acatgattat aacacttatc atgatgggca 8220
   acactggaaa ttttcttgac atgagcataa agataagcat gattcttttg accatgtgaa 8280
   gaatacaagc tactagccat aggagccttc cctttctctt tgttgagact ggaagtcctt 8340
   tggcttgtta agttcttggt ttcttatcgg aaaccaagtc catccttaat tgaggggtgt 8400
   ctaccaatga tgtaggcatc cctagcaaat ttgagtttct cataattatc tttgcaagtc 8460
   ttaagttgag cattaagact tgcaacatca ttgtttaact taataatagt agaattatgt 8520
   tcattacaag catcaacgtt aaaatttttg catctattgc aaataacaac atgctctatg 8580
   gatgaactag cagaatttgc ttcttttaac ttagcattta aatcatcatt taaattcttt 8640
   aggctagaga tagattcatt ataagcagat aattcagaag tcaatagttc attcttctta 8700
   gtttctagag caagggcttt ttgaacatta atgaatttat catgttcttc ataaagaata 8760
   tcttcttgct tttctagaag cctatccttt tcattaagag catcaattaa ttcatttatt 8820
   ttgtctactt tagttttatc taatccttta aacaaatcag tatagtccac ttcatcatca 8880
   gatgattcct catcactaga agaagaatac ttgggggtat ctcgaatacg tacctttttc 8940
   tccttagcca tgaggcacgt gtgtcgttcg ttggagaaga ggaaagactt gttgaaggtc 9000
   gaggctagtc cttcatcatc gaagtcggat gaagaacagt cggagtccca ctctttttca 9060
   atgtgcgcct cgcctttcgc cttcctgtag ttcttctttc tttccttctt cccatgcttc 9120
   tcttgtgcct ggtcattatc gttatcgagg cgttgtgcta taaaatgacc agacttaccg 9180
   catttgaagc aggagcattt tccccttgat ttgctcttgt tagggtactc cttgcgtcct 9240
   tttaaggcgg tcttgaagcg cttgatgatg agtgccatct catcctcgtt gagcccggca 9300
   tcctccactt gtgctagctt gctaggtagt acctccttgc tgctggttgc tttgagagca 9360
   acgggctgcg attcgtgtag aggaagcggt ccgtttaatg catcatccac atacctcgct 9420
   tcctttacca tcatgcgccc gctaacgaat tttccgatga tttcctcggg cgtcatcttg 9480
   gtgtacctgg ggttctcacg attaaggttt ataacatgag gatcaataac agtaaatgac 9540
   ctgagcatga gtcggacgac gtcatgatcc gtccatcttg tactcccgta gcttcggatt 9600
   ttgttgacaa gggtcttgag cctgttgtac gtttgtgttg gctcttctcc ccttatcatc 9660
   gcgaatctcc ctagctcgcc ttccaccagt tccattttag ggattatggt ggcatcattt 9720
   ccctcgtgag aaattttgag ggtgtctcat atttgtttgg cattatctag gccactttgt 9780
   tgtattcatc cctgcataga gatgctaata aaatagtagt agcttgtgta tttttgtgta 9840
   tttgctcatt gataaacaca gggttgttag agctatcaaa atgcattcaa ttttcaacaa 9900
   tctcccagat actaggatgg agggagaata aatgactacg cattttatga ctccaaaaag 9960
   aataatcctc tccatcaaag tgtgggggtt ttccaagtgg aatagagagc aaatgggcat 10020
   ttgaattgta ggggatgcga gaataatcaa aggaataatt ttgattaagc gttttctttt 10080
   tcgatgaaga gtcttcgtcg tcgtcatccc ttggtgaaga ggaggcgtca ctgtcgtagt 10140
   agatgatctt ctttatgtgc ctcttcttct ttctgtcctt cttcttgtga cttgagcctg 10200
   agtcagtggg cttgtcatct cttggctcgt tgaagaggga ctccttctcc ttgtcgttga 10260
   tcaccatccc cttaccctta ggatccatct ctttggtcgg ttagtcccta aatgaagagt 10320
   acgactctgc taccaattga gagcacctag aggggggtga ataggtgatc ctgtaaaatc 10380
   aagtactaaa tagtccaaac ttggttacga aatgttagtt agatcaaaac caagatgcta 10440
   tagcgagaac tcttcacttg attgttcctt tagaatgagt attaaactta ggaacaagag 10500
   cacaagtgat taagcggcct atgatggttt ctttattggc tcaacaacct ttaggtcttg 10560
   gaggaggatt tggaagagtt gggctcctgg aaaatgtaaa ttcttcatgt ggctggtggc 10620
   gcacggccgc tgctggaccg ctgaccggct ggcaaggaga ggcctgcctc atcctcaaag 10680
   ttgtctgtta tgtgaccagg aggaagaaac tatgaatcat ctgctagttt cctgtgtctt 10740
   ctcaagggaa gtctggttta atatcctgaa aacggtcggt ttgcagcacc tctctcccag 10800
   tctcgattct tcttcttttg atgattggtg ggagaatgta agcatgaccc ttattagccc 10860
   ctccgataag aagctgcgca aggggctgaa ctcccttatc attttaggag cttgggccat 10920
   ttggattcat cgcaatcaat gtgtcttcaa cggagagcag cctagggcta gcaaggtcat 10980
   aaattgggtc atggctgagt ctcatctctg gtgtagggct ggggctcgcg gattatctga 11040
   tatccttttt acctcggcat actatttgag gagttggtct ctagtatgct tagtgcactg 11100
   gtgctcctgc tcattttctt ttgttgggtg gtgtgttgag ttgtatgagg ttctttaaca 11160
   cctcacccca tctttttcta cttaatataa tgatacgcag ctctcctgcg tattcgagaa 11220
   aaaaaagaag aaaaaccaat cacaataaaa gatgacacaa gagacacggt gattttatcc 11280
   cgtgtttgac caatgcctac tccacattgt ggtgacctcc ttcggtccaa gaattgcaac 11340
   tcaatccctc tcaaatgatc caacgatcaa acttgagtac caccgttttc ttccttagtg 11400
   atgtttcccg tttgtgagga atctctacaa gttggagcct ctcaccctta caatgttgat 11460
   cacaagaaaa gcacaagagt aagggaggga aagaaacaca cacaaaacaa gaatcgcagc 11520
   aacaatacac acacaagcca agaagagagc acacaagaac aatgccacaa gttcacaaca 11580
   cactcagtgg tcaaatctca atcatgatga tccaaatgcg tatttgcgga gtttaggcgt 11640
   cttaggatgt ttagagaatg tttggtgtag tgctccatgc gcttaggggt cccttttata 11700
   gccccaaggc agctatgagc cattggaatt ccatttggaa ggcaattctt gccttctgtc 11760
   gggcggcaca ccggacagtc tggtgcacca ccggacatga acagtccttg ttcggtgccc 11820
   gatctccttc cttttctggc gaagccgatc gttgagccct tggtcccctt gtcgcaccgg 11880
   acattgtccg gtgcgaccaa gttaccgttg gcccaggcca cgcgtcaccc gctgattgcg 11940
   ctgccgaccg ttggcgaggg cgccgttggc tcaccggaca gtccggtgaa ttatagccgc 12000
   agcgccctcg gcgaattccc aagaacgacg agttcgtcgc cgagccagcc tgggcaccgg 12060
   acagtccggt gcacctcagg ctggttcaag tttggctgca cttagccaaa cttctccaat 12120
   ccaatcttag ttgattaacc aaggttccta gcacttagag aaatatgtta gtactcaaaa 12180
   acaatttact aaggccagag tcataccttg attctttgat ttgcatctct tccacactta 12240
   gtacatttga acctaagtaa tttgtgttgg gcatctaatc accaaaacat ttatagaaat 12300
   ggcccaaggg cacatttccc tttcacaaaa ccataaagag caatagcaaa aactacccaa 12360
   atagttcagg ggtaaacaag gtgaaaagat aaccagacta gggtgaccta ttgggtccca 12420
   tcaaaattaa acctatgcat gaataaatga ttataaagaa cattattggg taataaaagt 12480
   ggtaaagggc acaacttgcc ttcaatgagc tcctgctcag taacttctac ctgctaaaca 12540
   ccctgatcct cggccactgg ctcttctact cgtcacaata caaacaagca tggtataagg 12600
   aagaaattaa catcacacca aacatgtata caaaatacat aataataatc tacacattaa 12660
   aacgagatca taggaaaaag aatcactaaa ttcggagtta cagatttcaa gttatgattt 12720
   tccgaagtat ttatgtgctt ggtacaaaat tgattgtgtg atcaattcta atgtgggttt 12780
   catggtaaaa cagtaacact aagtgataaa caatattatt acaaaattat aggaactgga 12840
   atggattaat ttggacttaa tatgaattag ttttgtacta aaaatcaatt tccataatta 12900
   tttatccaat ttctctgttc tctgggccga gcactaaata taaggaaaca cgggggcaga 12960
   atcataagat ttccccagac tcagagcaac cctggggtgg acggcgggtt gattttaagt 13020
   attcctaagg gctcttttga gatattgcca cgcgaagggg tactgtgtga tctgagccgt 13080
   ccgatcaatt ccagacgctc ttgatttaat ttacccgagc gtgaaacggt acgctatggc 13140
   agccattaga tcgccgatcg acgacacgga ttcaaacacc ccacgccgcc accgagacgt 13200
   ccgatcggga tctagcggcg cggatctgtc caatcgaagg ggtacgcggc gtctaatcta 13260
   agccgtccat ttagcatcta acggttcaca ctcagtcgtc tacctctcag tcgtaatagc 13320
   gcgacggcgg cgaattctcc cgtgacggag atctctccgg cgagcaccca caggcgcacc 13380
   aacgccccga agccgaaatt gactaacaca tacaccctac gaggacatgg cgaataaact 13440
   ggggagtgaa tttacctcgg ttcacggcgg caaggtctct gttcacgaca caaggcggtg 13500
   cggcacccaa gcgaagcacc gacgagcaat taaggttgct ctgcttgtcg attcggccag 13560
   caagtgcttc ggtctccctc gcagcaactc ggcgcaggtt cctgactcga tctccggcac 13620
   tggacggtga cgggaacggg aacccgtgtc tccccacttc tgccacccct ggcttttctc 13680
   tccctaagct ctatacgcga cggcgagggt ttggcacgga agcgagaggc gcataaggtt 13740
   cggtgcagca gagttttatg gccgggtgag gttgggcggt tagcggccag ctgatccaca 13800
   aaccccgcgt ggtggcggac atctcggcca tggccgttgc ggaggtgagg gtgatgtttt 13860
   gacacggggg gcccagacgc agtgagggcg caacgcgacg tgttgagtcg gtctctcggg 13920
   gcccacggga cagaagcttc gtgcgaccga gttacgtaga ctggcaaggg ggccccgcaa 13980
   gtcagtcatg gattccggct gggccttgca gagaacgtgc tggttgggcc ggctaagccg 14040
   gattaggccc aggcgcacag ttcctttctt cctttttctt ttctatttta tttttcctat 14100
   ttctttcatt ttcagtttta agtttcgaat ttgaattcat atgtgtggag ctcatactta 14160
   ggttaaatgt tttcattcaa ataatagtat gaatcgaatt tatttattta attaatttaa 14220
   tttgtatagt atttttcttc ttttttctca gatcctagta ttccaattag atctttaatt 14280
   ccaaattggg ccagtaatat tttgctatta atattatttt tattgttacc aaatgcacac 14340
   acaaagaaac tccgacatga tgcacgattt ttttattggt tattggttaa ttacttactt 14400
   aaaataaggt tgttcgcatg tgatatcaaa tagaggaaaa tcatatagaa aaaaattatc 14460
   tcctttactt tgggttacaa gtttgggtat tacatgaaaa tcatgtgtag atccttcttt 14520
   tgctccctat actgctctat cacttgtctt gttaagaaaa ctagaacatg aggcgcccgt 14580
   aagggcgccc tatcaggact accgtataca aagctgaaca atagttataa ggacactaaa 14640
   gaaaggttca tgtatgtgaa tcacatggta acaggagtag tgatgatcgt tctttatttt 14700
   accaagatcg tcatccgtag ccttggactt ctctagaggc aacctaaacc acaacttgaa 14760
   agaggcagat gaaaaatata tcattcagat cagatataag catatctaga acaggataaa 14820
   ctgaggaaac gaggagtgag acatgaaagg caatacattc atatttgtca ggccttgcaa 14880
   aatttgtttc acaactctag caagatactc gatagggagg gactacctct gtgcaaacat 14940
   ggggttgttt gcataagtag cagaccatac cagtccattt aacatgtgtt tagaaattag 15000
   gatttccttt gcacaagaat tgatcaacat gtacattggc cacccgtagc aattaaaact 15060
   tcacctagag acaaattgta gaatgtgatt tatttatatg tagagtagag ctataagttt 15120
   ctcgttggat actttatctg tcctaaaata ggtgtatatc ttgcactccg agtcgacaaa 15180
   ttatatattt gattaagtat atgaaaaaac attaatattt atgatatcaa aacattaata 15240
   tttatgatat caaatagaca tcattaggtg agtcacattg tgtgtagtaa agatatttta 15300
   agatagaaat attaatacta tttttctaaa aatctagtca aagttgagaa tgtctggctt 15360
   ctcaaaatgt gaggtctaag atgtgcacct attgtaggat ggagtaacta tatagggtac 15420
   ataaaacaaa caaacaacgt agatatacca ttttaaatcg aaacacaagt aaaaacaaac 15480
   acctcgattg acgaatccca tttcttagta aattagttgg tggatgtgtc atgtacggta 15540
   agccgtacac aaggcgcagg cgcgctcgcg acatccggcg ggcagcggag cgcgccaaca 15600
   gtgcagcagc gacgcagatg gcaggcactc aagtttagga aagcagcagc cacctatggc 15660
   aagggataaa taggaaagta tcaatttggc tcttatggca aggaataagt aggaaagtag 15720
   caatttagca cttatggcaa ggaataagta ggaaagtagt attttggcag ggaagataat 15780
   tagggagctt gctccatttg tgtggccgcc ggccctatat atccccaagt gcgcagcact 15840
   gttgagggca ataaagtatc aagtcatctc ccacctctct ctctcaagcc tgcggttagg 15900
   aggaatactc ctaccggaga agacggccga aggctacgag ttacgtagcc gaggcccggc 15960
   gagtcaaggg ttcgacgctc ttgacagctt ggtatcccga gtccggagat ccttgcctca 16020
   cccagtcccg cccaccgatc cactaaccac accaccatcc ccgaccacgc ccaccagcgg 16080
   ttcgccatcc tccatccaca tggccgagcc cactttagcc gacgtcatcg agatgatgaa 16140
   ggccatgcag gccgacatca ccaggctgaa ggagaagtcc gactcctcct ccgcaagcgg 16200
   cgcccagccc gagcgtcatc gcgacatcga tcgcccgcca aggttccaga aatgggactt 16260
   tccgcggttt gatggcaagt ccgatcccct cctcttcatc aacaaatgtg agtcgtattt 16320
   tcgccagcag cgtacaatgg tggaggagca agtgtggatg gcctcgtata atctcgaggg 16380
   catcgcacag cactggtaca tacaactaca ggaagacgag ggcaccccgt cctggagcag 16440
   attcagggaa ctgctcaacc tgcgcttcgg gccagccctc cgttcagcgc cgttcttcga 16500
   gctcaccgag tgtcgacgca ccggcacggt cgaggagtac tccaaccgat tccaggagct 16560
   cctaccccgt gctggcagga tgacggagga gcaacgcgtc caactctaca ccggcggcct 16620
   gctgccaccg ctcagccacg cagtgcggat actcaacccc ggcaacctca acactgctat 16680
   gagcctagcg cggcaggtgg agcagatgga gctcgccaag atcccggcac cagccaagac 16740
   agggccgcgc ggcatcctgc cgccgccacc tccgcggcca gcgactcagg cgctcccggc 16800
   gccacaggcc cccttggcgc tgccagcacc gccactgtgc gcagcaacgg gccgtggcga 16860
   cggctccagg cgattatctc cagaggagat ggcggatcgg cgccgccaag gactgtgttt 16920
   taattgtaac gagaaatatt ctcgtggcca caaccgtttt tgcaggcgca tcttcttctt 16980
   ggatggcgtc gaaattgagg aggccgaggc ggaaacgggt gccgcggaga ccgacgctcc 17040
   ctgtttttcc ctccaggcag tggccggggt cgcgccaggg gacaccatgc agatcgccgt 17100
   caccctcggt tcagtctccc tggtggcact gctcgactcg ggaagcaccc acaattttat 17160
   atccgcagcg gcggcgcacc agtcgggcat ccccatccag cgccgacccc gcctcacggc 17220
   catggtggcc aatggcgagc ggatcacctg cgcgggcgtc cttcgcaatg cgccgctcca 17280
   cgtcaagggc gaactctacc cggctgacct cttcgtcatg ccattggccg ggtacgacgt 17340
   cgtcctcggc acacgatggc ttggtgcgtt ggggccaatc gtgtggaacc tcgccaatcg 17400
   ccgcatgacg ttccagcgcc aggggcgctc catctcctgg acgggcacgc ccagccccaa 17460
   ggagccggcg ctgcgcgcca ccaacggaac ggacgcccta ctggaggccc ttctcgcctc 17520
   gttcggggga ctcttcgcag ctccgatggg tttgcccccc aagcgcgccc acgaccatcg 17580
   cattctcctc aagcccgatg cgtcaccggt ggctgttcgg ccgtaccgct acccggctgc 17640
   acacaaggac gagctggagc ggcagtgcaa cgccatgatt gaccagggga tcgtgcgtcg 17700
   cagcgactcc ccattctcat cgcccgtgct cctggtcaag aagcccgatg gctcgtggcg 17760
   cttctgcgtc gactacaggg ccctcaacgc cctcaccatc aaggacgcgt tccccatccc 17820
   ggtcgtggag gagcttctcg acgagctcca cggcgccaaa ttcttcacca aactggatct 17880
   acggtcgggc tatcatcagg tccggatgcg gccagaagac gtgcacaaga cagcgttccg 17940
   cacacacgat ggcttatatg agttcctcgt gatggcgttc gggctctgca acgccccagc 18000
   cacgttccag gcactgatga atgacgtgct ccggcccttc ctccgacggt tcgtcctggt 18060
   attctttgat gacatcctca tttacagcag cacgtgggcc gatcacctcc gccatctacg 18120
   ggccgtcctc gacgagctac aacgccatca gctgttcatc aagcgttcga agtgttcttt 18180
   cggagcaaca tctgtcgcct acctcggcca catcatctcg gccgcagggg tggccatgga 18240
   cccggccaag gtggctgcca ttcacgactg gccgccgccg cgctcggtcc gggccgttcg 18300
   agggttcctc ggcctcaccg ggtactaccg ccgcttcgtc cacaactacg gcacggtggc 18360
   tgccccctta acggccctcc tcaaaaagga tggcttcgtc tgggatgacg cggccatggc 18420
   tgcctttacg gcgctcaagg cagccgtgtc gtctgctcct gtcctcgcca tgccagattt 18480
   cggcaagcgg ttcgtggtgg agtgcgatgc atcctccaca ggcttcggtg gggtgctcgt 18540
   ccaggaggga cacccggtcg cgttcttcag ccgacctatt gcaccccgcc accgcgacct 18600
   cgccgcctac gagcgcgaac ttatcggcct ggtccaggct gtccggcact ggcggccgta 18660
   cctatgggga cgacacttca tcgtcaagac ggaccacttc agtctcaagt atctcctcga 18720
   ccagaggttg gccacgattc cccaacacca ttgggtgggc aaacttctgg gattcgattt 18780
   tacggtcgaa taccgctcgg gcgccacgaa caccgtcgcc gacgcactat ccaggaggga 18840
   ccccgacgag ggcacactga tggccatttc agcaccccgt ttcgacttcg tcgagcgcct 18900
   gcgccaggca caggctacgg atccggctct cgtcgccctc tacgaagaga tccgcgtcgg 18960
   cacgcgcgct gcaccatggt ccatcatcgg tgggatggtc gccatggagg ggcgcctcta 19020
   tatcccagcc acttcacccc tattacggga gatcgtggcc gctgtacatg atgacggcca 19080
   cgagggcgtc caccgcaccc tgcatcgact acggcgcgac tttcattttc ccaacatgcg 19140
   tcgtattgtg caggaattcg tcagggcgtg cggcacatgc caaacataca agtccgatca 19200
   ccttcgtcca gctggtctcc tacagcccct gccgatccca aatggggtgt ggtcggacat 19260
   cggcatcgac ttcatcgaag ctctgccccg cgtgcaaggc aagacggtca ttctcactgt 19320
   cgtcgaccgt ttcagcaagt actgccactt catcccactg gcgcacccat acacggcgga 19380
   gtcggtggcg caagcattct gcgccgaggt ggtgaggctg cacggcatcc cgcagtccat 19440
   cgtatcggac cgagaccccg tcttcacgtc ggccttctgg agggagctaa tggccctgac 19500
   tggaaccaag ttgttcatgt cctccgcctt ccatccgcaa actgacggcc agtcggaggc 19560
   agccaaccgc gtcatcatga tgtaccttcg ctgcttcacc ggcgatcgac cccgacagtg 19620
   gctccggtgg cttccatggg cggagtacac ctacaacacc gcataccagt cctcactccg 19680
   cgagacacct ttccgggtgg tctacggccg cgacccaccg accatccggt cctacgagcc 19740
   gggtgacgca cgcgtgcccg cggtggcaca ggagatggag gaacgggctg ccttcctcga 19800
   cgacgttcgc taccgtctcc atcaggcgca agaatcccag aaacgcgcct acgatcagca 19860
   tcacaggcag gtcgtctaca atgtcggtga ctgggccctt cttcgtctcc gtcaacgacc 19920
   atcggcatcc ctgccacgtt ccaccacggg caagttgaag ccccgctacg tcggccccta 19980
   ccgtgtcaag gagatcatca acgaggcggc cgtacgcctt cagcttccat ccgacgccaa 20040
   aatccacgac gtcttccatg tgggcatcct caagaagttc gttggggcgc caccaacgga 20100
   accacctgcg ctaccggcca cactcaacgg cgcagtggtg tccacacctt cccgtgtcct 20160
   ggcgggtcgc ctggctcgcg gcattcggca agttctggta cagtggcacg gtgaaccagt 20220
   gtcgtctgcg acctgggagg attttgagga tttcagggca agcttccccg ctttccagct 20280
   cgaggacgag ctgactttcg aggccgggag agatgtcatg tacggtaagc cgtacacaag 20340
   gcgcaggcgc gctcgcgaca tccggcgggc agcggagcgc gccaacagtg cagcagcgac 20400
   gcagatggca ggcactcaag tttaggaaag cagcagccac ctatggcaag ggataaatag 20460
   gaaagtatca atttggctct tatggcaagg aataagtagg aaagtagcaa tttagcactt 20520
   atggcaagga ataagtagga aagtagtatt ttggcaggga agataattag ggagcttgct 20580
   ccatttgtgt ggccgccggc cctatatatc cccaagtgcg cagcactgtt gagggcaata 20640
   aagtatcaag tcatctccca cctctctctc tcaagcctgc ggttaggagg aatactccta 20700
   ccggagaaga cggccgaagg ctacgagtta cgtagccgag gcccggcgag tcaagggttc 20760
   gacgctcttg acaggatgcg agtcaaaatc atgtcatagt agattccatc taactcacca 20820
   gcacagctca aacaaaaaat gtaatggaca ataaggttat tccaaaattc taattccaga 20880
   gtctgatcac cttcgtatgg gtagtcccga tagcaacgaa actctccttg gtggtggcct 20940
   catcgtctgg tgtcttgcat ggcagcatgc tgaatgtcca gccttcaagg atgcatgtcc 21000
   tctgggtcat ccccttaaac accatcagtt gtaggaagat gaacctcaga agcatcaact 21060
   ctgcatcaca cagagacaaa gaggtaaggt ttgtgccgta aacatatgta tcttatccgg 21120
   aatcatagag aaaaggaaag gaagactgag ggtgtgtttg gattattgca tacctgaaaa 21180
   aagttacgtc ttgttgctgt caacattttt aaatagccaa ccgctcagtt tcattttggt 21240
   tcaattgtac gaaggacaat cagtgtccgg tgttcttgat agtcaaggct tagggcatac 21300
   aagttttatc ctcgtaaaac tgactttacc ttacattgtt ttgccatcct tttttatctg 21360
   tattatatcg ttatctatga tttacttatc ttgaagcgaa aatataaaga ttgggaaatg 21420
   gaggcacctt ctttgacctt gagcagctcc tcgaagagcg gcttttggtt cttcctcttg 21480
   agcgtctaca ggacacatca tcgaagcgct cctcgaaggc gaccccttat cttgaagcga 21540
   aaatataaag attgggaaat ggaggcacct tctttgacct tgagcagttc ctcgaagagc 21600
   ggcctctggt tcttcctctt gagcgcctgc aggacacatc atcaaagcgt tcctcgaaga 21660
   tgacctcacc ggaagcgagg aacaacaacg acaagagggc gaggaggcaa tagagcacgc 21720
   cgccacgctg gggcgtggag cccgcggtgg ccatgcgccc gcacgtgctc aaggtcccaa 21780
   gcagtgggga tgtcgcgcgc cgccgcggcc tcgggatctg catgctcgtg gggacgggcg 21840
   ccacgtcgtc gtctcgcgct ccggctccgt ccgcggagga ggctatgcga ccgtctttcg 21900
   tggccagtac gagatcctat ccacctcggt cgccgttgcc gccatcccct gttccctaga 21960
   gcgttccacg aaggtgaagg aaacccttga tgcaggtggg aagagatcgc agacatcaag 22020
   gtaggtggag ggagcaatag cactgtagca gtagcatact actcggcttg gctgttgact 22080
   gttgaggagg ctagaacttt tcggcacggc aagtttttta atataaggat tcatgagagg 22140
   acatgcatgc cgaaatgttt gaccagctga acaattcgcg ttcgtggtcg ctttccacac 22200
   gtcgggaccg gtgtggggaa aatcacgcga aacgcggaga gcgttccaaa acccatcgta 22260
   ttttaatact ttagatggct ttcattgttg accttccagg catgaaacca aattggttca 22320
   aagagatcct cgttattgct ttcaaacgat gcttgataac tctcccccat agcttcatag 22380
   tatgactcat caacttaatt ccttggtaat tattacagct ttaaatatct cccttattct 22440
   tgtaaatcgg tacaaataat acttctccac ttgttaggcg tattgttcga tcgaaagata 22500
   tggttgaaca acttggttag ccatactata gcaatatcct cgaggcatct tcatacctca 22560
   attggaatat cattaggatc catcacctta ccccctttca ttcttttcaa agcctcccta 22620
   accttagatt cttgaatctt acgcacaaag agcctattgg tgtcatcaaa agagtcatcc 22680
   aactgaaagg ttgtgtcccc attctcccca ttgattaatt tgtcaaagta ctcttaccat 22740
   atatgcctga tctcgtcctc cttcaccaag aggcgctctg tttcatcctt aatgcactta 22800
   actcgattga ggtctcttgt cttcctgtca cgaaccctgg ctatcctata tctgcccttc 22860
   tctcctttct tcgtactcaa gcattggtaa agatccttag acgcactatt ctttgctacc 22920
   atgtattttt ctatgctatc cacactccta cctactctta taaagcacca gtttccttgg 22980
   tttccaccat tgtccccaag gttttaaagt ctggcaacta gtcgagaagt cgatctgaac 23040
   gactcgactt tctctactag tcgctcgact cgttgactag tcacgagtag taacgactag 23100
   tcgcagcgat tgagtcgggt gacttatttg ggcctaggta taaaaatgtg agcccatcca 23160
   gccatctcaa ccctagtagg tatgagatgg aatgagatgg tatgaaatgc taatagacaa 23220
   gttttctacc ttatttatct atgatatgga atgattcatg acttgatgag gccaagacaa 23280
   atggttgaga tcttgtgagg catgcaacta tgttttgcaa ctactatgtt tgcaacatcc 23340
   ttccttacat tggctaatat gtggaaacaa agacaaggat cgaaagctct ctttgttagt 23400
   aatgaatggt gtgagagcaa aatgaagaac actgaagtag gaaatgcacg tgttgtttgt 23460
   cactctgttg cattttggca agcagtagaa gattgcatga gagcttcaca acctattcta 23520
   atattcctga ggatagttga tggtgatgag agactggcaa tggctatatg gtgggctgcc 23580
   atggaccatg aaaaaagagc atcaaaaaag ctttggagca catagaatca tagaaagaat 23640
   aatagatgaa gtaatttcta ttgttgataa ggggtgggac agtcagatgg ataccaatct 23700
   gtatagagca acattgtttt tggatccaaa caagttcttt gaaaccaaag agaacgatag 23760
   aagacttact acaatgctat attcaatatt taatgatata ttgtagaaga tggagatgga 23820
   tgaaaatata ctaaacaagg tgtttttagt gttctagcat gtttttagtc ttagcgtatg 23880
   tagaaagtta taaaaatata atcactgact tatgttcgac tagtcacgac tagtttgtcc 23940
   tagtctcgag tctctactag tcagtagttg cacgactcga ctcgaaaaca tcgattgtgt 24000
   gggaaaccct aaccctaaca aggggttggg tgatgtatta atagactggg tagttgggcc 24060
   tgacccatta cacaggggtg agaaggtaaa tacacggggt agaccccaaa ccctaaacga 24120
   gtactctaat actctaacag attgtccctt ttgcaaaaaa aggtccttat acttctttct 24180
   aatctaactg tagcccaact tatagatctt gatttcctcc cccatgtctt gcctaacaac 24240
   caaataagtg tgtgggtgtg tcacgctcga agcgtttaga tctaatttcc tctccttgtc 24300
   tggcgtaaca actagataag ataagtctta ccccttgcat gcacaaccaa ccaccacgag 24360
   tgcttcctcc tcctcgttat tggtgtcgta tattaacccc taacatcaca tctctttccc 24420
   catccctgcc tttctcaccc aaattcttcc gtggaggaca tgcagtaggc catcctgctg 24480
   gcctccaact cgccagtcgc tgtctccgcg ccctaccggc gccgccgcct ttaccttccc 24540
   ccgtcggcaa gcgcctcgat cttgtagcgc ctcctctctt tgccgccatg cgcctttgca 24600
   gtcagcgggg caagaacaac cgcgggagag taggaggcgt tgccgccgct ggcggtgcgt 24660
   ggccggacaa ttgagggaga ggaggcgctc tagattccga gtttagtttt gtattggttg 24720
   ttggcaaaaa aattgtgaga tattaaggtc gatgataata tatgtattgc tcgttttcat 24780
   gtaatcgttg tgcgctaata ttttatcgaa tatttttgtg ccgtcacaac acacgtgcat 24840
   tatactagtc atggtacaat gttagcattt ttttcttctc cttaatagcc ctttagactt 24900
   cctcattcca ctaccaagta tctttagctt cgcctctatt ctctctagtt actccacaca 24960
   cctcttaggt ggcctttcga atgcaggttt tcatcttctc ccacatgttg tttgtgtcat 25020
   cttccttcca agtgtcctct ttgagaagca agatgacaca aacattgaac ttgcattgta 25080
   ttttatggtg tttggcatgt gaattcattt agctgagagt tgattgaatt ggactacaat 25140
   tggacttccg tttttttaca cacatttggg aggggggcgg gagggcagac ccacccagtg 25200
   gtggaggttc ccacatgagt ggggttgggg aagggataaa ccaaggcaag ccttcccctc 25260
   gctaatacgg agatgctgat tcgaacccgt gtcctcgtga ctcactggga catctctcac 25320
   cactgcacta ggcctgcact ttatttgttt ttctcatttt tttttgtttt cctaacttgt 25380
   tcatagttat tgtacttccc gcttttttac acgttacagg aattgttgtt ttttgttctt 25440
   ttgaaggtaa ttgcaatagt agtttatgta gcttgtcaac tgtattctca ctgcttacat 25500
   gtgctttcta cacaggattt cttacatgag atgtcacgga aggacgaaaa agttgattcc 25560
   ttagttaaaa tggggtttcc tgaagacgag gctgcactgg ctattaccag atgcggtgtg 25620
   ctctctgaga ctttgttcca ttctcgtctc tttgcattag attcctcagc aaaataaaca 25680
   tcaatttcca accttgaaag taggatatgc tctcatatag aataaatcag ttgaataata 25740
   acattgccat gtttaattat atgtcaagct ttctattggt ttactctctg gattttgaga 25800
   attctcttca aatgctgtaa cttggaactc gtaaattggc tgcaatcttg acctgaggta 25860
   acctagaatg gttgaatctt tccaagcttg atttactgtt tgcatcaagt ttgggttaac 25920
   cagttatatt caaatctgaa gacaaagcat ttaggcatgc ccataataat ctgtagaaag 25980
   caaggcgtat gataaatatt atttcttaca agaataactg ggtgtttagg aagttatcca 26040
   tccaagtagt acatgtaaac ctttgtttct atgagtagca agcattcaga tgctccttca 26100
   aagcagtcaa cgaacagttt attgttcgtt tggggtcaga atattttaat ggcatttgct 26160
   gttaaataaa accttttttt gcatcttgca tgcaacttgt accgctcaga ttctgccccc 26220
   gtccctgttt tattttaact ggccatgcac cttacgccat ctactgtggt ctgtggttga 26280
   aaattactca gggccggatg catctatttc tgttctggtg gattcaatct atgcttcaca 26340
   gaccgcagga gatggttact gtggcaatct gtctgactat gaggtttgtc ttactgtcca 26400
   tacaagtctg caataagatt tagttctatt ataatggcct ggtaatggaa ctgtgtctgc 26460
   aatactgcag gataattcct atggagggag aagcacaggg aacaagaaaa agagaaaaag 26520
   atatggaggc caagcacagg gaagtagagg cccattagat ggtagctgtg atgaacccat 26580
   gcctctccca catccaatgg ttggatttaa cttgccagac cagtggtcaa gacgagtgga 26640
   cagatcgttg cctgcacaag ctattggtcc gccgtacttc tactacgaga acgttgctct 26700
   tgctccaaaa ggtgtctgga ctaccatatc aagattcttg tatgatattc aaccagagtt 26760
   tgtggactca aagtacttct gtgctgctgc caggaaaagg ggttacatac acaacctgcc 26820
   acttgagaac aggtcacctc tcctccccat acccccaaag acgatatcgg aagcatttcc 26880
   tcggaccaag aggtggtggc cttcatggga cccaagacga cagttcaatt gcctccagac 26940
   ttgcgtgtct agtgcaaaat tgttagagag gattcgcgta gccctcacaa acagttcaga 27000
   cccacctcct ccaagagttc agaagtatgt gttggaggag tgtaggaaat ggaacctggc 27060
   atgggttggc ttaaacaagg ttgctcctct agagcctgac gagatggagt ttctactcgg 27120
   ctttccgaag gatcacacga gaggtatcag caggacagag aggtatcgat ctctaggaaa 27180
   ttcatttcag gtcgatactg ttgcttacca tctctcagtt ctgaaggatc tgttcccaca 27240
   aggcatgaat gtgctgtctt tattctctgg tattggagga gcagaggtgg ctctccacag 27300
   gcttggcata cagatgaaca cggttatttc agtggagaag tccgaggtca accggacgat 27360
   tctgaagagt tggtgggatc agacgcagac gggtactctg attgagatca ctgatgtgca 27420
   gacactgtca tctgagagga tcgaggcgta tattagaaga attgggggct tcgatcttgt 27480
   gattggtgga agtccctgta acaacctcac tgggagcaac cgtcaccaca gagatggttt 27540
   ggagggcgag cattctgcat tgttccatca ttattttagg atcttacacg ccgtcaagtc 27600
   catcatggag cgtttgtagt tcttagaatc tttactgttg ttttggttct agtagaatac 27660
   tttgtgaccg aacttgtaaa tggtttgcag gttgactggt ttagttctct tgccactcta 27720
   atttaggcta gttttttttt tatttaatct tctttggtga tcttggtgca gttctgtggc 27780
   actgttgggt attcaaatgc agattgatcc gagaggctgc cctgttgcat tttcttttta 27840
   aaatttgtat ttgtcaatga gagatcaggt attctggttg ccaaaaaaaa atctgaagtt 27900
   gtggagctga gaaaaggacg gtgctaagct acaaccaccc actgccacac catgcgcacc 27960
   cccaacccaa aagatcctgc ttttgctcac gcttatcacc actcccatct ccatccaaaa 28020
   ccataaaaaa aaccagtgcg agctgaagca tccaggctct ggaatcatcg tcgggatgtc 28080
   tgtgccagca tcagcccagg tgcctcacac catacgatct gctgggagct gctgcttcag 28140
   gtcgtcgcat caactatctg cgcagaatct gggccgacgt gagttcccga tctcagcttt 28200
   gagtggcctt ctttttgaaa gtgagcacag gttcttccaa agcccgttgt gttcgtgtag 28260
   atcgcaggtg gcgggggacc ggaaagaaga agatcctttc cacccgtggc gtgcttgagt 28320
   ccagaaacgg cgcacatcct ggtggcctta tgaagagaag gtgagaacct ggtgaaagac 28380
   atgcggagct gtagctagac agctagctga tctagtagca gcgcacgcct tgcaggaagg 28440
   tcgtggagca catcatcctg ctcagagcca agcccaacat ctcagacgct gaagagaaag 28500
   acatgctgga ctacctatac acatcg 28526
<210> 14
   <211> 26772
   <212> DNA
   <213> Artificial sequence
<220>
   <223> Nucleic acid
<400> 14
   ccaacaaact gactgatact cctttcgtcc acaaatctct gtcgcccgat cttttttcat     60
   ttttctatta gtcttcgttg tattagcatg ttcctctgtc ttctcagtat gtctgtcgct    120
   tcattacttc tgcgcattct ttaagaatct ttattgatat cgggtattat gaaaacttgt    180
   agtgattaaa agcaaatcga cgctccttgg tcactgcacc aatgcctaat acgctggaga    240
   tttaggaatg gatggagtat aatatactta gaaattaatt atataaacat agttaaaaat    300
   taatatatat gaagagtcgt gtaaagataa acttttaaat atagttgaga gtcaatatat    360
   ttgagaagac catcttttat cttttttatt tacattaaat gatattgtac ctgctctgac    420
   taaagcggtc agacggtgga gacagacaga ggacgactat cttttatatt tttatttaca    480
   ttaaatgata ttgtatcttt attgtcttgc cctaaagcgg tcagtgggtg gaggcagcca    540
   agagtcaccc tgtcgccgtt gccgtcgccg aggcaggcag agtctccctg tcgccgttgc    600
   cgtcgccggc ctcctcctcc tctgccactt cctcccaact cccagccgca ggggccgacg    660
   gcgacgggag ggaagaggcg gcagaggtac tccgaggggc gcggagagag gctgtgccag    720
   gccacgccgt ccagtgcctc cgggtccgtc gccgcagctt ccggccgcgt cggagaggta    780
   gcccccggag ctcttcgcgg aggctcggga atggtggtga gagtttccct tcgcgtcccc    840
   tcgtcttctc tgctctgcct ccttccgtgt actgtgaaga gatgctatct cgcgcaatga    900
   attcttgacg ctaataatat ggtccgctgc tacggatatt ccgcgcgtta gattggggtc    960
   tttgatgtgg cgaggtcatt acggtgtcct agccctcata ttcagcgctc agtgttgggg   1020
   ttttcttaac actggattga ggagataagt gctattatgg acgaagaggg aaggggattc   1080
   gacgaaatgg aggcgttggc gttggcttct gtgttttgga gacgcacgcg acagccaaac   1140
   tccaaaacgg atacgagaca gctcttgggg ctgcgtaaac aggtattagt tttctgtccc 1200
   cgtttaccgt tcccgtgcgc agacgccgtc acgcgtactc ttcttgtctc cgtcgccgcg 1260
   ctctacggcg gtgctggcgt ccgtgctgta ctccgcgccg gcagaggccg cgcgcgcgtc 1320
   gcccgcggac ggcgccacgc cgctccacct cgccgcggcc gtgcacatcc tcctcgccgc 1380
   gggcgcgtcc gtggacgcgc gcgccttctc gggcctccgc gccggggacc tcctcctccc 1440
   gcgcgccaac gaggccgccg ccgccgcaga ccgggcgctc cgcgtgctcc tgaagttccc 1500
   cgcggcgtca ctgtcgtcct cgcccaagaa gtccgcctcg ccgccgccgg ccccggaggc 1560
   gaggaaggag tacccgcccg acctgacgct gccggacctc aagagcgggc tgttcagcac 1620
   cgacgagcaa cggacgcacc ttacagctgt cctagcacta attactccta aattcatcat 1680
   acaccaaagt ttcaattagt aaaaggtttt ggtattattt tctttacaag actaaaagca 1740
   tccactcgta tttgccatgg aaatattgcc aaaatggtta ccgaagaacc aatgaaaaat 1800
   ggcggtatcc gtccacctgt aacttgtcca aggctcttcc agcagagtag tgccgccatg 1860
   acgattgaca gagacacgag acgaaacaag cagaaggtcc ccgcggcgtc actgtcgtcc 1920
   tcgcccaaga agtccgcctc gccgccgccg gccccggagg cgaggaagga gtacccgccc 1980
   gacctgacgc tgccggacct caagagcggg ctgttcagca ccgacgagtt ccgcatgtac 2040
   agcttcaagg tgaagccctg ctcccgcacc tactcgcaca actggaccga gtgcccttcg 2100
   tgcacccggg cgagaacgcg cggcgccgtg acccgcgcgt actcctacag ctgcgtgccc 2160
   tgccccgagt tccgcaaggg ccggcgctgc cgcaagggcg acggctgcga gtacgcgcac 2220
   gacgtcttcg agtgctggct ccaccccggc gcagtaccgg acgcgcctct gcaaggacga 2280
   ggtgggctgc gcgcgccgca tctgcttctt cgcgcacaag cgcgaggagc tccgcgccgt 2340
   caacccctcc gcggtgttcg tcggcatgca gatgcagccc accgtgtcgc cgccgccgcc 2400
   caacggcctc ggcgacatgc tcagcccggc ggcctggccc tcctcccccg cgagcaggct 2460
   caataaggcc gcgctcggcg gccgggacct ggaccagtac cagcacatgt tgttcgacaa 2520
   ggtgtcgtcg ccgagggcca gttggagaca agaagagtac gcgtgacgga gatgcgacgg 2580
   agaaaaaggg tacgcgtgac ggcgtctgca cacgggaacg gtaaacgggg acaagaaact 2640
   aatacctgtt tacgcagccc caagtgctgt ctcgtatccg ttttggagtt tggctgtcgc 2700
   gtgcgtctcc aaaacagaga agccaacgcc aacgcctcca tttcgtcgaa tccccttccc 2760
   tcttcgtcca taatggcaat tatctcctcg tgttcctaga gcttgcgttt tggatgtttt 2820
   tttcgagatt gatggtggtt tggtcctgtt gttgatcgat gtggctgata tgtacttctt 2880
   aattcgtgtc acagcactgg gttagcgaca gtgatggcag tgataacttc gaatgggaca 2940
   gtgatggtaa cggggagcag acagtgagct tcaacgctgc tggtgctggt tcatcagctc 3000
   tggcagcgac gaacactgat gctcctggcc catcgacacg ggtaagccag ctaacggatc 3060
   catacatata tgcaacaatg tgtgagttca gcagctagtt cgtgttactc aggggtgttc 3120
   aatttaaacc acttgtgcca aactggccgg ggtggggttg ggtggtggat ataataatct 3180
   tgaacaaacg caggatcatg attagtctac agtgacacgg acaccctcaa tgttactgtt 3240
   accttgtttc tagtggctac gattgtaagg tttctatttg tttgtggggt tggttttaag 3300
   gttgctaatg gcaatgggaa ggctgggcga tctgcctctt tggttcagaa gtatgtggac 3360
   atgggtttct cagaagagat tgttctgaag gccatgaagg acaatggtac ttaactaata 3420
   gctcatctct tctcatttat ttagattcta tgtggtttaa tataccctat atcacagttt 3480
   ttttttcttt caggggataa tggagcagat tcattagttg agctccttct tacttaccag 3540
   gtattcattt gtgttcctgc ttctatgtta ttctttgatg atgttgttgc atggtatcaa 3600
   agagaattta ttttacaaag tgaataagtg atgccaaatg caggaactag gcaatgacct 3660
   caaagtggat aatgactttg cttctagttg tgcccccaaa actgctgacg atagtgatga 3720
   tgatgacaca ctggaaatct gggatgatga ggatgctgga gggagaagca ccagggttgc 3780
   taactctgtt gatgattctg atgacgaggt attttcatgc tccccttggt gattgtaagc 3840
   gtttacttta ttcttttttg agaaacctac tatgcattta ctttgcttca atcatatata 3900
   caacgacaaa cagcaacaac aaaatctttt attccaaaca agttgcgtag actagagtta 3960
   aaaaattaat agaacaacaa gtcatggttc ggccacatgg atagttgttt tttatgaact 4020
   catattcaag gctaaatctt tgggttgggt atatcccatc cttatctcca tttactgtct 4080
   cttcccatgt caactttagt cttcctcttc ctcccttctc aattctcata actaaggcgc 4140
   cttagaattc cactacgcac ttgcgtctat ggaggtctac gttggacatg tccaaaccat 4200
   ctcaaacgtt gttggacaag tttttcttca actggtgcta ctcctagcct atcacgtata 4260
   tcatcattcc ggatttgatc tcttcttgta tggccacaaa ttaaacacaa catacacatt 4320
   ttcgggacac ttaattgttg aacatgtcgt cttttgtagg gcaacattct gcaccataca 4380
   acatagaagg tttaatcatt gtcctataaa tcttttcttt tagcttctgt ggtaccctct 4440
   tgtcatatag aactccagat gcttgacgcc acttcatcca tccgcttcga ttctatggct 4500
   aacatcttca ttaatatccc cgtctctcta tagcattgat cctaaatacc taaatgtgtc 4560
   cttctaggca ctgcctgact ttccaaacta acatctcctt tctcatgtgt agtcccgaaa 4620
   tcacatttca tatattttgt tttagttgta ctgagtctaa aacctttgga ttctagagtc 4680
   tctcgccaca actctagttt cctatttact cttgcccacc acataacatt ccttggtatc 4740
   ttgtcataat tttccatgtc aatgaaatgt cacacccgga tttaagggat aaagccgggt 4800
   gtgtctcata tgtgcgccaa aaaagaacaa cacatataat gataaagtgt atagagataa 4860
   atgtcacaat atcataaaag taattattac atagcggaag tcttacaaaa ataaatgata 4920
   aatataaaac gaactaatgt ccatccatgg cgccataacg ttgactggga gacgccacct 4980
   agttcaactc gtatgcttca ttgtagggcg gctcctcttc gaccacctgc tattaacctg 5040
   tggggggggg gggggtttat atatcgcaag agtgagctca caaaatatca tagcttaagt 5100
   gattaagtga gaactcaaga agaaaaatca atcacaataa aagatgacac aagagacaca 5160
   gtgattttat cccgtggttc agccaatgcc tactccacgt tgtggtgacc tccttcggtc 5220
   aaggattgca ctcaatccct ctcaagtgat ccaacaatca aacttgagta tcacagtttt 5280
   cttccttagt gatgtttccc gtttgtgagg aatctccaca agttggagcc tctcaccctt 5340
   acaatgttga tcacaagaaa agcacaagag taagggaggg aaagaaacac acacaagact 5400
   agaatcgcaa caacaataca cacacaagcc aagaagagag cacacaaaaa caatgccaca 5460
   agttcacaac acacttagtg ctcaaatctc agtcacgatg atccaaatgc gtatttgcgg 5520
   agtctaggcg tcttaggatg tttagagaat gcttggtgta gtgctccatg cgcctagggg 5580
   ttccttttat agccccaagg cagctaggag ccgttggaat tccatttgga aaggtaattc 5640
   ttgcctttag aagcttatga aggttcttca ttccaacatg tgctagatgg cggtgccaga 5700
   gccagcccat attagtctta gcgattaagc atgcatctag atcgacattc tcttttgaga 5760
   aatcaactaa atagagcttg ccgtgtaata cagccttaaa agttaatgaa caatcactcc 5820
   ttctaaagac agatacatca atatttgtaa ataaacaatt atagcccata tgacataatt 5880
   gacttacaaa caataaatta tacccgagcg attctactaa aaacacattt gaaattgaat 5940
   gctcggttgt gatggctatc ttgcttaagc ccttaacctt gccttggttt ccatctccaa 6000
   aaatgatagt atcttgggaa tccctgttct tgatgtaaga ggtgaacatt ttcttctccc 6060
   ccgtcatgtg gtttttgcat ccgctatcaa taatccagct tgagctcccg gatgcataaa 6120
   cctgcaaggt aattaagctt ggcttttagg tacccaagtc ttgttgggtc ctacaaggtt 6180
   agttacaaca aattttggaa cccaaatgca tgtttctccc ttgcatttag atcccaattt 6240
   tctagcaact actttggcat ttttacatga caatacaaaa gaaacattac atgtttgata 6300
   catagtagtt ggaccagtgt atactttcct aggtgcatga gaatcactat gattacgcct 6360
   aggcctactt ctaccatgaa catatgtgga gctagatgca aacatagcat gagaattatg 6420
   ataagtaggt agagcaacat gattataaca cttatcatga tgggcaacac tggaaatttt 6480
   cttgacatga gcataaagat aagcatgatt cttttgacca tgtgaagaat acaagctact 6540
   agccatagga gccttccctt tctctttgtt gagactggaa gtcctttggc ttgttaagtt 6600
   cttggtttct tatcggaaac caagtccatc cttaattgag gggtgtctac caatgatgta 6660
   ggcatcccta gcaaatttga gtttctcata attatctttg caagtcttaa gttgagcatt 6720
   aagacttgca acatcattgt ttaacttaat aatagtagaa ttatgttcat tacaagcatc 6780
   aacgttaaaa tttttgcatc tattgcaaat aacaacatgc tctatggatg aactagcaga 6840
   atttgcttct tttaacttag catttaaatc atcatttaaa ttctttaggc tagagataga 6900
   ttcattataa gcagataatt cagaagtcaa tagttcattc ttcttagttt ctagagcaag 6960
   ggctttttga acattaatga atttatcatg ttcttcataa agaatatctt cttgcttttc 7020
   tagaagccta tccttttcat taagagcatc aattaattca tttattttgt ctactttagt 7080
   tttatctaat cctttaaaca aatcagtata gtccacttca tcatcagatg attcctcatc 7140
   actagaagaa gaatacttgg gggtatctcg aatacgtacc tttttctcct tagccatgag 7200
   gcacgtgtgt cgttcgttgg agaagaggaa agacttgttg aaggtcgagg ctagtccttc 7260
   atcatcgaag tcggatgaag aacagtcgga gtcccactct ttttcaatgt gcgcctcgcc 7320
   tttcgccttc ctgtagttct tctttctttc cttcttccca tgcttctctt gtgcctggtc 7380
   attatcgtta tcgaggcgtt gtgctataaa atgaccagac ttaccgcatt tgaagcagga 7440
   gcattttccc cttgatttgc tcttgttagg gtactccttg cgtcctttta aggcggtctt 7500
   gaagcgcttg atgatgagtg ccatctcatc ctcgttgagc ccggcatcct ccacttgtgc 7560
   tagcttgcta ggtagtacct ccttgctgct ggttgctttg agagcaacgg gctgcgattc 7620
   gtgtagagga agcggtccgt ttaatgcatc atccacatac ctcgcttcct ttaccatcat 7680
   gcgcccgcta acgaattttc cgatgatttc ctcgggcgtc atcttggtgt acctggggtt 7740
   ctcacgatta aggtttataa catgaggatc aataacagta aatgacctga gcatgagtcg 7800
   gacgacgtca tgatccgtcc atcttgtact cccgtagctt cggattttgt tgacaagggt 7860
   cttgagcctg ttgtacgttt gtgttggctc ttctcccctt atcatcgcga atctccctag 7920
   ctcgccttcc accagttcca ttttagggat tatggtggca tcatttccct cgtgagaaat 7980
   tttgagggtg tctcatattt gtttggcatt atctaggcca ctttgttgta ttcatccctg 8040
   catagagatg ctaataaaat agtagtagct tgtgtatttt tgtgtatttg ctcattgata 8100
   aacacagggt tgttagagct atcaaaatgc attcaatttt caacaatctc ccagatacta 8160
   ggatggaggg agaataaatg actacgcatt ttatgactcc aaaaagaata atcctctcca 8220
   tcaaagtgtg ggggttttcc aagtggaata gagagcaaat gggcatttga attgtagggg 8280
   atgcgagaat aatcaaagga ataattttga ttaagcgttt tctttttcga tgaagagtct 8340
   tcgtcgtcgt catcccttgg tgaagaggag gcgtcactgt cgtagtagat gatcttcttt 8400
   atgtgcctct tcttctttct gtccttcttc ttgtgacttg agcctgagtc agtgggcttg 8460
   tcatctcttg gctcgttgaa gagggactcc ttctccttgt cgttgatcac catcccctta 8520
   cccttaggat ccatctcttt ggtcggttag tccctaaatg aagagtacga ctctgctacc 8580
   aattgagagc acctagaggg gggtgaatag gtgatcctgt aaaatcaagt actaaatagt 8640
   ccaaacttgg ttacgaaatg ttagttagat caaaaccaag atgctatagc gagaactctt 8700
   cacttgattg ttcctttaga atgagtatta aacttaggaa caagagcaca agtgattaag 8760
   cggcctatga tggtttcttt attggctcaa caacctttag gtcttggagg aggatttgga 8820
   agagttgggc tcctggaaaa tgtaaattct tcatgtggct ggtggcgcac ggccgctgct 8880
   ggaccgctga ccggctggca aggagaggcc tgcctcatcc tcaaagttgt ctgttatgtg 8940
   accaggagga agaaactatg aatcatctgc tagtttcctg tgtcttctca agggaagtct 9000
   ggtttaatat cctgaaaacg gtcggtttgc agcacctctc tcccagtctc gattcttctt 9060
   cttttgatga ttggtgggag aatgtaagca tgacccttat tagcccctcc gataagaagc 9120
   tgcgcaaggg gctgaactcc cttatcattt taggagcttg ggccatttgg attcatcgca 9180
   atcaatgtgt cttcaacgga gagcagccta gggctagcaa ggtcataaat tgggtcatgg 9240
   ctgagtctca tctctggtgt agggctgggg ctcgcggatt atctgatatc ctttttacct 9300
   cggcatacta tttgaggagt tggtctctag tatgcttagt gcactggtgc tcctgctcat 9360
   tttcttttgt tgggtggtgt gttgagttgt atgaggttct ttaacacctc accccatctt 9420
   tttctactta atataatgat acgcagctct cctgcgtatt cgagaaaaaa aagaagaaaa 9480
   accaatcaca ataaaagatg acacaagaga cacggtgatt ttatcccgtg tttgaccaat 9540
   gcctactcca cattgtggtg acctccttcg gtccaagaat tgcaactcaa tccctctcaa 9600
   atgatccaac gatcaaactt gagtaccacc gttttcttcc ttagtgatgt ttcccgtttg 9660
   tgaggaatct ctacaagttg gagcctctca cccttacaat gttgatcaca agaaaagcac 9720
   aagagtaagg gagggaaaga aacacacaca aaacaagaat cgcagcaaca atacacacac 9780
   aagccaagaa gagagcacac aagaacaatg ccacaagttc acaacacact cagtggtcaa 9840
   atctcaatca tgatgatcca aatgcgtatt tgcggagttt aggcgtctta ggatgtttag 9900
   agaatgtttg gtgtagtgct ccatgcgctt aggggtccct tttatagccc caaggcagct 9960
   atgagccatt ggaattccat ttggaaggca attcttgcct tctgtcgggc ggcacaccgg 10020
   acagtctggt gcaccaccgg acatgaacag tccttgttcg gtgcccgatc tccttccttt 10080
   tctggcgaag ccgatcgttg agcccttggt ccccttgtcg caccggacat tgtccggtgc 10140
   gaccaagtta ccgttggccc aggccacgcg tcacccgctg attgcgctgc cgaccgttgg 10200
   cgagggcgcc gttggctcac cggacagtcc ggtgaattat agccgcagcg ccctcggcga 10260
   attcccaaga acgacgagtt cgtcgccgag ccagcctggg caccggacag tccggtgcac 10320
   ctcaggctgg ttcaagtttg gctgcactta gccaaacttc tccaatccaa tcttagttga 10380
   ttaaccaagg ttcctagcac ttagagaaat atgttagtac tcaaaaacaa tttactaagg 10440
   ccagagtcat accttgattc tttgatttgc atctcttcca cacttagtac atttgaacct 10500
   aagtaatttg tgttgggcat ctaatcacca aaacatttat agaaatggcc caagggcaca 10560
   tttccctttc acaaaaccat aaagagcaat agcaaaaact acccaaatag ttcaggggta 10620
   aacaaggtga aaagataacc agactagggt gacctattgg gtcccatcaa aattaaacct 10680
   atgcatgaat aaatgattat aaagaacatt attgggtaat aaaagtggta aagggcacaa 10740
   cttgccttca atgagctcct gctcagtaac ttctacctgc taaacaccct gatcctcggc 10800
   cactggctct tctactcgtc acaatacaaa caagcatggt ataaggaaga aattaacatc 10860
   acaccaaaca tgtatacaaa atacataata ataatctaca cattaaaacg agatcatagg 10920
   aaaaagaatc actaaattcg gagttacaga tttcaagtta tgattttccg aagtatttat 10980
   gtgcttggta caaaattgat tgtgtgatca attctaatgt gggtttcatg gtaaaacagt 11040
   aacactaagt gataaacaat attattacaa aattatagga actggaatgg attaatttgg 11100
   acttaatatg aattagtttt gtactaaaaa tcaatttcca taattattta tccaatttct 11160
   ctgttctctg ggccgagcac taaatataag gaaacacggg ggcagaatca taagatttcc 11220
   ccagactcag agcaaccctg gggtggacgg cgggttgatt ttaagtattc ctaagggctc 11280
   ttttgagata ttgccacgcg aaggggtact gtgtgatctg agccgtccga tcaattccag 11340
   acgctcttga tttaatttac ccgagcgtga aacggtacgc tatggcagcc attagatcgc 11400
   cgatcgacga cacggattca aacaccccac gccgccaccg agacgtccga tcgggatcta 11460
   gcggcgcgga tctgtccaat cgaaggggta cgcggcgtct aatctaagcc gtccatttag 11520
   catctaacgg ttcacactca gtcgtctacc tctcagtcgt aatagcgcga cggcggcgaa 11580
   ttctcccgtg acggagatct ctccggcgag cacccacagg cgcaccaacg ccccgaagcc 11640
   gaaattgact aacacataca ccctacgagg acatggcgaa taaactgggg agtgaattta 11700
   cctcggttca cggcggcaag gtctctgttc acgacacaag gcggtgcggc acccaagcga 11760
   agcaccgacg agcaattaag gttgctctgc ttgtcgattc ggccagcaag tgcttcggtc 11820
   tccctcgcag caactcggcg caggttcctg actcgatctc cggcactgga cggtgacggg 11880
   aacgggaacc cgtgtctccc cacttctgcc acccctggct tttctctccc taagctctat 11940
   acgcgacggc gagggtttgg cacggaagcg agaggcgcat aaggttcggt gcagcagagt 12000
   tttatggccg ggtgaggttg ggcggttagc ggccagctga tccacaaacc ccgcgtggtg 12060
   gcggacatct cggccatggc cgttgcggag gtgagggtga tgttttgaca cggggggccc 12120
   agacgcagtg agggcgcaac gcgacgtgtt gagtcggtct ctcggggccc acgggacaga 12180
   agcttcgtgc gaccgagtta cgtagactgg caagggggcc ccgcaagtca gtcatggatt 12240
   ccggctgggc cttgcagaga acgtgctggt tgggccggct aagccggatt aggcccaggc 12300
   gcacagttcc tttcttcctt tttcttttct attttatttt tcctatttct ttcattttca 12360
   gttttaagtt tcgaatttga attcatatgt gtggagctca tacttaggtt aaatgttttc 12420
   attcaaataa tagtatgaat cgaatttatt tatttaatta atttaatttg tatagtattt 12480
   ttcttctttt ttctcagatc ctagtattcc aattagatct ttaattccaa attgggccag 12540
   taatattttg ctattaatat tatttttatt gttaccaaat gcacacacaa agaaactccg 12600
   acatgatgca cgattttttt attggttatt ggttaattac ttacttaaaa taaggttgtt 12660
   cgcatgtgat atcaaataga ggaaaatcat atagaaaaaa attatctcct ttactttggg 12720
   ttacaagttt gggtattaca tgaaaatcat gtgtagatcc ttcttttgct ccctatactg 12780
   ctctatcact tgtcttgtta agaaaactag aacatgaggc gcccgtaagg gcgccctatc 12840
   aggactaccg tatacaaagc tgaacaatag ttataaggac actaaagaaa ggttcatgta 12900
   tgtgaatcac atggtaacag gagtagtgat gatcgttctt tattttacca agatcgtcat 12960
   ccgtagcctt ggacttctct agaggcaacc taaaccacaa cttgaaagag gcagatgaaa 13020
   aatatatcat tcagatcaga tataagcata tctagaacag gataaactga ggaaacgagg 13080
   agtgagacat gaaaggcaat acattcatat ttgtcaggcc ttgcaaaatt tgtttcacaa 13140
   ctctagcaag atactcgata gggagggact acctctgtgc aaacatgggg ttgtttgcat 13200
   aagtagcaga ccataccagt ccatttaaca tgtgtttaga aattaggatt tcctttgcac 13260
   aagaattgat caacatgtac attggccacc cgtagcaatt aaaacttcac ctagagacaa 13320
   attgtagaat gtgatttatt tatatgtaga gtagagctat aagtttctcg ttggatactt 13380
   tatctgtcct aaaataggtg tatatcttgc actccgagtc gacaaattat atatttgatt 13440
   aagtatatga aaaaacatta atatttatga tatcaaaaca ttaatattta tgatatcaaa 13500
   tagacatcat taggtgagtc acattgtgtg tagtaaagat attttaagat agaaatatta 13560
   atactatttt tctaaaaatc tagtcaaagt tgagaatgtc tggcttctca aaatgtgagg 13620
   tctaagatgt gcacctattg taggatggag taactatata gggtacataa aacaaacaaa 13680
   caacgtagat ataccatttt aaatcgaaac acaagtaaaa acaaacacct cgattgacga 13740
   atcccatttc ttagtaaatt agttggtgga tgtgtcatgt acggtaagcc gtacacaagg 13800
   cgcaggcgcg ctcgcgacat ccggcgggca gcggagcgcg ccaacagtgc agcagcgacg 13860
   cagatggcag gcactcaagt ttaggaaagc agcagccacc tatggcaagg gataaatagg 13920
   aaagtatcaa tttggctctt atggcaagga ataagtagga aagtagcaat ttagcactta 13980
   tggcaaggaa taagtaggaa agtagtattt tggcagggaa gataattagg gagcttgctc 14040
   catttgtgtg gccgccggcc ctatatatcc ccaagtgcgc agcactgttg agggcaataa 14100
   agtatcaagt catctcccac ctctctctct caagcctgcg gttaggagga atactcctac 14160
   cggagaagac ggccgaaggc tacgagttac gtagccgagg cccggcgagt caagggttcg 14220
   acgctcttga cagcttggta tcccgagtcc ggagatcctt gcctcaccca gtcccgccca 14280
   ccgatccact aaccacacca ccatccccga ccacgcccac cagcggttcg ccatcctcca 14340
   tccacatggc cgagcccact ttagccgacg tcatcgagat gatgaaggcc atgcaggccg 14400
   acatcaccag gctgaaggag aagtccgact cctcctccgc aagcggcgcc cagcccgagc 14460
   gtcatcgcga catcgatcgc ccgccaaggt tccagaaatg ggactttccg cggtttgatg 14520
   gcaagtccga tcccctcctc ttcatcaaca aatgtgagtc gtattttcgc cagcagcgta 14580
   caatggtgga ggagcaagtg tggatggcct cgtataatct cgagggcatc gcacagcact 14640
   ggtacataca actacaggaa gacgagggca ccccgtcctg gagcagattc agggaactgc 14700
   tcaacctgcg cttcgggcca gccctccgtt cagcgccgtt cttcgagctc accgagtgtc 14760
   gacgcaccgg cacggtcgag gagtactcca accgattcca ggagctccta ccccgtgctg 14820
   gcaggatgac ggaggagcaa cgcgtccaac tctacaccgg cggcctgctg ccaccgctca 14880
   gccacgcagt gcggatactc aaccccggca acctcaacac tgctatgagc ctagcgcggc 14940
   aggtggagca gatggagctc gccaagatcc cggcaccagc caagacaggg ccgcgcggca 15000
   tcctgccgcc gccacctccg cggccagcga ctcaggcgct cccggcgcca caggccccct 15060
   tggcgctgcc agcaccgcca ctgtgcgcag caacgggccg tggcgacggc tccaggcgat 15120
   tatctccaga ggagatggcg gatcggcgcc gccaaggact gtgttttaat tgtaacgaga 15180
   aatattctcg tggccacaac cgtttttgca ggcgcatctt cttcttggat ggcgtcgaaa 15240
   ttgaggaggc cgaggcggaa acgggtgccg cggagaccga cgctccctgt ttttccctcc 15300
   aggcagtggc cggggtcgcg ccaggggaca ccatgcagat cgccgtcacc ctcggttcag 15360
   tctccctggt ggcactgctc gactcgggaa gcacccacaa ttttatatcc gcagcggcgg 15420
   cgcaccagtc gggcatcccc atccagcgcc gaccccgcct cacggccatg gtggccaatg 15480
   gcgagcggat cacctgcgcg ggcgtccttc gcaatgcgcc gctccacgtc aagggcgaac 15540
   tctacccggc tgacctcttc gtcatgccat tggccgggta cgacgtcgtc ctcggcacac 15600
   gatggcttgg tgcgttgggg ccaatcgtgt ggaacctcgc caatcgccgc atgacgttcc 15660
   agcgccaggg gcgctccatc tcctggacgg gcacgcccag ccccaaggag ccggcgctgc 15720
   gcgccaccaa cggaacggac gccctactgg aggcccttct cgcctcgttc gggggactct 15780
   tcgcagctcc gatgggtttg ccccccaagc gcgcccacga ccatcgcatt ctcctcaagc 15840
   ccgatgcgtc accggtggct gttcggccgt accgctaccc ggctgcacac aaggacgagc 15900
   tggagcggca gtgcaacgcc atgattgacc aggggatcgt gcgtcgcagc gactccccat 15960
   tctcatcgcc cgtgctcctg gtcaagaagc ccgatggctc gtggcgcttc tgcgtcgact 16020
   acagggccct caacgccctc accatcaagg acgcgttccc catcccggtc gtggaggagc 16080
   ttctcgacga gctccacggc gccaaattct tcaccaaact ggatctacgg tcgggctatc 16140
   atcaggtccg gatgcggcca gaagacgtgc acaagacagc gttccgcaca cacgatggct 16200
   tatatgagtt cctcgtgatg gcgttcgggc tctgcaacgc cccagccacg ttccaggcac 16260
   tgatgaatga cgtgctccgg cccttcctcc gacggttcgt cctggtattc tttgatgaca 16320
   tcctcattta cagcagcacg tgggccgatc acctccgcca tctacgggcc gtcctcgacg 16380
   agctacaacg ccatcagctg ttcatcaagc gttcgaagtg ttctttcgga gcaacatctg 16440
   tcgcctacct cggccacatc atctcggccg caggggtggc catggacccg gccaaggtgg 16500
   ctgccattca cgactggccg ccgccgcgct cggtccgggc cgttcgaggg ttcctcggcc 16560
   tcaccgggta ctaccgccgc ttcgtccaca actacggcac ggtggctgcc cccttaacgg 16620
   ccctcctcaa aaaggatggc ttcgtctggg atgacgcggc catggctgcc tttacggcgc 16680
   tcaaggcagc cgtgtcgtct gctcctgtcc tcgccatgcc agatttcggc aagcggttcg 16740
   tggtggagtg cgatgcatcc tccacaggct tcggtggggt gctcgtccag gagggacacc 16800
   cggtcgcgtt cttcagccga cctattgcac cccgccaccg cgacctcgcc gcctacgagc 16860
   gcgaacttat cggcctggtc caggctgtcc ggcactggcg gccgtaccta tggggacgac 16920
   acttcatcgt caagacggac cacttcagtc tcaagtatct cctcgaccag aggttggcca 16980
   cgattcccca acaccattgg gtgggcaaac ttctgggatt cgattttacg gtcgaatacc 17040
   gctcgggcgc cacgaacacc gtcgccgacg cactatccag gagggacccc gacgagggca 17100
   cactgatggc catttcagca ccccgtttcg acttcgtcga gcgcctgcgc caggcacagg 17160
   ctacggatcc ggctctcgtc gccctctacg aagagatccg cgtcggcacg cgcgctgcac 17220
   catggtccat catcggtggg atggtcgcca tggaggggcg cctctatatc ccagccactt 17280
   cacccctatt acgggagatc gtggccgctg tacatgatga cggccacgag ggcgtccacc 17340
   gcaccctgca tcgactacgg cgcgactttc attttcccaa catgcgtcgt attgtgcagg 17400
   aattcgtcag ggcgtgcggc acatgccaaa catacaagtc cgatcacctt cgtccagctg 17460
   gtctcctaca gcccctgccg atcccaaatg gggtgtggtc ggacatcggc atcgacttca 17520
   tcgaagctct gccccgcgtg caaggcaaga cggtcattct cactgtcgtc gaccgtttca 17580
   gcaagtactg ccacttcatc ccactggcgc acccatacac ggcggagtcg gtggcgcaag 17640
   cattctgcgc cgaggtggtg aggctgcacg gcatcccgca gtccatcgta tcggaccgag 17700
   accccgtctt cacgtcggcc ttctggaggg agctaatggc cctgactgga accaagttgt 17760
   tcatgtcctc cgccttccat ccgcaaactg acggccagtc ggaggcagcc aaccgcgtca 17820
   tcatgatgta ccttcgctgc ttcaccggcg atcgaccccg acagtggctc cggtggcttc 17880
   catgggcgga gtacacctac aacaccgcat accagtcctc actccgcgag acacctttcc 17940
   gggtggtcta cggccgcgac ccaccgacca tccggtccta cgagccgggt gacgcacgcg 18000
   tgcccgcggt ggcacaggag atggaggaac gggctgcctt cctcgacgac gttcgctacc 18060
   gtctccatca ggcgcaagaa tcccagaaac gcgcctacga tcagcatcac aggcaggtcg 18120
   tctacaatgt cggtgactgg gcccttcttc gtctccgtca acgaccatcg gcatccctgc 18180
   cacgttccac cacgggcaag ttgaagcccc gctacgtcgg cccctaccgt gtcaaggaga 18240
   tcatcaacga ggcggccgta cgccttcagc ttccatccga cgccaaaatc cacgacgtct 18300
   tccatgtggg catcctcaag aagttcgttg gggcgccacc aacggaacca cctgcgctac 18360
   cggccacact caacggcgca gtggtgtcca caccttcccg tgtcctggcg ggtcgcctgg 18420
   ctcgcggcat tcggcaagtt ctggtacagt ggcacggtga accagtgtcg tctgcgacct 18480
   gggaggattt tgaggatttc agggcaagct tccccgcttt ccagctcgag gacgagctga 18540
   ctttcgaggc cgggagagat gtcatgtacg gtaagccgta cacaaggcgc aggcgcgctc 18600
   gcgacatccg gcgggcagcg gagcgcgcca acagtgcagc agcgacgcag atggcaggca 18660
   ctcaagttta ggaaagcagc agccacctat ggcaagggat aaataggaaa gtatcaattt 18720
   ggctcttatg gcaaggaata agtaggaaag tagcaattta gcacttatgg caaggaataa 18780
   gtaggaaagt agtattttgg cagggaagat aattagggag cttgctccat ttgtgtggcc 18840
   gccggcccta tatatcccca agtgcgcagc actgttgagg gcaataaagt atcaagtcat 18900
   ctcccacctc tctctctcaa gcctgcggtt aggaggaata ctcctaccgg agaagacggc 18960
   cgaaggctac gagttacgta gccgaggccc ggcgagtcaa gggttcgacg ctcttgacag 19020
   gatgcgagtc aaaatcatgt catagtagat tccatctaac tcaccagcac agctcaaaca 19080
   aaaaatgtaa tggacaataa ggttattcca aaattctaat tccagagtct gatcaccttc 19140
   gtatgggtag tcccgatagc aacgaaactc tccttggtgg tggcctcatc gtctggtgtc 19200
   ttgcatggca gcatgctgaa tgtccagcct tcaaggatgc atgtcctctg ggtcatcccc 19260
   ttaaacacca tcagttgtag gaagatgaac ctcagaagca tcaactctgc atcacacaga 19320
   gacaaagagg taaggtttgt gccgtaaaca tatgtatctt atccggaatc atagagaaaa 19380
   ggaaaggaag actgagggtg tgtttggatt attgcatacc tgaaaaaagt tacgtcttgt 19440
   tgctgtcaac atttttaaat agccaaccgc tcagtttcat tttggttcaa ttgtacgaag 19500
   gacaatcagt gtccggtgtt cttgatagtc aaggcttagg gcatacaagt tttatcctcg 19560
   taaaactgac tttaccttac attgttttgc catccttttt tatctgtatt atatcgttat 19620
   ctatgattta cttatcttga agcgaaaata taaagattgg gaaatggagg caccttcttt 19680
   gaccttgagc agctcctcga agagcggctt ttggttcttc ctcttgagcg tctacaggac 19740
   acatcatcga agcgctcctc gaaggcgacc ccttatcttg aagcgaaaat ataaagattg 19800
   ggaaatggag gcaccttctt tgaccttgag cagttcctcg aagagcggcc tctggttctt 19860
   cctcttgagc gcctgcagga cacatcatca aagcgttcct cgaagatgac ctcaccggaa 19920
   gcgaggaaca acaacgacaa gagggcgagg aggcaataga gcacgccgcc acgctggggc 19980
   gtggagcccg cggtggccat gcgcccgcac gtgctcaagg tcccaagcag tggggatgtc 20040
   gcgcgccgcc gcggcctcgg gatctgcatg ctcgtgggga cgggcgccac gtcgtcgtct 20100
   cgcgctccgg ctccgtccgc ggaggaggct atgcgaccgt ctttcgtggc cagtacgaga 20160
   tcctatccac ctcggtcgcc gttgccgcca tcccctgttc cctagagcgt tccacgaagg 20220
   tgaaggaaac ccttgatgca ggtgggaaga gatcgcagac atcaaggtag gtggagggag 20280
   caatagcact gtagcagtag catactactc ggcttggctg ttgactgttg aggaggctag 20340
   aacttttcgg cacggcaagt tttttaatat aaggattcat gagaggacat gcatgccgaa 20400
   atgtttgacc agctgaacaa ttcgcgttcg tggtcgcttt ccacacgtcg ggaccggtgt 20460
   ggggaaaatc acgcgaaacg cggagagcgt tccaaaaccc atcgtatttt aatactttag 20520
   atggctttca ttgttgacct tccaggcatg aaaccaaatt ggttcaaaga gatcctcgtt 20580
   attgctttca aacgatgctt gataactctc ccccatagct tcatagtatg actcatcaac 20640
   ttaattcctt ggtaattatt acagctttaa atatctccct tattcttgta aatcggtaca    20700
   aataatactt ctccacttgt taggcgtatt gttcgatcga aagatatggt tgaacaactt    20760
   ggttagccat actatagcaa tatcctcgag gcatcttcat acctcaattg gaatatcatt    20820
   aggatccatc accttacccc ctttcattct tttcaaagcc tccctaacct tagattcttg    20880
   aatcttacgc acaaagagcc tattggtgtc atcaaaagag tcatccaact gaaaggttgt    20940
   gtccccattc tccccattga ttaatttgtc aaagtactct taccatatat gcctgatctc    21000
   gtcctccttc accaagaggc gctctgtttc atccttaatg cacttaactc gattgaggtc    21060
   tcttgtcttc ctgtcacgaa ccctggctat cctatatctg cccttctctc ctttcttcgt    21120
   actcaagcat tggtaaagat ccttagacgc actattcttt gctaccatgt atttttctat    21180
   gctatccaca ctcctaccta ctcttataaa gcaccagttt ccttggtttc caccattgtc    21240
   cccaaggttt taaagtctgg caactagtcg agaagtcgat ctgaacgact cgactttctc    21300
   tactagtcgc tcgactcgtt gactagtcac gagtagtaac gactagtcgc agcgattgag    21360
   tcgggtgact tatttgggcc taggtataaa aatgtgagcc catccagcca tctcaaccct    21420
   agtaggtatg agatggaatg agatggtatg aaatgctaat agacaagttt tctaccttat    21480
   ttatctatga tatggaatga ttcatgactt gatgaggcca agacaaatgg ttgagatctt    21540
   gtgaggcatg caactatgtt ttgcaactac tatgtttgca acatccttcc ttacattggc    21600
   taatatgtgg aaacaaagac aaggatcgaa agctctcttt gttagtaatg aatggtgtga    21660
   gagcaaaatg aagaacactg aagtaggaaa tgcacgtgtt gtttgtcact ctgttgcatt    21720
   ttggcaagca gtagaagatt gcatgagagc ttcacaacct attctaatat tcctgaggat    21780
   agttgatggt gatgagagac tggcaatggc tatatggtgg gctgccatgg accatgaaaa    21840
   aagagcatca aaaaagcttt ggagcacata gaatcataga aagaataata gatgaagtaa    21900
   tttctattgt tgataagggg tgggacagtc agatggatac caatctgtat agagcaacat    21960
   tgtttttgga tccaaacaag ttctttgaaa ccaaagagaa cgatagaaga cttactacaa    22020
   tgctatattc aatatttaat gatatattgt agaagatgga gatggatgaa aatatactaa    22080
   acaaggtgtt tttagtgttc tagcatgttt ttagtcttag cgtatgtaga aagttataaa    22140
   aatataatca ctgacttatg ttcgactagt cacgactagt ttgtcctagt ctcgagtctc    22200
   tactagtcag tagttgcacg actcgactcg aaaacatcga ttgtgtggga aaccctaacc    22260
   ctaacaaggg gttgggtgat gtattaatag actgggtagt tgggcctgac ccattacaca    22320
   ggggtgagaa ggtaaataca cggggtagac cccaaaccct aaacgagtac tctaatactc    22380
   taacagattg tcccttttgc aaaaaaaggt ccttatactt ctttctaatc taactgtagc    22440
   ccaacttata gatcttgatt tcctccccca tgtcttgcct aacaaccaaa taagtgtgtg    22500
   ggtgtgtcac gctcgaagcg tttagatcta atttcctctc cttgtctggc gtaacaacta    22560
   gataagataa gtcttacccc ttgcatgcac aaccaaccac cacgagtgct tcctcctcct 22620
   cgttattggt gtcgtatatt aacccctaac atcacatctc tttccccatc cctgcctttc 22680
   tcacccaaat tcttccgtgg aggacatgca gtaggccatc ctgctggcct ccaactcgcc 22740
   agtcgctgtc tccgcgccct accggcgccg ccgcctttac cttcccccgt cggcaagcgc 22800
   ctcgatcttg tagcgcctcc tctctttgcc gccatgcgcc tttgcagtca gcggggcaag 22860
   aacaaccgcg ggagagtagg aggcgttgcc gccgctggcg gtgcgtggcc ggacaattga 22920
   gggagaggag gcgctctaga ttccgagttt agttttgtat tggttgttgg caaaaaaatt 22980
   gtgagatatt aaggtcgatg ataatatatg tattgctcgt tttcatgtaa tcgttgtgcg 23040
   ctaatatttt atcgaatatt tttgtgccgt cacaacacac gtgcattata ctagtcatgg 23100
   tacaatgtta gcattttttt cttctcctta atagcccttt agacttcctc attccactac 23160
   caagtatctt tagcttcgcc tctattctct ctagttactc cacacacctc ttaggtggcc 23220
   tttcgaatgc aggttttcat cttctcccac atgttgtttg tgtcatcttc cttccaagtg 23280
   tcctctttga gaagcaagat gacacaaaca ttgaacttgc attgtatttt atggtgtttg 23340
   gcatgtgaat tcatttagct gagagttgat tgaattggac tacaattgga cttccgtttt 23400
   tttacacaca tttgggaggg gggcgggagg gcagacccac ccagtggtgg aggttcccac 23460
   atgagtgggg ttggggaagg gataaaccaa ggcaagcctt cccctcgcta atacggagat 23520
   gctgattcga acccgtgtcc tcgtgactca ctgggacatc tctcaccact gcactaggcc 23580
   tgcactttat ttgtttttct catttttttt tgttttccta acttgttcat agttattgta 23640
   cttcccgctt ttttacacgt tacaggaatt gttgtttttt gttcttttga aggtaattgc 23700
   aatagtagtt tatgtagctt gtcaactgta ttctcactgc ttacatgtgc tttctacaca 23760
   ggatttctta catgagatgt cacggaagga cgaaaaagtt gattccttag ttaaaatggg 23820
   gtttcctgaa gacgaggctg cactggctat taccagatgc ggtgtgctct ctgagacttt 23880
   gttccattct cgtctctttg cattagattc ctcagcaaaa taaacatcaa tttccaacct 23940
   tgaaagtagg atatgctctc atatagaata aatcagttga ataataacat tgccatgttt 24000
   aattatatgt caagctttct attggtttac tctctggatt ttgagaattc tcttcaaatg 24060
   ctgtaacttg gaactcgtaa attggctgca atcttgacct gaggtaacct agaatggttg 24120
   aatctttcca agcttgattt actgtttgca tcaagtttgg gttaaccagt tatattcaaa 24180
   tctgaagaca aagcatttag gcatgcccat aataatctgt agaaagcaag gcgtatgata 24240
   aatattattt cttacaagaa taactgggtg tttaggaagt tatccatcca agtagtacat 24300
   gtaaaccttt gtttctatga gtagcaagca ttcagatgct ccttcaaagc agtcaacgaa 24360
   cagtttattg ttcgtttggg gtcagaatat tttaatggca tttgctgtta aataaaacct 24420
   ttttttgcat cttgcatgca acttgtaccg ctcagattct gcccccgtcc ctgttttatt 24480
   ttaactggcc atgcacctta cgccatctac tgtggtctgt ggttgaaaat tactcagggc 24540
   cggatgcatc tatttctgtt ctggtggatt caatctatgc ttcacagacc gcaggagatg 24600
   gttactgtgg caatctgtct gactatgagg tttgtcttac tgtccataca agtctgcaat 24660
   aagatttagt tctattataa tggcctggta atggaactgt gtctgcaata ctgcaggata 24720
   attcctatgg agggagaagc acagggaaca agaaaaagag aaaaagatat ggaggccaag 24780
   cacagggaag tagaggccca ttagatggta gctgtgatga acccatgcct ctcccacatc 24840
   caatggttgg atttaacttg ccagaccagt ggtcaagacg agtggacaga tcgttgcctg 24900
   cacaagctat tggtccgccg tacttctact acgagaacgt tgctcttgct ccaaaaggtg 24960
   tctggactac catatcaaga ttcttgtatg atattcaacc agagtttgtg gactcaaagt 25020
   acttctgtgc tgctgccagg aaaaggggtt acatacacaa cctgccactt gagaacaggt 25080
   cacctctcct ccccataccc ccaaagacga tatcggaagc atttcctcgg accaagaggt 25140
   ggtggccttc atgggaccca agacgacagt tcaattgcct ccagacttgc gtgtctagtg 25200
   caaaattgtt agagaggatt cgcgtagccc tcacaaacag ttcagaccca cctcctccaa 25260
   gagttcagaa gtatgtgttg gaggagtgta ggaaatggaa cctggcatgg gttggcttaa 25320
   acaaggttgc tcctctagag cctgacgaga tggagtttct actcggcttt ccgaaggatc 25380
   acacgagagg tatcagcagg acagagaggt atcgatctct aggaaattca tttcaggtcg 25440
   atactgttgc ttaccatctc tcagttctga aggatctgtt cccacaaggc atgaatgtgc 25500
   tgtctttatt ctctggtatt ggaggagcag aggtggctct ccacaggctt ggcatacaga 25560
   tgaacacggt tatttcagtg gagaagtccg aggtcaaccg gacgattctg aagagttggt 25620
   gggatcagac gcagacgggt actctgattg agatcactga tgtgcagaca ctgtcatctg 25680
   agaggatcga ggcgtatatt agaagaattg ggggcttcga tcttgtgatt ggtggaagtc 25740
   cctgtaacaa cctcactggg agcaaccgtc accacagaga tggtttggag ggcgagcatt 25800
   ctgcattgtt ccatcattat tttaggatct tacacgccgt caagtccatc atggagcgtt 25860
   tgtagttctt agaatcttta ctgttgtttt ggttctagta gaatactttg tgaccgaact 25920
   tgtaaatggt ttgcaggttg actggtttag ttctcttgcc actctaattt aggctagttt 25980
   tttttttatt taatcttctt tggtgatctt ggtgcagttc tgtggcactg ttgggtattc 26040
   aaatgcagat tgatccgaga ggctgccctg ttgcattttc tttttaaaat ttgtatttgt 26100
   caatgagaga tcaggtattc tggttgccaa aaaaaaatct gaagttgtgg agctgagaaa 26160
   aggacggtgc taagctacaa ccacccactg ccacaccatg cgcaccccca acccaaaaga 26220
   tcctgctttt gctcacgctt atcaccactc ccatctccat ccaaaaccat aaaaaaaacc 26280
   agtgcgagct gaagcatcca ggctctggaa tcatcgtcgg gatgtctgtg ccagcatcag 26340
   cccaggtgcc tcacaccata cgatctgctg ggagctgctg cttcaggtcg tcgcatcaac 26400
   tatctgcgca gaatctgggc cgacgtgagt tcccgatctc agctttgagt ggccttcttt 26460
   ttgaaagtga gcacaggttc ttccaaagcc cgttgtgttc gtgtagatcg caggtggcgg   26520
   gggaccggaa agaagaagat cctttccacc cgtggcgtgc ttgagtccag aaacggcgca   26580
   catcctggtg gccttatgaa gagaaggtga gaacctggtg aaagacatgc ggagctgtag   26640
   ctagacagct agctgatcta gtagcagcgc acgccttgca ggaaggtcgt ggagcacatc   26700
   atcctgctca gagccaagcc caacatctca gacgctgaag agaaagacat gctggactac   26760
   ctatacacat cg                                                       26772